(19) Europäisches Patentamt
European Patent Office
Office européen des brevets

(11) EP 1 241 176 A1

(12) EUROPEAN PATENT APPLICATION

(43) Date of publication:
18.09.2002 Bulletin 2002/38

(21) Application number: 02251174.5

(22) Date of filing: 20.02.2002

(51) Int Cl.[7]: C07H 19/16, C07D 473/34, C07D 471/04, A61K 31/7076, A61P 9/10 // (C07D471/04, 235:00, 221:00)

(84) Designated Contracting States:
AT BE CH CY DE DK ES FI FR GB GR IE IT LI LU MC NL PT SE TR
Designated Extension States:
AL LT LV MK RO SI

(30) Priority: 16.03.2001 US 276411 P

(71) Applicant: Pfizer Products Inc.
Groton, Connecticut 06340 (US)

(72) Inventors:
- Deninno, Michael Paul, Pfizer Global Research Groton, Connecticut 06340 (US)
- Masamune, Hiroko, Pfizer Global Research Groton, Connecticut 06340 (US)

(74) Representative: Hayles, James Richard et al
UK Patent Department,
Pfizer Limited,
Ramsgate Road
Sandwich, Kent CT13 9NJ (GB)

# (54) Purine derivatives for the treatment of ischemia

(57) $A_3$ agonists having Formula I are described herein as well as methods of using such $A_3$ agonists and pharmaceutical compositions containing such $A_3$ agonists.

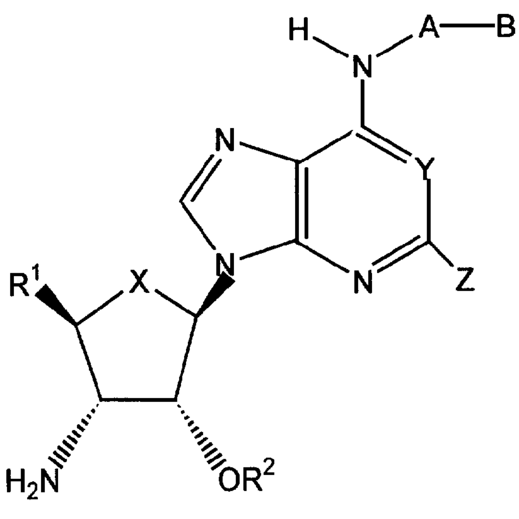

Formula I

The $A_3$ agonists are useful for the reduction of tissue damage resulting from tissue ischemia or hypoxia.

EP 1 241 176 A1

**Description**

FIELD OF THE INVENTION

The present invention relates to adenosine A-3 receptor agonists, pharmaceutical compositions containing such agonists and the use of such agonists to treat, for example, ischemia, in particular, perioperative myocardial ischemic injury in mammals, including humans.

BACKGROUND

Myocardial ischemic injury can occur in out-patient as well as in perioperative settings and can lead to the development of sudden death, myocardial infarction or congestive heart failure. There is an unmet medical need to prevent or minimize myocardial ischemic injury, particularly perioperative ischemic injury. Such a therapy is anticipated to be life-saving and reduce hospitalizations, enhance quality of life and reduce overall health care costs of high risk patients.

Pharmacological cardioprotection would reduce the damage from myocardial infarction and dysfunction occurring in these surgical settings (perioperatively). In addition to reducing myocardial damage and improving postischemic myocardial function in patients with ischemic heart disease, cardioprotection would also decrease the incidence of cardiac morbidity and mortality due to myocardial infarction and dysfunction in patients "at risk" (such as greater than 65 years, exercise intolerant, coronary artery disease, diabetes mellitus, hypertension) that require noncardiac surgery.

U.S. Patent No. 5,604,210 discloses the use of certain adenosine type compounds for the prevention or treatment of a brain edema, an intracranial hemorrhage and a cerebral infarction.

U.S. Patent No. 5,688,774 discloses $A_3$ selective agonists, particularly, adenine compounds having selected substituents at the 2, 6 and 9 positions, and related substituted compounds, particularly those containing substituents on the benzyl and/or uronamide groups as agents which activate the $A_3$ receptor.

U.S. Patent No. 5,773,423 discloses $N^6$-benzyladenosine-5'-N-uronamide and related substituted compounds, particularly those containing substituents on the benzyl and/or uronamide groups, and modified xanthine ribosides for the activation of the $A_3$ adenosine receptor.

J. Med. Chem. 1994, 37, 636-646, "Structure-Activity Relationships of $N^6$-Benzyladenosine-5'-uronamides as $A_3$-Selective Agonists" discloses the synthesis of adenosine analogues modified at the 5'-position as uronamides and/or as $N^6$-benzyl derivatives which are potentially useful as pharmacological and biochemical probes for $A_3$ receptors.

J. Med. Chem. 1995, 38, 1174-1188, "Search for New Purine- and Ribose-Modified Adenosine Analogues as Selective Agonists and Antagonists at Adenosine Receptors", discloses that the binding affinities at rat $A_1$, $A_{2a}$, and $A_3$ adenosine receptors of a wide range of derivatives of adenosine have been determined. In particular, 3'-β-amino compounds were found to have no activity.

J. Med. Chem. 1995, 38, 1720-1735, "Structure-Activity Relationships of 9-Alkyladenine and Ribose-Modified Adenosine Derivatives at Rat $A_3$ Adenosine Receptors" discloses the synthesis of 9-alkyladenine derivatives and ribose-modified $N^6$-benzyladenosine derivatives as leads for the development of antagonists for the rat $A_3$ adenosine receptor.

U.S. Patent No. 5,817,760 discloses recombinant human adenosine receptors A1, A2a, A2b, and A3 which were prepared by cDNA cloning and polymerase chain reaction techniques. The recombinant adenosine receptors can be utilized in an assay to identify and evaluate entities that bind to or enhance binding to adenosine receptors.

There is clearly a need and a continuing search in this field of art for treatments for perioperative myocardial ischemia.

SUMMARY OF THE INVENTION

The present invention is directed to a compound of Formula (I)

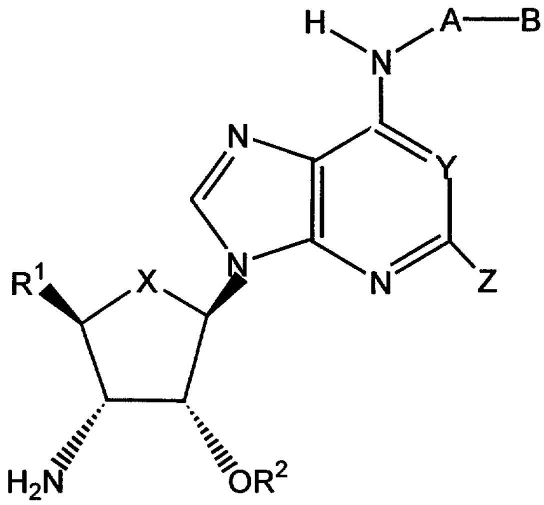

(I)

wherein

X is oxy, methylene or thio;
Y is CH or N;
Z is H, $(C_1-C_4)$alkyl, $(C_1-C_4)$alkyloxy, trifluoromethyl or halo;
$R^1$ is hydroxymethyl, $(C_1-C_3)$alkoxymethyl, $(C_3-C_5)$cycloalkoxymethyl, carboxy, $(C_1-C_3)$alkoxycarbonyl, $(C_3-C_5)$cycloalkoxycarbonyl, 1,1-aminoiminomethyl, 1,1-(mono-N- or di-N,N-$(C_1-C_4)$alkylamino)iminomethyl, 1,1-(mono-N- or di-N,N-$(C_3-C_5)$cycloalkylamino)iminomethyl, carbamoyl, mono-N- or di-N,N-$(C_1-C_4)$alkylaminocarbonyl, mono-N- or di-N,N-$(C_3-C_5)$cycloalkylaminocarbonyl or N-$(C_1-C_4)$alkyl-N-$(C_3-C_5)$cycloalkylaminocarbonyl;
$R^2$ is H, $(C_1-C_3)$alkyl or $(C_3-C_5)$cycloalkyl;
A is $-(CH_2)_n-$ where n is 1 to 4, or $-(C_mH_{2m-2})-$ where m is 3 to 6 (e.g., cyclopropyl, cyclobutyl, methylcyclopropyl, cyclopentyl, methylcyclobutyl, cyclohexyl, methylcyclopentyl, ethylcyclobutyl, propylcyclopropyl, methylcyclopropylethyl, etc.); and
B is hydrogen, substituted or unsubstituted heteroaryl, substituted or unsubstituted aryl, $-CH(aryl)_2$, or

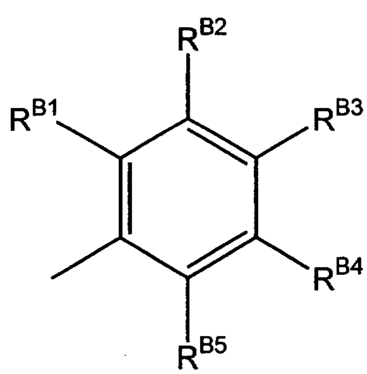

where $R^{B1}$, $R^{B2}$, $R^{B3}$, $R^{B4}$ and $R^{B5}$ are each independently selected from the group consisting of hydrogen, $(C_1-C_4)$alkyl, halo, hydroxy, thio, amino, $(C_1-C_6)$alkyloxy, $(C_1-C_6)$alkylthio, $(C_1-C_6)$alkylamino and -D-G, where

D is oxy, thio, NH, $(C_1-C_6)$alkyloxy, $(C_1-C_6)$alkylthio or $(C_1-C_6)$alkylamino and
G is a partially saturated, fully saturated or fully unsaturated five to eight membered ring optionally having one to three heteroatoms selected independently from oxygen, sulfur and nitrogen, or a bicyclic ring consisting of two

fused partially saturated, fully saturated or fully unsaturated three to six membered rings, taken independently, optionally having one to four heteroatoms selected independently from nitrogen, sulfur and oxygen, wherein G is optionally mono-, di- or tri-substituted independently with halo, $(C_1\text{-}C_3)$alkyl, trifluoromethyl, trifluoromethoxy, nitro, cyano, $(C_3\text{-}C_5)$cycloalkyl, hydroxy or $(C_1\text{-}C_3)$alkoxy, or
G is cyano, $(C_1\text{-}C_4)$alkoxycarbonyl, $(C_3\text{-}C_5)$cycloalkoxycarbonyl, C(O)$NR^4R^5$, C(S)$NR^4R^5$, C(NH)$NR^4R^5$, C(N$(C_1\text{-}C_3)$alkyl)$NR^4R^5$ or C(N$(C_3\text{-}C_{10})$cycloalkyl)$NR^4R^5$, where

$R^4$ is H, $(C_1\text{-}C_{10})$alkyl, hydroxy, $(C_1\text{-}C_{10})$alkoxy, $(C_3\text{-}C_{10})$cycloalkoxy or a partially saturated, fully saturated or fully unsaturated five to eight membered ring, optionally linked through $(C_1\text{-}C_3)$alkyl, optionally having one to three heteroatoms selected independently from oxygen, sulfur and nitrogen, or, a bicyclic ring or a bicyclic ring with optional $(C_1\text{-}C_3)$ bridge (e.g., adamantane) optionally linked through $(C_1\text{-}C_3)$alkyl, said bicyclic ring or bridged bicyclic ring optionally having one to four heteroatoms selected independently from nitrogen, sulfur and oxygen wherein said $(C_1\text{-}C_{10})$alkyl,$(C_1\text{-}C_{10})$alkoxy, $(C_3\text{-}C_{10})$cycloalkoxy or $R^4$ ring(s) is optionally mono-, di- or tri-substituted independently with halo, $(C_1\text{-}C_3)$alkyl, trifluoromethyl, nitro, cyano, $(C_3\text{-}C_5)$cycloalkyl, hydroxy or $(C_1\text{-}C_3)$alkoxy, and
$R^5$ is H, $(C_1\text{-}C_{10})$alkyl or $(C_1\text{-}C_{10})$cycloalkyl; or $R^4$ and $R^5$ taken together with the nitrogen to which they are attached form a fully saturated or partially unsaturated four to nine membered ring, said ring optionally bridged, optionally having one to three additional heteroatoms selected independently from oxygen, sulfur and nitrogen, said ring optionally mono- or di-substituted independently with oxo, hydroxy, $(C_1\text{-}C_6)$alkoxy, $(C_1\text{-}C_8)$alkyl, amino, mono-N- or di-N,N-$(C_1\text{-}C_4)$alkylaminocarbonyl, mono-N- or di-N,N-$(C_3\text{-}C_5)$cycloalkylaminocarbonyl, N-$(C_1\text{-}C_4)$alkyl-N-$(C_3\text{-}C_5)$cycloalkylaminocarbonyl, mono-N- or di-N,N-$(C_1\text{-}C_4)$alkylamino, mono-N- or di-N,N-$(C_3\text{-}C_5)$cycloalkylamino, N-$(C_1\text{-}C_4)$alkyl-N-$(C_3\text{-}C_5)$cycloalkylamino, formylamino, $(C_1\text{-}C_4)$alkylcarbonylamino, $(C_3\text{-}C_5)$cycloalkyl- carbonylamino, $(C_1\text{-}C_4)$alkoxycarbonylamino, N-$(C_1\text{-}C_4)$alkoxycarbonyl-N-$(C_1\text{-}C_4)$alkylamino, $(C_1\text{-}C_4)$sulfamoyl, $(C_1\text{-}C_4)$alkylsulfonylamino, $(C_3\text{-}C_5)$cycloalkyl- sulfonylamino or a partially saturated, fully saturated or fully unsaturated five to eight membered ring, optionally linked through $(C_1\text{-}C_3)$alkyl, optionally having one to three heteroatoms selected independently from oxygen, sulfur and nitrogen, or a bicyclic ring consisting of two fused partially saturated, fully saturated or fully unsaturated three to six membered rings, taken independently, optionally linked through $(C_1\text{-}C_3)$alkyl, optionally having one to four heteroatoms selected independently from nitrogen, sulfur and oxygen, optionally mono- or di-substituted with halo, trifluoromethyl, trifluoromethoxy, $(C_1\text{-}C_3)$alkyl or $(C_1\text{-}C_3)$alkoxy;
provided that A is not $-(CH_2)_1-$, when $R^{B1}$ is -D-G, $R^{B4}$ is halo,
trifluoromethyl, cyano, $(C_1\text{-}C_3)$ alkyl, $(C_1\text{-}C_3)$ alkyloxy, ethenyl or ethynyl, and $R^{B2}$, $R^{B3}$ and $R^{B5}$ are hydrogen;
a prodrug thereof, or pharmaceutically acceptable salt, solvate, or hydrate of the compound or the prodrug.

In a preferred embodiment, X is oxy; Y is N; Z is H or Cl; $R^1$ is $(C_1\text{-}C_6)$alkylcarbamoyl; $R^2$ is H; A is $-(CH_2)_n-$, where n is 1 or 2, or cyclopropyl; B is substituted or unsubstituted heteroaryl, naphthyl,

$-CH(aryl)_2$, or

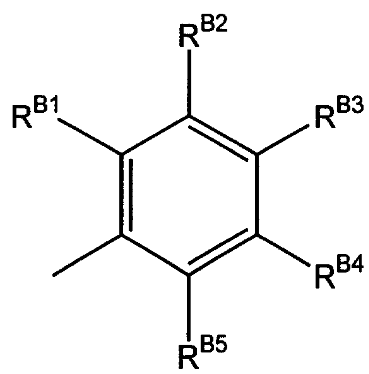

where $R^{B1}$, $R^{B2}$, $R^{B3}$, $R^{B4}$ and $R^{B5}$ are each independently selected from the group consisting of hydrogen, $(C_1\text{-}C_4)$alkyl, halo, hydroxy, thio, amino, $(C_1\text{-}C_6)$alkyloxy, $(C_1\text{-}C_6)$alkylthio, $(C_1\text{-}C_6)$alkylamino and -D-G, where

D is oxy, thio, $(C_1\text{-}C_6)$alkyloxy or $(C_1\text{-}C_6)$alkylthio, and

G is phenyl, pyridyl, pyrimidinyl, pyrazinyl, thiazolyl, oxazolyl, isoxazolyl, pyridinazinyl, tetrazolyl, isothiazolyl, thiophenyl, furanyl, 1,2,4-oxadiazolyl, 1,2,4-thiadiazolyl, pyrazolyl, pyrrolyl, indolyl, naphthalenyl, quinolinyl, isoquinolinyl, benzo[b]furanyl, benzo[b]thiophenyl, benzothiazolyl, tetrahydrofuranyl, pyrrolidinyl, piperidinyl, tetrahydropyranyl, morpholinyl wherein said G is optionally mono-, di- or tri-substituted independently with halo, $(C_1\text{-}C_3)$alkyl or $(C_1\text{-}C_3)$alkoxy; a prodrug thereof, or a pharmaceutically acceptable salt, solvate, or hydrate of the compound or the prodrug.

In a more preferred embodiment, B is either (i) a substituted or unsubstituted pyridyl, indolyl or thiazolyl where the substituted pyridyl, indolyl or thiazolyl is substituted with at least one substituent selected from the group consisting of $(C_1\text{-}C_4)$alkyl, halo, hydroxy, thio, amino, $(C_1\text{-}C_6)$alkyloxy, $(C_1\text{-}C_6)$alkylthio, $(C_1\text{-}C_6)$alkylamino and -D-G, where D is oxy, thio, $(C_1\text{-}C_6)$alkyloxy or $(C_1\text{-}C_6)$alkylthio, and G is phenyl, pyridyl, pyrimidinyl, pyrazinyl, thiazolyl, oxazolyl, isoxazolyl, pyridinazinyl, tetrazolyl, isothiazolyl, thiophenyl, furanyl, 1,2,4-oxadiazolyl, 1,2,4-thiadiazolyl, pyrazolyl, pyrrolyl, indolyl, naphthalenyl, quinolinyl, isoquinolinyl, benzo[b]furanyl, benzo[b]thiophenyl, benzothiazolyl, tetrahydrofuranyl, pyrrolidinyl, piperidinyl, tetrahydropyranyl, morpholinyl wherein said G is optionally mono-, di- or tri-substituted independently with halo, $(C_1\text{-}C_3)$alkyl or $(C_1\text{-}C_3)$alkoxy; or ii)

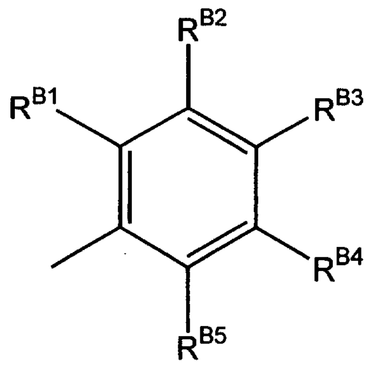

where $R^{B1}$, $R^{B2}$, $R^{B3}$, $R^{B4}$ and $R^{B5}$ are each independently selected from the group consisting of hydrogen, $(C_1\text{-}C_4)$alkyl, halo, hydroxy, $(C_1\text{-}C_6)$alkyloxy and -D-G, where D is $(C_1\text{-}C_6)$alkoxy and G is phenyl, pyridyl, thiazolyl, oxazolyl, isoxazolyl, isothiazolyl, furanyl, 1,2,4-oxadiazolyl, 1,2,4-thiadiazolyl, pyrazolyl, pyrrolyl, or morpholinyl wherein said G is optionally mono-, di- or tri-substituted independently with halo, $(C_1\text{-}C_3)$alkyl, trifluoromethoxy or $(C_1\text{-}C_3)$alkoxy; a prodrug thereof, or a pharmaceutically acceptable salt, solvate, or hydrate of the compound or the prodrug.

Another aspect of this invention are methods of treating a mammal (e.g., human) having a disease or condition mediated by an $A_3$ adenosine receptor by administering a therapeutically effective amount of a compound of Formula (I), prodrug thereof, or a pharmaceutically acceptable salt, solvate, or hydrate of the compound or the prodrug to the mammal.

One embodiment of the present invention is directed to methods of reducing tissue damage (e.g., substantially preventing tissue damage, inducing tissue protection) resulting from ischemia or hypoxia comprising administering to a mammal (e.g., a female or male human) in need of such treatment a therapeutically effective amount of a compound of Formula I, a prodrug thereof, or a pharmaceutically acceptable salt, solvate, or hydrate of the compound or the prodrug. The method may include reduction of tissue damage resulting from ischemia/hypoxia during organ transplantation.

Preferred ischemic/hypoxic tissues taken individually or as a group include ischemic/hypoxic tissues such as cardiac, brain, liver, kidney, lung, gut, skeletal muscle, spleen, pancreas, nerve, spinal cord, retina tissue, the vasculature, and intestinal tissue. An especially preferred ischemic/hypoxic tissue is cardiac tissue.

It is preferred that the compounds are administered to prevent perioperative myocardial ischemic injury. Preferably, the compounds of the present invention are administered prophylactically.

Preferably, the compounds of the present invention are administered prior to, during and/or shortly after, cardiac surgery or non-cardiac surgery (e.g., infusion either continuously or in multiple bolus doses over a three to four day period). The compounds of the present invention may also be administered locally.

A preferred dosage is about 0.001 to about 100 mg/kg/day of the Formula I compound, a prodrug thereof or a pharmaceutically acceptable salt of said compound or of said prodrug. An especially preferred dosage is about 0.01 to about 50 mg/kg/day of a compound of Formula I, a prodrug thereof, or a pharmaceutically acceptable salt, solvate, or hydrate of the compound or the prodrug.

Another embodiment of the present invention is directed to methods of reducing myocardial tissue damage

(e.g., substantially preventing tissue damage, inducing tissue protection) during surgery (e.g., coronary artery bypass grafting (CABG) surgeries, vascular surgeries, percutaneous transluminal coronary angioplasty (PTCA) or any percutaneous transluminal coronary intervention (PTCI), organ transplantation, or other non-cardiac surgeries) comprising administering to a mammal (e.g., a human) a therapeutically effective amount of a compound of Formula I, a prodrug thereof, or a pharmaceutically acceptable salt, solvate, or hydrate of the compound or prodrug.

The methods of the present invention may also be used to reduce myocardial tissue damage (e.g., substantially preventing tissue damage, inducing tissue protection) in a patient presenting with ongoing cardiac (acute coronary syndromes, e.g., myocardial infarction or unstable angina) or cerebral ischemic events (e.g., stroke), or a patient with diagnosed coronary heart disease (e.g., previous myocardial infarction or unstable angina) or a patient who is at high risk for myocardial infarction (e.g.,age > 65 and two or more risk factors for coronary heart disease).

In yet another embodiment of the present invention, methods of preventing ischemic/hypoxic damage is provided which comprises the chronic oral administration to a mammal (e.g., human) in need of such treatment of a therapeutically effective amount of a compound of Formula I, a prodrug thereof, or a pharmaceutically acceptable salt, solvate, or hydrate of the compound or the prodrug.

The present invention is also useful for treating cardiovascular diseases, arteriosclerosis, arrhythmia, angina pectoris, cardiac hypertrophy, renal diseases, diabetic complications, restenosis, organ hypertrophies or hyperplasias, septic shock and other inflammatory diseases (e.g., septicemia and endotoxcemia), cerebro ischemic disorders, myocardial stunning, myocardial dysfunction, and cerebrovascular diseases by administering to a mammal (e.g., a human) a therapeutically effective amount of a compound of Formula I, a prodrug thereof, or a pharmaceutically acceptable salt, solvate, or hydrate of the compound or the prodrug.

In another aspect of the present invention, pharmaceutical compositions are provided which comprise a therapeutically effective amount of a compound of Formula I, a prodrug thereof, or a pharmaceutically acceptable salt, solvate, or hydrate of the compound or the prodrug, and a pharmaceutically acceptable carrier, vehicle or diluent.

The present invention is also directed to a pharmaceutical composition for the reduction of tissue damage resulting from ischemia or hypoxia which comprises an ischemia or hypoxia tissue damage reducing amount of a compound of Formula (I), a prodrug thereof, or a pharmaceutically acceptable salt, solvate, or hydrate of the compound or the prodrug, and a pharmaceutically acceptable carrier, vehicle or diluent.

In yet another aspect of the present invention, a pharmaceutical kit for use by a consumer having or at risk of having a disease or condition resulting from, for example, ischemia or hypoxia is provided. The kit comprises a) a suitable dosage form such as an injectable parenteral solution particularly adapted for intravenous or intramuscular injection comprising a compound of Formula I, prodrug thereof, or a pharmaceutically acceptable salt, hydrate or solvate of the compound or prodrug; and b) instructions describing a method of using the dosage form to reduce tissue damage resulting from ischemia or hypoxia.

Yet another aspect of this invention are combinations of a compound of Formula I, a prodrug thereof, or a pharmaceutically acceptable salt, solvate, or hydrate or the compound or the prodrug, and other compounds as described below.

In one embodiment, a pharmaceutical combination composition is provided which comprises a therapeutically effective amount of a composition comprising

a) a first compound, said first compound being a compound Formula I, a prodrug thereof, or a pharmaceutically acceptable salt, solvate, or hydrate of the compound or the prodrug;
b) a second compound, said second compound being a cardiovascular agent, glycogen phosphorylase inhibitor, sorbitol dehydrogenase inhibitor, or aldose reductase inhibitor; and, optionally,
c) a pharmaceutical carrier, vehicle or diluent.

In another embodiment of the present invention methods of reducing tissue damage (e.g., substantially preventing tissue damage, inducing tissue protection) resulting from or which could result from ischemia or hypoxia is provided which comprises administering to a mammal (e.g., a human)

a) a first compound, said first compound being a compound of Formula (I), a prodrug thereof, or a pharmaceutically acceptable salt, solvate, or hydrate of the compound or the prodrug; and
b) a second compound, said second compound being a cardiovascular agent, glycogen phosphorylase inhibitor, sorbitol dehydrogenase inhibitor, or aldose reductase inhibitor wherein the amounts of the first and second compounds result in a therapeutic effect.

In yet another embodiment of the present invention, a kit is provided which comprises:

a) a compound of Formula I, a prodrug thereof, or a pharmaceutically acceptable salt, solvate,or hydrate of the

compound or the prodrug, and a pharmaceutically acceptable carrier, vehicle or diluent in a first unit dosage form;
b) a cardiovascular agent, a glycogen phosphorylase inhibitor, sorbitol dehydrogenase inhibitor, or aldose reductase inhibitor and a pharmaceutically acceptable carrier, vehicle or diluent in a second unit dosage form; and
c) a container.

In the above combination compositions, combination methods and pharmaceutical kits, preferably the cardiovascular agents including the pharmaceutically acceptable salts, solvates and hydrates thereof (e.g., agents having a cardiovascular effect) are, for example, β-blockers (e.g., acebutolol, atenolol, bopindolol, labetolol, mepindolol, nadolol, oxprenol, pindolol, propranolol, sotalol), calcium channel blockers (e.g., amlodipine, nifedipine, nisoldipine, nitrendipine, verapamil), potassium channel openers, adenosine, adenosine receptor agonists, sodium-hydrogen exchanger type 1 (NHE-1) inhibitors, ACE inhibitors (e.g., captopril, enalapril), nitric oxide donors (e.g., isosorbide dinitrate, isosorbide 5-mononitrate, glyceryl trinitrate), diuretics (e.g., hydrochlorothiazide, indapamide, piretanide, xipamide), glycosides (e.g., digoxin, metildigoxin), thrombolytics (e.g. tPA), platelet inhibitors (e.g., ReoPro™), aspirin, dipyridamol, potassium chloride, clonidine, prazosin, pyruvate dehydrogenase kinase inhibitors (e.g., dichloroacetate), pyruvate dehydrogenase complex activators, biguanides (e.g., metformin) or other adenosine receptor agonists. Other cardiovascular agents include angiotensin II (All) receptor antagonists, C5a inhibitors, soluble complement receptor type 1 (sCR1) or analogues, partial fatty acid oxidation (PFOX) inhibitors (specifically, ranolazine), acetyl CoA carboxylase activators, malonyl CoA decarboxylase inhibitors, 5'AMP-activated protein kinase (AMPK) inhibitors, adenosine nucleoside inhibitors, anti-apoptotic agents (e.g., caspase inhibitors), monophosphoryl lipid A or analogues, nitric oxide synthase activators/inhibitors, protein kinase C activators (specifically, protein kinase ε), protein kinase δ inhibitors, poly (ADP ribose) synthetase (PARS, PARP) inhibitors, metformin (gluconeogenesis inhibitors, insulin sensitizers), endothelin converting enzyme (ECE) inhibitors, endothelin ETA receptor antagonists, (thrombin activated fibrinolytic inhibitor) TAFI inhibitors and Na/Ca exchanger modulators.

Preferred NHE-1 inhibitors are [1-(8-bromoquinolin-5-yl)-5-cyclopropyl-1*H*-pyrazole-4-carbonyl]guanidine; [1-(6-chloroquinolin-5-yl)-5-cyclopropyl-1*H*-pyrazole-4-carbonyl]guanidine; [1-(indazol-7-yl)-5-cyclopropyl-1*H*-pyrazole-4-carbonyl]guanidine; [1-(benzimidazol-5-yl)-5-cyclopropyl-1*H*-pyrazole-4-carbonyl]guanidine; [1-(1-isoquinolyl)-5-cyclopropyl-1*H*-pyrazole-4-carbonyl]guanidine; [5-cyclopropyl-1-(4-quinolinyl)-1*H*-pyrazole-4-carbonyl]guanidine; [5-cyclopropyl-1-(quinolin-5-yl)-1H-pyrazole-4-carbonyl]guanidine; [5-cyclopropyl-1-(quinolin-8-yl)-1H-pyrazole-4-carbonyl]guanidine; [1-(indazol-6-yl)-5-ethyl-1*H*-pyrazole-4-carbonyl]guanidine; [1-(indazol-5-yl)-5-ethyl-1*H*-pyrazole-4-carbonyl]guanidine; [1-(benzimidazol-5-yl)-5-ethyl-1*H*-pyrazole-4-carbonyl]guanidine; [1-(1-methylbenzimidazol-6-yl)-5-ethyl-1*H*-pyrazole-4-carbonyl]guanidine; 1-(5-quinolinyl)-5-*n*-propyl-1*H*-pyrazole-4-carbonyl]guanidine; [1-(5-quinolinyl)-5-isopropyl-1*H*-pyrazole-4-carbonyl]guanidine; [5-ethyl-1-(6-quinolinyl)-1*H*-pyrazole-4-carbonyl]guanidine; [1-(2-methylbenzimidazol-5-yl)-5-ethyl-1*H*-pyrazole-4-carbonyl]guanidine; [1-(1,4-benzodioxan-6-yl)-5-ethyl-1 *H*-pyrazole-4-carbonyl]guanidine; [1-(benzotriazol-5-yl)-5-ethyl-1*H*-pyrazole-4-carbonyl]guanidine; [1-(3-chloroindazol-5-yl)-5-ethyl-1*H*-pyrazole-4-carbonyl]guanidine; [1 -(5-quinolinyl)-5-butyl-1*H*-pyrazole-4-carbonyl]guanidine; [5-propyl-1-(6-quinolinyl)-1*H*-pyrazole-4-carbonyl]guanidine; [5-isopropyl-1-(6-quinolinyl)-1*H*-pyrazole-4-carbonyl]guanidine; [1-(2-chloro-4-methylsulfonylphenyl)-5-cyclopropyl-1*H*-pyrazole-4-carbonyl]guanidine; [1-(2-chlorophenyl)-5-cyclopropyl-1*H*-pyrazole-4-carbonyl]guanidine; [1-(2-trifluoromethyl-4-fluorophenyl)-5-cyclopropyl-1*H*-pyrazole-4-carbonyl]guanidine; [1-(2-bromophenyl)-5-cyclopropyl-1*H*-pyrazole-4-carbonyl]guanidine; [1-(2-fluorophenyl)-5-cyclopropyl-1*H*-pyrazole-4-carbonyl]guanidine; [1-(2-chloro-5-methoxyphenyl)-5-cyclopropyl-1*H*-pyrazole-4-carbonyl]guanidine; [1-(2-chloro-4-methylaminosulfonylphenyl)-5-cyclopropyl-1*H*-pyrazole-4-carbonyl]guanidine; [1-(2,5-dichlorophenyl)-5-cyclopropyl-1*H*-pyrazole-4-carbonyl]guanidine; [1-(2,3-dichlorophenyl)-5-cyclopropyl-1*H*-pyrazole-4-carbonyl]guanidine; [1-(2-chloro-5-aminocarbonylphenyl)-5-cyclopropyl-1*H*-pyrazole-4-carbonyl]guanidine; [1-(2-chloro-5-aminosulfonylphenyl)-5-cyclopropyl-1*H*-pyrazole-4-carbonyl]guanidine; [1-(2-fluoro-6-trifluoromethylphenyl)-5-cyclopropyl-1*H*-pyrazole-4-carbonyl]guanidine; [1-(2-chloro-5-methylsulfonylphenyl)-5-cyclopropyl-1*H*-pyrazole-4-carbonyl]guanidine; [1-(2-chloro-5-dimethylaminosulfonylphenyl)-5-cyclopropyl-1*H*-pyrazole-4-carbonyl]guanidine; [1-(2-trifluoromethyl-4-chlorophenyl)-5-cyclopropyl-1*H*-pyrazole-4-carbonyl]guanidine; [1-(2-chlorophenyl)-5-methyl-1H-pyrazole-4-carbonyl]guanidine; [5-methyl-1-(2-trifluoromethylphenyl)-1H-pyrazole-4-carbonyl]guanidine; [5-ethyl-1-phenyl-1H-pyrazole-4-carbonyl]guanidine; [5-cyclopropyl-1-(2-trifluoromethylphenyl)-1H-pyrazole-4-carbonyl]guanidine; [5-cyclopropyl-1-phenyl-1 H-pyrazoie-4-carbonyl]guanidine; [5-cyclopropyl-1-(2,6-dichlorophenyl)-1H-pyrazole-4-carbonyl]guanidine; and pharmaceutically acceptable salts, hydrates, and solvates thereof.

In the above combination compositions, combination methods and kits preferred glycogen phosphorylase inhibitors are

5-chloro-1 H-indole-2-carboxylic acid [(1S)-((R)-hydroxy-dimethylcarbamoyl-methyl)-2-phenyl-ethyl]-amide,
5,6-dichloro-1H-indole-2-carboxylic acid {(1S)-[(R)-hydroxy-(methoxy-methyl-carbamoyl)-methyl]-2-phenyl-ethyl}-amide,
5-chloro-1 H-indole-2-carboxylic acid {(1S)-[(R)-hydroxy-(methoxy-methylcarbamoyl)-methyl]-2-phenyl-ethyl}-

amide,
5-chloro-1H-indole-2-carboxylic acid ((1S)-{(R)-hydroxy-[(2-hydroxy-ethyl)-methyl-carbamoyl]-methyl}-2-phenyl-ethyl)-amide,
5-chloro-1 H-indole-2-carboxylic acid {(1S)-[(R)-hydroxy-(methyl-pyridin-2-yl-carbamoyl)-methyl]-2-phenyl-ethyl}-amide,
5-chloro-1 H-indole-2-carboxylic acid ((1 S)-{(R)-hydroxy-[methyl-(2-pyridin-2-yl-ethyl)-carbamoyl]-methyl}-2-phenyl-ethyl)-amide,
5-chloro-1H-indole-2-carboxylic acid [(1S)-benzyl-(2R)-hydroxy-3-(4-methyl-piperazin-1-yl)-3-oxo-propyl]-amide hydrochloride,
5-chloro-1H-indole-2-carboxylic acid [(1 S)-benzyl-(2R)-hydroxy-3-(3-hydroxy-azetidin-1-yl)-3-oxo-propyl]-amide,
5-chloro-1H-indole-2-carboxylic acid ((1 S)-benzyl-(2R)-hydroxy-3-isoxazolidin-2-yl-3-oxo-propyl)-amide,
5-chloro-1 H-indole-2-carboxylic acid ((1 S)-benzyl-(2R)-hydroxy-3-[1,2]oxazinan-2-yl-3-oxo-propyl)-amide,
5-chloro-1 H-indole-2-carboxylic acid [(1 S)-benzyl-(2R)-hydroxy-3-((3S)-hydroxy-pyrrolidin-1-yl)-3-oxo-propyl]-amide,
5-chloro-1 H-indole-2-carboxylic acid [(1 S)-benzyl-3-((3S,4S)-dihydroxypyrrolidin-1-yl)-(2R)-hydroxy-3-oxo-propyl]-amide,
5-chloro-1 H-indole-2-carboxylic acid [(1S)-benzyl-3-((3R,4S)-dihydroxypyrrolidin-1-yl)-(2R)-hydroxy-3-oxo-propyl]-amide,
5-chloro-1H-indole-2-carboxylic acid ((1S)-benzyl-(2R)-hydroxy-3-morpholin-4-yl-3-oxo-propyl)-amide,
5-chloro-1H-indole-2-carboxylic acid [(1 S)-benzyl-2-(3-hydroxyimino-pyrrolidin-1-yl)-2-oxo-ethyl]-amide,
5-chloro-1H-indole-2-carboxylic acid [2-(cis-3,4-dihydroxy-pyrrolidin-1-yl)-2-oxo-ethyl]-amide,
5-chloro-1H-indole-2-carboxylic acid [2-((3S,4S)-dihydroxy-pyrrolidin-1-yl)-2-oxo-ethyl]-amide,
5-chloro-1H-indole-2-carboxylic acid [(1 S)-benzyl-2-(cis-3,4-dihydroxy-pyrrolidin-1-yl)-2-oxo-ethyl]-amide,
5-chloro-1 H-indole-2-carboxylic acid [2-(1,1-dioxo-thiazolidin-3-yl)-2-oxo-ethyl]-amide,
5-chloro-1 H-indole-2-carboxylic acid (2-oxo-2-thiazolidin-3-yl-ethyl)-amide,
5-chloro-1 H-indole-2-carboxylic acid [(1 S)-(4-fluoro-benzyl)-2-(4-hydroxypiperidin-1-yl)-2-oxo-ethyl]-amide,
5-chloro-1 H-indole-2-carboxylic acid [(1 S)-benzyl-2-((3RS)-hydroxypiperidin-1-yl)-2-oxo-ethyl]-amide,
5-chloro-1 H-indole-2-carboxylic acid [2-oxo-2-((1 RS)-oxo-1-thiazolidin-3-yl)-ethyl]-amide,
5-chloro-1 H-indole-2-carboxylic acid [(1 S)-(2-fluoro-benzyl)-2-(4-hydroxy-piperidin-1-yl)-2-oxo-ethyl]-amide,
5-chloro-1H-indole-2-carboxylic acid [(1 S)-benzyl-2-((3S,4S)-dihydroxy-pyrrolidin-1-yl)-2-oxo-ethyl]-amide,
5-chloro-1H-indole-2-carboxylic acid [(1S)-benzyl-2-(3-hydroxy-azetidin-1-yl)-2-oxo-ethyl]-amide,
5-chloro-1H-indole-2-carboxylic acid [(1S)-benzyl-2-(3-hydroxyimino-azetidin-1-yl)-2-oxo-ethyl]-amide; and
5-chloro-1H-indole-2-carboxylic acid [(1S)-benzyl-2-(4-hydroxyimino-piperidin-1-yl)-2-oxo-ethyl]-amide.

In the above combination compositions, combination methods and kits a preferred aldose reductase inhibitor is zopolrestat: 1-phthalazineacetic acid, 3,4-dihydro-4-oxo-3-[[5-trifluoromethyl)-2-benzothiazolyl]methyl]-.

The compositions containing the compounds of the present invention described herein are useful in the treatment, reduction and/or prevention of tissue damage resulting from or which could result from ischemia or hypoxia. Accordingly, the compounds of the present invention (including the pharmaceutical compositions and combinations described herein) may be used in the manufacture of a medicament for the therapeutic applications described herein.

***Definitions***

The term "reduction" is intended to include partial prevention or prevention which, although greater than that which would result from taking no compound or from taking a placebo, is less than 100% in addition to substantially total prevention.

The term "damage resulting from ischemia or hypoxia " as employed herein refers to a condition or conditions directly associated with reduced blood flow or oxygen delivery to tissue, for example due to a clot or obstruction of blood vessels which supply blood to the subject tissue and which result, *inter alia,* in lowered oxygen transport to such tissue, impaired tissue performance, tissue dysfunction and/or necrosis and/or apoptosis. Alternatively, where blood flow or organ perfusion may be quantitatively adequate, the oxygen carrying capacity of the blood or organ perfusion medium may be reduced, e.g., a in hypoxic environment, such that oxygen supply to the tissue is lowered, and impaired tissue performance, tissue dysfunction, and/or tissue necrosis and/or apoptosis ensues.

The term "treating", "treat" or "treatment" as used herein includes preventative (e.g., prophylactic) and palliative treatment.

The term "pharmaceutically acceptable" refers to carriers, diluents, excipients, and/or salts that are compatible with the other ingredients of the formulation, and not deleterious to the recipient thereof.

The term "prodrug" refers to compounds that are drug precursors which, following administration, release the

drug *in vivo* via some chemical or physiological process (e.g., a prodrug on being brought to the physiological pH or through enzyme action is converted to the desired drug form).

The term "solvate" refers to a molecular complex of a compound represented by Formula I (including the prodrug and the pharmaceutically acceptable salt thereof) with one or more solvent molecules. Such solvent molecules are those commonly used in the pharmaceutical art, which are known to be innocuous to the recipient, e.g., water, ethanol, and the like. The term "hydrate" refers to the complex where the solvent molecule is water.

The term "aryl" refers to aromatic moieties having single (e.g., phenyl) or fused ring systems (e.g., naphthalene, anthracene, phenanthrene, etc.). Fused ring systems are also referred to herein as fully unsaturated bicyclic rings. The aryl groups may be substituted or unsubstituted.

The term "substituted aryl" refers to those aryl groups described above having one or more substituents selected from the group consisting of $(C_1-C_4)$alkyl, halo, hydroxy, thio, amino, $(C_1-C_6)$alkyloxy, $(C_1-C_6)$alkylthio, $(C_1-C_6)$alkylamino and -D-G, where D and G have the same meaning as described above for compounds of Formula (I).

The term "heteroaryl" refers to aromatic moieties containing at least one heteratom within the aromatic ring system (e.g., pyrrole, pyridine, indole, thiophene, furan, benzofuran, imidazole, pyrimidine, purine, benzimidazole, quinoline, etc.). The aromatic moiety may consist of a single or fused ring system. Fused ring systems are also referred to herein as fully unsaturated bicyclic rings. The heteroaryl groups may be substituted or unsubstituted.

The term "substituted heteroaryl" refers to those heteroaryl groups described above having at least one substituent selected from the group consisting of $(C_1-C_4)$alkyl, halo, hydroxy, thio, amino, $(C_1-C_6)$alkyloxy, $(C_1-C_6)$alkylthio, $(C_1-C_6)$alkylamino and -D-G, where D and G have the same meaning as described above for compounds of Formula (I).

Exemplary five to six membered aromatic rings optionally having one or two heteroatoms selected independently from oxygen, nitrogen and sulfur are phenyl, furyl, thienyl, pyrrolyl, oxazolyl, thiazolyl, imidazolyl, pyrazolyl, isoxazolyl, isothiazolyl, pyridinyl, pyridiazinyl, pyrimidinyl and pyrazinyl.

Exemplary partially saturated, fully saturated or fully unsaturated five to eight membered rings optionally having one to three heteroatoms selected independently from oxygen, sulfur and nitrogen are cyclopentyl, cyclohexyl, cycloheptyl, cyclooctyl and phenyl. Further exemplary five membered rings are furyl, thienyl, pyrrolyl, 2-pyrrolinyl, 3-pyrrolinyl, pyrrolidinyl, 1,3-dioxolanyl, oxazolyl, thiazolyl, imidazolyl, 2H-imidazolyl, 2-imidazolinyl, imidazolidinyl, pyrazolyl, 2-pyrazolinyl, pyrazolidinyl, isoxazolyl, isothiazolyl, 1,2-dithiolyl, 1,3-dithiolyl, 3H-1,2-oxathiolyl, 1,2,3-oxadizaolyl, 1,2,4-oxadiazolyl, 1,2,5-oxadiazolyl, 1,3,4-oxadiazolyl, 1,2,3-triazolyl, 1,2,4-trizaolyl, 1,3,4-thiadiazolyl, 3H-1,2,3-dioxazolyl, 1,2,4-dioxazolyl, 1,3,2-dioxazolyl, 1,3,4-dioxazolyl, 5H-1,2,5-oxathiazolyl and 1,3-oxathiolyl.

Further exemplary six membered rings are 2H-pyranyl, 4H-pyranyl, pyridinyl, piperidinyl, 1,2-dioxinyl, 1,3-dioxinyl, 1,4-dioxanyl, morpholinyl, 1,4-dithianyl, thiomorpholinyl, pyridazinyl, pyrimidinyl, pyrazinyl, piperazinyl, 1,3,5-triazinyl, 1,2,4-triazinyl, 1,2,3-triazinyl, 1,3,5-trithianyl, 4H-1,2-oxazinyl, 2H-1,3-oxazinyl, 6H-1,3-oxazinyl, 6H-1,2-oxazinyl, 1,4-oxazinyl, 2H-1,2-oxazinyl, 4H-1,4-oxazinyl, 1,2,5-oxathiazinyl, 1,4-oxazinyl, o-isoxazinyl, p-isoxazinyl, 1,2,5-oxathiazinyl, 1,2,6-oxathiazinyl and 1,4,2-oxadiazinyl.

Further exemplary seven membered rings are azepinyl, oxepinyl, thiepinyl and 1,4-diazepinyl.

Further exemplary eight membered rings are cyclooctenyl and cyclooctadienyl.

Exemplary bicyclic rings consisting of two fused partially saturated, fully saturated or fully unsaturated five and/or six membered rings, taken independently, optionally having one to four heteroatoms selected independently from nitrogen, sulfur and oxygen are indolizinyl, indolyl, isoindolyl, indolinyl, cyclopenta(b)pyridinyl, pyrano(3,4-b)pyrrolyl, benzofuryl, isobenzofuryl, benzo(b)thienyl, benzo(c)thienyl, 1 H-indazolyl, indoxazinyl, benzoxazolyl, anthranilyl, benzimidazolyl, benzthiazolyl, purinyl, quinolinyl, isoquinolinyl, cinnolinyl, phthalazinyl, quinazolinyl, quinoxalinyl, 1,8-naphthyridinyl, pteridinyl, indenyl, isoindenyl, naphthyl, tetralinyl, decalinyl, 2H-1-benzopyranyl, pyrido(3,4-b)-pyridinyl, pyrido(3,2-b)-pyridinyl, pyrido(4,3-b)-pyridinyl, 2H-1,3-benzoxazinyl, 2H-1,4-benzoxazinyl, 1H-2,3-benzoxazinyl, 4H-3,1-benzoxazinyl, 2H-1,2-benzoxazinyl and 4H-1,4-benzoxazinyl. The fully unsaturated bicyclic ring systems containing heteroatoms are included in the definition for "heteroaryl" and the fully unsaturated bicyclic ring systems containing no heteroatoms are included in the definition for "aryl."

The term "alkylene" refers to a saturated hydrocarbon (straight chain or branched) wherein a hydrogen atom is removed from each of the terminal carbons. Exemplary of such groups (assuming the designated length encompases the particular example) are methylene, ethylene, propylene, butylene, pentylene, hexylene, heptylene. Of course, such linking moieties may also be referred to as the substituent without the "ene" suffix (e.g., methyl) as is commonly done by those skilled in the art, and still refer to a linking group.

The term "halo" refers to chloro, bromo, iodo, or fluoro.

The term "alkyl" refers to a straight chain saturated hydrocarbon or branched saturated hydrocarbon. Exemplary of such alkyl groups (assuming the designated length encompasses the particular example) are methyl, ethyl, propyl, *iso*-propyl, butyl, *sec*-butyl, *tertiary*-butyl, pentyl, *iso*-pentyl, *neo*-pentyl, *tertiary*-pentyl, 1-methylbutyl, 2-methylbutyl, 3-methylbutyl, hexyl, *iso*-hexyl, heptyl and octyl.

The term "alkoxy" refers to a straight chain saturated alkyl or branched saturated alkyl bonded through an oxygen. Exemplary of such alkoxy groups (assuming the designated length encompasses the particular example) are

methoxy, ethoxy, propoxy, isopropoxy, butoxy, isobutoxy, tertiary butoxy, pentoxy, isopentoxy, neopentoxy, tertiary pentoxy, hexoxy, isohexoxy, heptoxy and octoxy .

As used herein the term mono-N- or di-N,N-($C_1$-$C_x$)alkyl..refers to the ($C_1$-$C_x$)alkyl moiety taken independently when it is di-N,N-($C_1$-$C_x$)alkyl...(x refers to integers).

It is to be understood that if a carbocyclic or heterocyclic moiety may be bonded or otherwise attached to a designated substrate through differing ring atoms without denoting a specific point of attachment, then all possible points are intended, whether through a carbon atom or, for example, a trivalent nitrogen atom. For example, the term "pyridyl" means 2-, 3-, or 4-pyridyl, the term "thienyl" means 2-, or 3-thienyl, and so forth.

The expression "pharmaceutically-acceptable salt" refers to nontoxic anionic salts containing anions such as (but not limited to) chloride, bromide, iodide, sulfate, bisulfate, phosphate, acetate, maleate, fumarate, oxalate, lactate, tartrate, citrate, gluconate, methanesulfonate and 4-toluene-sulfonate. Where more than one basic moiety exists the expression includes multiple salts (e.g., di-salt). The expression also refers to nontoxic cationic salts such as (but not limited to) sodium, potassium, calcium, magnesium, ammonium or protonated benzathine (N,N'-dibenzylethylenediamine), choline, ethanolamine, diethanolamine, ethylenediamine, meglamine (N-methyl-glucamine), benethamine (N-benzylphenethylamine), piperazine or tromethamine (2-amino-2-hydroxymethyl-1,3-propanediol).

As used herein, the expressions "reaction-inert solvent" and "inert solvent" refers to a solvent or mixture of solvents which does not interact with starting materials, reagents, intermediates or products in a manner which adversely affects the yield of the desired product.

The chemist of ordinary skill will recognize that certain compounds of this invention will contain one or more atoms that may be in a particular stereochemical or geometric configuration, giving rise to stereoisomers and configurational isomers. All such isomers and mixtures thereof are included in this invention. Hydrates of the compounds of this invention are also included.

DMF means N,N-dimethylformamide. DMSO means dimethyl sulfoxide. THF means tetrahydrofuran.

The subject invention also includes isotopically-labelled compounds, which are identical to those recited in Formula I, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, fluorine and chlorine, such as $^2H$, $^3H$, $^{13}C$, $^{14}C$, $^{15}N$, $^{18}O$, $^{17}O$, $^{31}P$, $^{32}P$, $^{35}S$, $^{18}F$, and $^{36}Cl$, respectively. Compounds of the present invention (including the prodrugs thereof and the pharmaceutically acceptable salts of the compounds and the prodrugs) which contain the aforementioned isotopes and/or other isotopes of other atoms are within the scope of this invention. Certain isotopically-labelled compounds of the present invention, for example those into which radioactive isotopes such as $^3H$ and $^{14}C$ are incorporated, are useful in drug and/or substrate tissue distribution assays. Tritiated, i.e., $^3H$, and carbon-14, i.e., $^{14}C$, isotopes are particularly preferred for their ease of preparation and detectability. Further, substitution with heavier isotopes such as deuterium, i.e., $^2H$, may afford certain therapeutic advantages resulting from greater metabolic stability, for example increased *in vivo* half-life or reduced dosage requirements and, hence, may be preferred in some circumstances. Isotopically labelled compounds of Formula (I) of this invention and prodrugs thereof can generally be prepared by carrying out the procedures disclosed in the Schemes and/or in the Examples below, by substituting a readily available isotopically labelled reagent for a non-isotopically labelled reagent.

Other features and advantages will be apparent from this description and claims that describe the invention.

## DETAILED DESCRIPTION OF THE INVENTION

In general the compounds of this invention can be made by processes which include processes analogous to those known in the chemical arts, particularly in light of the description contained herein. Certain processes for the manufacture of the compounds of this invention are illustrated by the following reaction schemes. Other processes are described in the experimental section.

SCHEME I

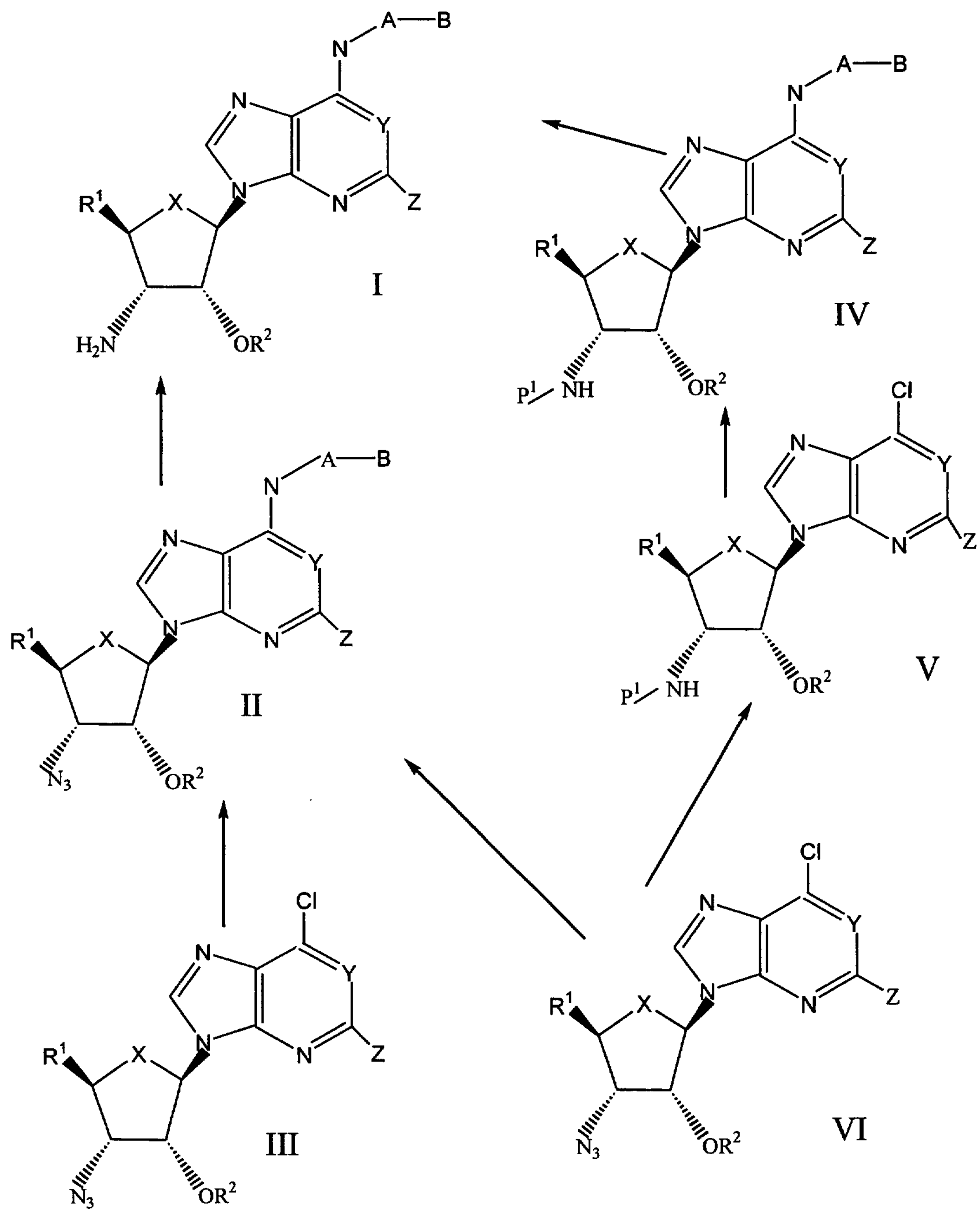

$R^1$ = ester

$R^1$ = amide

In general, the compounds of this invention can be made by coupling the desired chloropurine riboside and desired amine followed by azide reduction. The following text which is keyed to SCHEMES I, II and III depicted herein, provides a more detailed description.

According to reaction SCHEME I, the desired Formula I compounds may be prepared by reduction of the azide in the corresponding Formula II compound. Typically the reduction is accomplished by combining the Formula II compound with a trialkyl or triaryl phosphine, preferably triphenyl phosphine, in a reaction inert solvent such as

tetrahydrofuran, at temperatures of about 0°C to about 65°C, typically at ambient temperature, for about thirty minutes to about two hours. The reaction is then treated with a base, preferably an amine base, most preferably ammonium hydroxide for about six hours to about forty-eight hours at a temperature of about 0°C to about 65°C, preferably at ambient temperature.

The desired Formula II compound wherein $R^1$ is an ester may be prepared from the appropriate Formula III compound and amine derivative ($H_2N$-A-B, where A and B are as defined above). Typically, the condensation reaction is run in a polar solvent, such as ethanol, in the presence of a base, preferably an amine base, most preferably triethylamine at elevated temperatures of about 40 °C to about 75°C for about two hours to about twenty-four hours.

Analogously, the desired Formula II compound wherein $R^1$ is an amide may be prepared from the appropriate Formula VI compound and amine derivative ($H_2N$-A-B, where A and B are as defined above). Typically, the condensation reaction is run in a polar solvent, such as ethanol, in the presence of a base, preferably an amine base, most preferably triethylamine at elevated temperatures of about 40 °C to about 75°C for about two hours to about twenty-four hours.

The Formula II amide may be prepared from the corresponding Formula III ester by consecutive amine additions. Typically, the appropriate amine ($H_2N$-A-B) is added to the Formula III ester in the presence of an amine base, preferably triethyl amine, at a temperature of about 15 °C to about 50 °C for about one hour to about twenty-four hours in a polar solvent such as methanol. A second amine (e.g., N-methyl amine) is then added in excess and the reaction is stirred at room temperature for about 2 to about 24 hours.

Some of the methods useful for the preparation of the compounds described herein may require protection of remote functionality (e.g., primary amine, secondary amine, carboxyl in Formula I precursors). The need for such protection will vary depending on the nature of the remote functionality and the conditions of the preparation methods. The need for such protection is readily determined by one skilled in the art. The use of such protection/deprotection methods is also within the skill in the art. For a general description of protecting groups and their use, see T.W. Greene, *Protective Groups in Organic Synthesis,* John Wiley & Sons, New York, 1991.

Thus, for example, in an alternative reaction sequence the desired Formula I compound may be prepared from the corresponding Formula VI compound by protection and amine addition followed by deprotection. Thus, the Formula VI compound undergoes azide reduction. Typically the reduction is accomplished by combining the Formula VI compound with a trialkyl or triaryl phosphine, preferably triphenyl phosphine, in a reaction inert solvent such as tetrahydrofuran, at temperatures of about 0 °C to about 65°C, typically at ambient temperature, for about thirty minutes to about two hours. The reaction is then treated with a base, preferably an amine base, most preferably ammonium hydroxide for about six hours to about forty-eight hours at a temperature of about 0°C to about 65 °C. Following reduction, the amine moiety is protected ($P^1$).

Preferably the amine is protected with a *tert*-butoxycarbonyl group. The protection is accomplished by treating the amine with tert-butoxycarbonyl anhydride and a base, preferably an amine base, most preferably triethylamine, in an anhydrous solvent such as dichloromethane, at ambient temperature for about five hours to about twenty-four hours.

The desired Formula IV compound is prepared from the appropriate Formula V compound and amine derivative ($H_2N$-A-B, where A and B are as defined above). Typically, the condensation reaction is run in a polar solvent, such as ethanol, in the presence of a base, preferably an amine base, most preferably triethylamine at elevated temperatures of about 40°C to about 75°C for about two hours to about twenty-four hours.

Following amine addition the desired Formula I compound may be prepared from the corresponding protected Formula IV compound by an appropriate catalyzed deprotection reaction. Typically, the protected (e.g., tertiary butoxy carbonyl protected) compound is treated with a strong acid, preferably trifluoroacetic acid at about 10°C to about 50°C, preferably at ambient temperature, for about one hour to about eight hours to remove the protecting moiety.

## SCHEME II

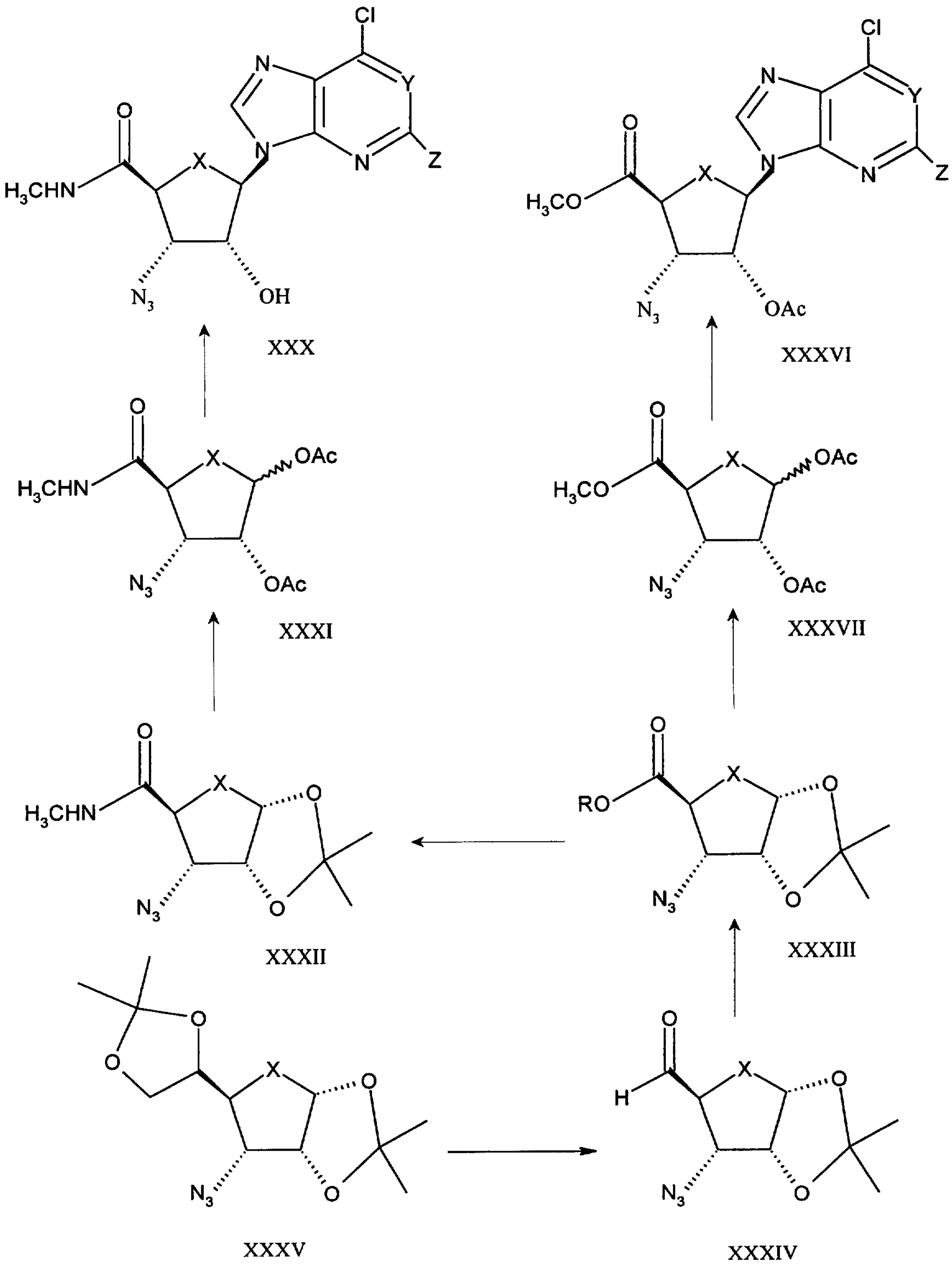

XXX

XXXVI

XXXI

XXXVII

XXXII

XXXIII

XXXV

XXXIV

According to SCHEME II, the Formula XXX compounds are prepared from a glycosidation reaction between the appropriate Formula XXXI compound and a silylated 6-chloro purine. Typically, the reaction is catalyzed by a Lewis acid, preferably trimethylsilyltriflate, in a reaction inert solvent, such as dichloroethane or acetonitrile, at temperatures from about 30°C to about 75°C, typically at about 60°C for about thirty minutes to about six hours.

The desired Formula XXXI compounds may be prepared by an acid catalyzed hydrolysis of the appropriate

Formula XXXII compound. Typically the acid is a strong mineral acid, preferably sulfuric acid, in a protic solvent mixture of acetic acid and acetic anhydride at a temperature of about 5°C to about 40°C for about two hours to about twenty-four hours.

Analogously, the desired Formula XXXVI compounds may be prepared from the appropriate Formula XXXIII compound using the glycosidation and hydrolysis reactions described above.

The desired Formula XXXII compound is prepared from the appropriate Formula XXXIII compound by activation of the carboxylic acid followed by reaction with an amine. Typically, the Formula XXXIII compound may be activated by conversion to an acid chloride by, for example, treatment with oxalyl chloride in a non-polar aprotic solvent, preferably dichloromethane with a catalytic amount of dimethyl formamide, at a temperature of about 0°C with warming to ambient temperature for about two hours to about eight hours. The acid chloride is then treated with excess of the appropriate amine at a temperature of about 0°C to about 30°C.

The desired Formula XXXIII compound is prepared by oxidation of the appropriate Formula XXXIV compound. Generally the oxidant is ruthenium tetroxide, prepared using a catalytic amount of ruthenium trichloride and a stoichiometric amount of sodium periodate in a solvent mixture of chloroform, acetonitrile and water. The reaction is conveniently performed at ambient temperature for about four hours to about twenty-four hours.

The desired Formula XXXIV compound is prepared from the appropriate Formula XXXV compound by treatment with periodic acid which hydrolyzes the isopropylidene group and cleaves the glycol to furnish the aldehyde. The reaction is run in ethereal solvents, typically diethyl ether conveniently at ambient temperature for about two hours to about twenty-four hours.

The desired Formula XXXV compound is prepared from the corresponding hydroxyl compound by activation of the hydroxyl group and displacement with azide ion. Typically, activation is achieved by converting the hydroxyl group to the corresponding triflate derivative by reaction with triflic anhydride in the presence of an amine base, preferably pyridine at about -30 °C to about 0°C for about thirty minutes to about two hours. The resulting triflate is treated with an alkali metal azide, preferably sodium azide, in a polar aprotic solvent, preferably dimethylformamide at about ambient temperature to about 50°C for about six hours to about twenty-four hours.

**SCHEME III**

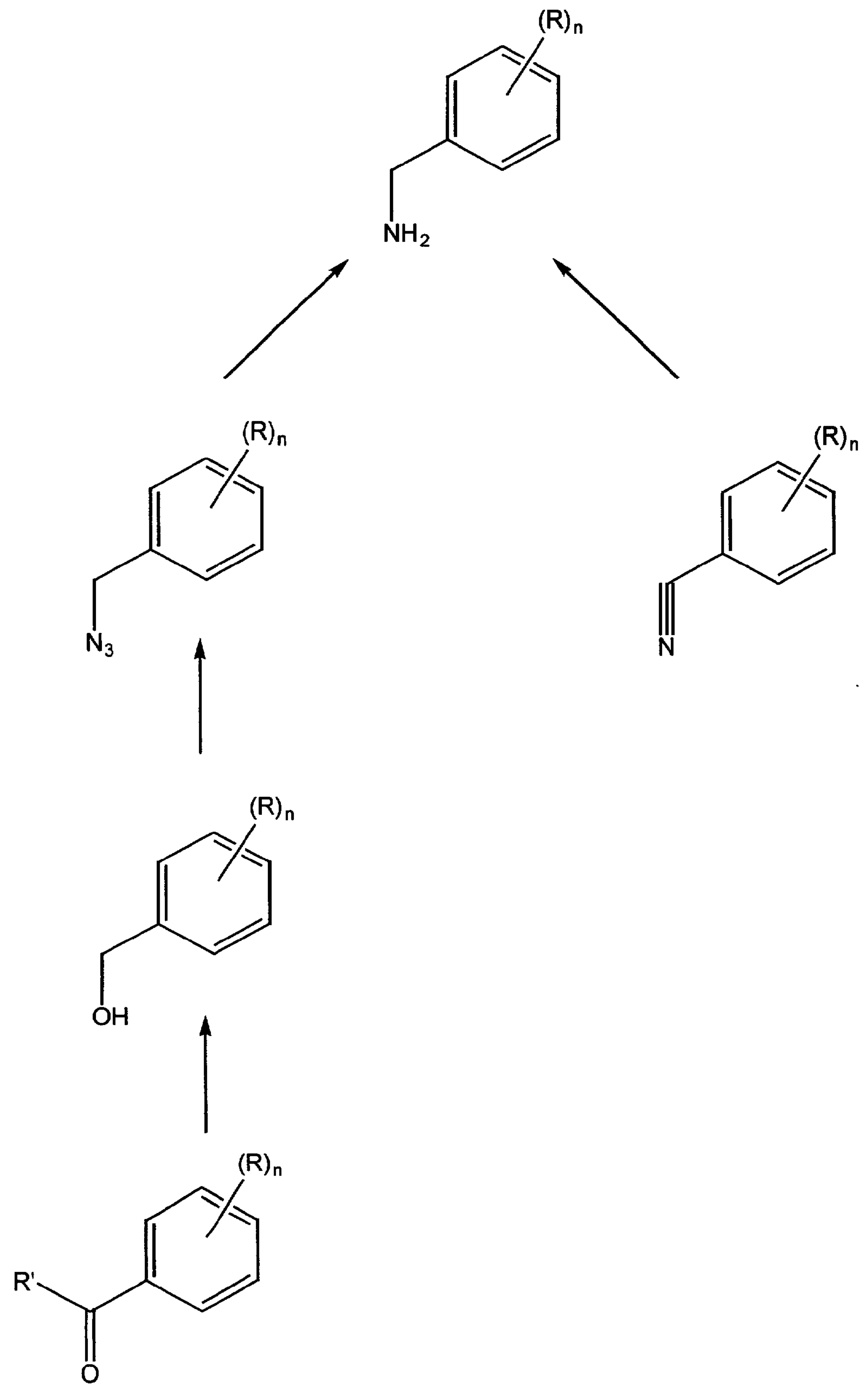

SCHEME III depicts two potential synthetic routes that may be used to prepare the benzyl amine compounds

which may be coupled to the chloropurine riboside compound as depicted in SCHEME I. The desired benzyl amine may be prepared via reduction of the corresponding benzonitrile. Generally, the reduction is accomplished using a reducing agent such as lithium aluminum hydride under anhydrous conditions. The amine is then isolated as its acid salt. For a more detailed description of the reaction conditions see Preparation M in the Examples. Alternatively, the benzyl amine may be prepared from the corresponding azide which is prepared from the more commonly available benzaldehyde or benzyl alcohol. The azide may be prepared by reacting the corresponding benzyl alcohol with diphenylphosphoryl azide in the presence of a base, such as 1,8-diazabicyclo[5.4.0]undec-7-ene. The resulting azide is then reduced to the desired amine by reacting the azide with triphenylphospine. For a more detailed description of the reaction conditions see Preparations P and N in the Examples.

Those skilled in the art will appreciate that the schemes and discussions above serve as illustrative examples and that other modifications to the compounds may be realized by using alternative starting materials (e.g., phenethylamine instead of benzyl amine) or using conventional chemistry well-known to those skilled in the art. For example, ester or carboxylic acid groups may generally be converted to an amide group by coupling the ester or acid with the appropriate amine in the presence of a suitable coupling agent. A suitable coupling agent is one that transforms a carboxylic acid into a reactive species that forms an amide linkage on reaction with an amine. The coupling agent may be a reagent that effects this condensation in a one pot process when mixed together with the carboxylic acid and amine. Exemplary coupling reagents are 1-(3-dimethylaminopropyl)-3-ethylcarbodiimide hydrochloride-hydroxybenzotriazole (EDC/HOBT), dlcyclohexylcarbodiimide/hydroxy-benzotriazole(HOBT), 2-ethoxy-1-ethoxycarbonyl-1,2-dihydroquinoline (EEDQ), and diethylphosphorylcyanide. The coupling is performed in an inert solvent, preferably an aprotic solvent at a temperature of about -20°C to about 50°C for about one to about forty-eight hours, in the presence of excess amine as base. Exemplary solvents include acetonitrile, dichloromethane, dimethylformamide, chloroform and mixtures thereof.

The coupling agent may also be that agent that converts the carboxylic acid to an activated intermediate which is isolated and/or formed in a first step and allowed to react with the amine in a second step. Suitable coupling agents and activated intermediates include thionyl chloride or oxalyl chloride to form the acid chloride, cyanuric fluoride to form an acid fluoride or an alkyl chloroformate such as isobutyl or isopropenyl chloroformate or propanephosphonic anhydride (propanephosphonic acid anhydride, PPA) (with a tertiary amine base) to form a mixed anhydride of the carboxylic acid, or carbonyldiimidazole to form an acylimidazole. If the coupling agent is oxalyl chloride, it is advantageous to employ a small amount of dimethylformamide as cosolvent with another solvent (such as dichloromethane) to catalyze the formation of the acid chloride. This activated acid derivative may be coupled by mixing with excess amine in an appropriate solvent together with an appropriate base. Appropriate solvent/base combinations include, for example, dichloromethane, dimethylformamide or acetonitrile or mixtures thereof in the presence of excess amine as base. Other appropriate solvent/base combinations include water or a ($C_1$-$C_5$)alcohol or a mixture thereof together with a cosolvent such as dichloromethane, tetrahydrofuran or dioxane and a base such as sodium, potassium or lithium hydroxide in sufficient quantity to consume the acid liberated in the reaction. Use of these coupling agents and appropriate selection of solvents and temperatures are known to those skilled in the art or can be readily determined from the literature. These and other exemplary conditions useful for coupling carboxylic acids are described in Houben-Weyl, Vol XV, part II, E. Wunsch, Ed., G. Theime Verlag, 1974, Stuttgart; M. Bodansky, Principles of Peptide Synthesis, Springer-Verlag, Berlin 1984; and The Peptides, Analysis, Synthesis and Biology (ed. E. Gross and J. Meienhofer), vols 1-5 (Academic Press, NY 1979-1983).

The starting materials and reagents for the above described compounds are readily available or can be easily synthesized by those skilled in the art using conventional methods of organic synthesis. For example, many of the compounds used herein are related to, or are derived from compounds found in nature, in which there is a large scientific interest and commercial need, and accordingly many such compounds are commercially available or are reported in the literature or are easily prepared from other commonly available substances by methods which are reported in the literature. The nitriles, caboxylic acids and amines depicted in Scheme III may be purchased from commercial suppliers such as Aldrich Chemicals Co. (Milwaukee, WI), Lancaster Synthesis, Inc. (Windham, NH) and Acros Organics (Fairlawn, NJ).

Some of the compounds of this invention have asymmetric carbon atoms and can therefore exist as enantiomers or diastereomers. Diasteromeric mixtures can be separated into their individual diastereomers on the basis of their physical chemical differences by methods known *per se*, for example, by chromatography and/or fractional crystallization. Enantiomers can be separated by converting the enantiomeric mixture into a diasteromeric mixture by reaction with an appropriate optically active compound (e.g., alcohol), separating the diastereomers and converting (e.g., hydrolyzing) the individual diastereomers to the corresponding pure enantiomers. All such isomers, including diastereomers, enantiomers and mixtures thereof are considered as part of this invention. Also, some of the compounds of this invention are atropisomers (e.g., substituted biaryls) and are considered as part of this invention.

Those skilled in the art will recognize that the compounds of Formula (I) can exist in several tautomeric forms. All such tautomeric forms are considered as part of this invention. For example all enol-keto forms of the compounds

of Formula (I) are included in this invention.

Some of the compounds of this invention are acidic and they form a salt with a pharmaceutically acceptable cation. Some of the compounds of this invention are basic and they form a salt with a pharmaceutically acceptable anion. All such salts, including di-salts are within the scope of this invention and they can be prepared by conventional methods. For example, they can be prepared simply by contacting the acidic and basic entities, in either an aqueous, non-aqueous or partially aqueous medium. The salts are recovered either by filtration, by precipitation with a non-solvent followed by filtration, by evaporation of the solvent, or, in the case of aqueous solutions, by lyophilization, as appropriate. Consequently, the compounds of the present invention may be isolated and used *per se* or as their pharmaceutically acceptable salt.

In addition, when the compounds of this invention form metabolites, hydrates or solvates they are also within the scope of the invention.

Other cardiovascular agents (e.g., agents having a cardiovascular effect) known to those skilled in the art such as those described above in the Summary may be used in conjunction with the compounds of this invention.

In combination therapy treatment, both the compounds of this invention and the other drug therapies are administered to mammals (e.g., humans) by conventional methods.

Any NHE-1 inhibitor may be used as the second compound (second active agent) of this invention for combination therapies. The term "NHE-1 inhibitor" refers to compounds which inhibit the sodium/proton (Na+/H+) exchange transport system and hence are useful as a therapeutic or prophylactic agent for diseases caused or aggravated by the acceleration of the sodium/proton (Na+/H+) exchange transport system. Such inhibition is readily determined by those skilled in the art according to standard assays such as are described herein below and in conventional preclinical cardioprotection assays [see the *in vivo* assay in Klein, H. et al., Circulation 92:912-917 (1995); the isolated heart assay in Scholz, W. et al., Cardiovascular Research 29:260-268 (1995); the antiarrhythmic assay in Yasutake M. et al., Am. J. Physiol., 36:H2430-H2440 (1994); the NMR assay in Kolke et al., J. Thorac. Cardiovasc. Surg. 112: 765-775 (1996)]. A variety of NHE-1 inhibitors are described and referenced below; however, other NHE-1 inhibitors will be known to those skilled in the art such as are disclosed in WO99/43663 published September 2, 1999.

Accordingly, examples of NHE-1 inhibitors useful in the compositions and methods of this invention include: [1-(8-bromoquinolin-5-yl)-5-cyclopropyl-1*H*-pyrazole-4-carbonyl]guanidine; [1-(6-chloroquinolin-5-yl)-5-cyclopropyl-1*H*-pyrazole-4-carbonyl]guanidine; [1-(indazol-7-yl)-5-cyclopropyl-1*H*-pyrazole-4-carbonyl]guanidine; [1-(benzimidazol-5-yl)-5-cyclopropyl-1*H*-pyrazole-4-carbonyl]guanidine; [1-(1-isoquinolyl)-5-cyclopropyl-1*H*-pyrazole-4-carbonyl] guanidine; [5-cyclopropyl-1-(4-quinolinyl)-1*H*-pyrazole-4-carbonyl]guanidine; [5-cyclopropyl-1-(quinolin-5-yl)-1H-pyrazole-4-carbonyl]guanidine; [5-cyclopropyl-1-(quinolin-8-yl)-1H-pyrazole-4-carbonyl]guanidine; [1 -(indazol-6-yl)-5-ethyl-1*H*-pyrazole-4-carbonyl]guanidine; [1-(indazol-5-yl)-5-ethyl-1*H*-pyrazole-4-carbonyl]guanidine; [1-(benzimidazol-5-yl)-5-ethyl-1*H*-pyrazole-4-carbonyl]guanidine; [1-(1-methylbenzimidazol-6-yl)-5-ethyl-1*H*-pyrazole-4-carbonyl] guanidine; 1-(5-quinolinyl)-5-*n*-propyl-1*H*-pyrazole-4-carbonyl]guanidine; [1-(5-quinolinyl)-5-isopropyl-1*H*-pyrazole-4-carbonyl]guanidine; [5-ethyl-1-(6-quinolinyl)-1*H*-pyrazole-4-carbonyl]guanidine; [1-(2-methylbenzimidazol-5-yl)-5-ethyl-1*H*-pyrazole-4-carbonyl]guanidine; [1-(1,4-benzodioxan-6-yl)-5-ethyl-1*H*-pyrazole-4-carbonyl]guanidine; [1-(benzotriazol-5-yl)-5-ethyl-1*H*-pyrazole-4-carbonyl]guanidine; [1-(3-chloroindazol-5-yl)-5-ethyl-1*H*-pyrazole-4-carbonyl]guanidine; [1-(5-quinolinyl)-5-butyl-1*H*-pyrazole-4-carbonyl]guanidine; [5-propyl-1-(6-quinolinyl)-1*H*-pyrazole-4-carbonyl]guanidine; [5-isopropyl-1-(6-quinolinyl)-1*H*-pyrazole-4-carbonyl]guanidine; [1-(2-chloro-4-methylsulfonylphenyl)-5-cyclopropyl-1*H*-pyrazole-4-carbonyl]guanidine; [1-(2-chlorophenyl)-5-cyclopropyl-1*H*-pyrazole-4-carbonyl] guanidine; [1-(2-trifluoromethyl-4-fluorophenyl)-5-cyclopropyl-1*H*-pyrazole-4-carbonyl]guanidine; [1-(2-bromophenyl)-5-cyclopropyl-1*H*-pyrazole-4-carbonyl]guanidine; [1-(2-fluorophenyl)-5-cyclopropyl-1*H*-pyrazole-4-carbonyl]guanidine; [1-(2-chloro-5-methoxyphenyl)-5-cyclopropyl-1*H*-pyrazole-4-carbonyl]guanidine; [1-(2-chloro-4-methylaminosulfonylphenyl)-5-cyclopropyl-1*H*-pyrazole-4-carbonyl]guanidine; [1-(2,5-dichlorophenyl)-5-cyclopropyl-1*H*-pyrazole-4-carbonyl]guanidine; [1-(2,3-dichlorophenyl)-5-cyclopropyl-1*H*-pyrazole-4-carbonyl]guanidine; [1-(2-chloro-5-aminocarbonylphenyl)-5-cyclopropyl-1*H*-pyrazole-4-carbonyl]guanidine; [1-(2-chloro-5-aminosulfonylphenyl)-5-cyclopropyl-1*H*-pyrazole-4-carbonyl]guanidine; [1-(2-fluoro-6-trifluoromethylphenyl)-5-cyclopropyl-1*H*-pyrazole-4-carbonyl]guanidine; [1-(2-chloro-5-methylsulfonylphenyl)-5-cyclopropyl-1*H*-pyrazole-4-carbonyl]guanidine; [1-(2-chloro-5-dimethylaminosulfonylphenyl)-5-cyclopropyl-1*H*-pyrazole-4-carbonyl]guanidine; [1-(2-trifluoromethyl-4-chlorophenyl)-5-cyclopropyl-1*H*-pyrazole-4-carbonyl]guanidine; [1-(2-chlorophenyl)-5-methyl-1H-pyrazole-4-carbonyl]guanidine; [5-methyl-1-(2-trifluoromethylphenyl)-1H-pyrazole-4-carbonyl]guanidine; [5-ethyl-1-phenyl-1H-pyrazole-4-carbonyl]guanidine; [5-cyclopropyl-1-(2-trifluoromethylphenyl)-1H-pyrazole-4-carbonyl]guanidine; [5-cyclopropyl-1-phenyl-1 H-pyrazole-4-carbonyl]guanidine; [5-cyclopropyl-1-(2,6-dichlorophenyl)-1H-pyrazole-4-carbonyl]guanidine; and pharmaceutically acceptable salts, solvates or hydrates thereof.

Any aldose reductase inhibitor may be used as the second compound (second active agent) of this invention for combination therapies. The term aldose reductase inhibitor refers to compounds that inhibit the bioconversion of glucose to sorbitol catalyzed by the enzyme aldose reductase. Such inhibition is readily determined by those skilled in the art according to standard assays (J. Malone, Diabetes, **29**:861-864, 1980. "Red Cell Sorbitol, an Indicator of

Diabetic Control"). A variety of aldose reductase inhibitors are described and referenced below, however, other aldose reductase inhibitors will be known to those skilled in the art. The disclosures of U.S. patents listed below are hereby incorporated by reference. Also, common chemical USAN names or other designation are in parentheses where applicable, together with reference to appropriate patent literature disclosing the compound.

The activity of an aldose reductase inhibitor in a tissue can be determined by testing the amount of aldose reductase inhibitor that is required to lower tissue sorbitol (i.e., by inhibiting the further production of sorbitol consequent to blocking aldose reductase) or lower tissue fructose (by inhibiting the production of sorbitol consequent to blocking aldose reductase and consequently the production of fructose). While not wishing to be bound by any particular theory or mechanism, it is believed that an aldose reductase inhibitor, by inhibiting aldose reductase, prevents or reduces ischemic or hypoxic damage as described hereinafter.

Accordingly, examples of aldose reductase inhibitors useful in the compositions and methods of this invention include:

1. 3-(4-bromo-2-fluorobenzyl)-3,4-dihydro-4-oxo-1-phthalazineacetic acid (ponalrestat, US 4,251,528);
2. N-[[(5-trifluoromethyl)-6-methoxy-1-naphthalenyl]thioxomethyl}-N-methylglycine (tolrestat, US 4,600,724);
3. 5-[(Z,E)-β-methylcinnamylidene]-4-oxo-2-thioxo-3-thiazolideneacetic acid (epalrestat, US 4,464,382, US 4,791,126, US 4,831,045);
4. 3-(4-bromo-2-fluorobenzyl)-7-chloro-3,4-dihydro-2,4-dioxo-1(2H)-quinazolineacetic acid (zenarestat, US 4,734,419, and 4,883,800);
5. 2R,4R-6,7-dichloro-4-hydroxy-2-methylchroman-4-acetic acid (US 4,883,410);
6. 2R,4R-6,7-dichloro-6-fluoro-4-hydroxy-2-methylchroman-4-acetic acid (US 4,883,410);
7. 3,4-dihydro-2,8-diisopropyl-3-oxo-2H-1,4-benzoxazine-4-acetic acid (US 4,771,050);
8. 3,4-dihydro-3-oxo-4-[(4,5,7-trifluoro-2-benzothiazolyl)methyl]-2H-1,4-benzothiazine-2-acetic acid (SPR-210, U.S. 5,252,572);
9. N-[3,5-dimethyl-4-[(nitromethyl)sulfonyl]phenyl]-2-methylbenzeneacetamide (ZD5522, U.S. 5,270,342 and U.S. 5,430,060);
10. (S)-6-fluorospiro[chroman-4,4'-imidazolidine]-2,5'-dione (sorbinil, US 4,130,714);
11. d-2-methyl-6-fluoro-spiro(chroman-4',4'-imidazolidine)-2',5'-dione (US 4,540,704);
12. 2-fluoro-spiro(9H-fluorene-9,4'imidazolidine)2',5'-dione (US 4,438,272);
13. 2,7-di-fiuoro-spiro(9H-fluorene-9,4'imidazoiidine)2',5'-dione (US 4,436,745, US 4,438,272);
14. 2,7-di-fluoro-5-methoxy-spiro(9H-fluorene-9,4' imidazolidine)2',5'-dione (US 4,436,745, US 4,438,272);
15. 7-fluoro-spiro(5H-indenol[1,2-b]pyridine-5,3'-pyrrolidine)2,5'-dione (US 4,436,745, US 4,438,272);
16. d-cis-6'-chloro-2',3'-dihydro-2'-methyl-spiro-(imidazolidine-4,4'-4'-H-pyrano(2,3-b)pyridine)-2,5-dione (US 4,980,357);
17. spiro[imidazolidine-4,5'(6H)-quinoline]2,5-dione-3'-chloro-7,'8'-dihydro-7'-methyl-(5'-cis) (US 5,066,659);
18. (2S,4S)-6-fluoro-2',5'-dioxospiro(chroman-4,4'-imidazolidine)-2-carboxamide (US 5,447,946); and
19. 2-[(4-bromo-2-fluorophenyl)methyl]-6-fluorospiro[Isoquinoline-4(1H),3'-pyrrolidine]-1,2',3,5'(2H)-tetrone (ARI-509, US 5,037,831).

Other aldose reductase inhibitors include compounds having formula IB

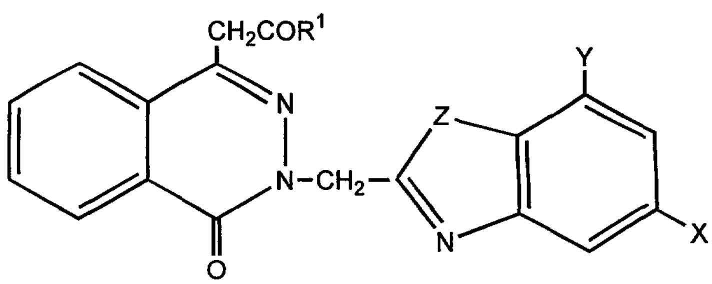

IB

and pharmaceutically acceptable salts thereof, wherein

Z is O or S;
$R^1$ is hydroxy or a group capable of being removed *in vivo* to produce a compound of formula IB wherein $R^1$ is OH; and

X and Y are the same or different and are selected from hydrogen, trifluoromethyl, fluoro, and chloro.

A preferred subgroup within the above group of aldose reductase inhibitors includes the above numbered compounds 1, 2, 3, 4, 5, 6, 9, 10, and 17, and the following compounds of Formula IB:

20. 3,4-dihydro-3-(5-fluorobenzothiazol-2-ylmethyl)-4-oxophthalazin-1-ylacetic acid [$R^1$=hydroxy; X=F; Y=H];
21. 3-(5,7-difluorobenzothiazol-2-ylmethyl)-3,4-dihydro-4-oxophthalazin-1-ylacetic acid [$R^1$=hydroxy; X=Y=F];
22. 3-(5-chlorobenzothiazol-2-ylmethyl)-3,4-dihydro-4-oxophthalazin-1-ylacetic acid [$R^1$=hydroxy; X=Cl; Y=H];
23. 3-(5,7-dichlorobenzothiazol-2-ylmethyl)-3,4-dihydro-4-oxophthalazin-1-ylacetic acid [$R^1$=hydroxy; X=Y=Cl];
24. 3,4-dihydro-4-oxo-3-(5-trifluoromethylbenzoxazol-2-ylmethyl)phthalazin-1-ylacetic acid [$R^1$=hydroxy; X=$CF_3$; Y=H];
25. 3,4-dihydro-3-(5-fluorobenzoxazol-2-ylmethyl)-4-oxophthalazin-1-ylacetic acid [$R^1$=hydroxy; X=F; Y=H];
26. 3-(5,7-difluorobenzoxazol-2-ylmethyl)-3,4-dihydro-4-oxophthalazin-1-ylacetic acid [$R^1$=hydroxy; X=Y=F];
27. 3-(5-chlorobenzoxazol-2-ylmethyl)-3,4-dihydro-4-oxophthalazin-1-ylacetic acid [$R^1$=hydroxy; X=Cl; Y=H];
28. 3-(5,7-dichlorobenzoxazol-2-ylmethyl)-3,4-dihydro-4-oxophthalazin-1-ylacetic acid [$R^1$=hydroxy; X=Y=Cl]; and
29. zopolrestat; 1-phthalazineacetic acid, 3,4-dihydro-4-oxo-3-[[5-(trifluoromethyl)-2-benzothiazolyl]methyl]- [$R^1$=hydroxy; X=trifluoromethyl; Y=H].

In compounds 20-23, and 29 Z is S. In compounds 24-28, Z is O.

Of the above subgroup, compounds 20-29 are more preferred with 29 more preferred.

An especially preferred aldose reductase inhibitor is 1-phthalazineacetic acid, 3,4-dihydro-4-oxo-3-[[5-trifluoromethyl)-2-benzothiazolyl]methyl]-.

The aldose reductase inhibitor compounds of this invention are readily available or can be easily synthesized by those skilled in the art using conventional methods of organic synthesis, particularly in view of the pertinent patent specification descriptions.

An amount of the aldose reductase inhibitor of this invention that is effective for the activities of this invention may be used. Typically, an effective dosage for the aldose reductase inhibitors for the combination compositions, methods and kits of this invention is in the range from about 0.1 mg/kg/day to about 100 mg/kg/day in single or divided doses, preferably from about 0.1 mg/kg/day to about 20 mg/kg/day in single or divided doses.

Any glycogen phosphorylase inhibitor may be used as the second compound of this invention. The term glycogen phosphorylase inhibitor refers to any substance or agent or any combination of substances and/or agents which reduces, retards, or eliminates the enzymatic action of glycogen phosphorylase. The currently known enzymatic action of glycogen phosphorylase is the degradation of glycogen by catalysis of the reversible reaction of a glycogen macromolecule and inorganic phosphate to glucose-1-phosphate and a glycogen macromolecule which is one glucosyl residue shorter than the original glycogen macromolecule (forward direction of glycogenolysis). Such actions are readily determined by those skilled in the art according to standard assays (e.g., as described hereinafter). A variety of these compounds are described in U.S. Patent No. 6,107,329. However, other glycogen phosphorylase inhibitors useful in the combinations, methods, and kits of this invention will be known to those skilled in the art.

Preferred glycogen phosphorylase inhibitors include compounds having the Formula IC

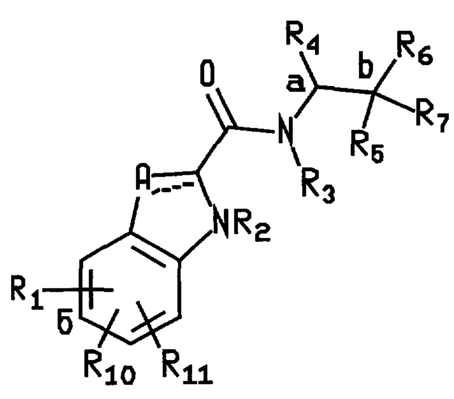

Formula IC

and the pharmaceutically acceptable salts and prodrugs thereof wherein

the dotted line (---) is an optional bond;

A is -C(H)=, -C(($C_1$-$C_4$)alkyl)= or -C(halo)= when the dotted line (---) is a bond, or A is methylene or -CH(($C_1$-$C_4$) alkyl)- when the dotted line (---) is not a bond;

$R_1$, $R_{10}$ or $R_{11}$ are each independently H, halo, 4-, 6- or 7-nitro, cyano, ($C_1$-$C_4$)alkyl, ($C_1$-$C_4$)alkoxy, fluoromethyl, difluoromethyl or trifluoromethyl;

$R_2$ is H;

$R_3$ is H or ($C_1$-$C_5$)alkyl;

$R_4$ is H, methyl, ethyl, n-propyl, hydroxy($C_1$-$C_3$)alkyl, ($C_1$-$C_3$)alkoxy($C_1$-$C_3$)alkyl, phenyl($C_1$-$C_4$)alkyl, phenylhydroxy($C_1$-$C_4$)alkyl, phenyl($C_1$-$C_4$)alkoxy($C_1$-$C_4$)alkyl, thien-2- or -3-yl($C_1$-$C_4$)alkyl or fur-2- or -3-yl($C_1$-$C_4$)alkyl wherein said $R_4$ rings are mono-, di- or tri-substituted independently on carbon with H, halo, ($C_1$-$C_4$)alkyl, ($C_1$-$C_4$) alkoxy, trifluoromethyl, hydroxy, amino or cyano; or

$R_4$ is pyrid-2-, -3- or -4-yl($C_1$-$C_4$)alkyl, thiazol-2-, -4- or -5-yl($C_1$-$C_4$)alkyl, imidazol -1-, -2-, -4- or -5-yl($C_1$-$C_4$)alkyl, pyrrol-2- or -3-yl($C_1$-$C_4$)alkyl, oxazol-2-,-4- or -5-yl-($C_1$-$C_4$)alkyl, pyrazol-3-, -4- or -5-yl($C_1$-$C_4$)alkyl, isoxazol-3-, -4- or -5-yl($C_1$-$C_4$)alkyl, isothiazol-3-, -4- or -5-yl($C_1$-$C_4$)alkyl, pyridazin-3- or -4-yl-($C_1$-$C_4$)alkyl, pyrimidin-2-, -4-, -5- or -6-yl($C_1$-$C_4$)alkyl, pyrazin-2- or -3-yl($C_1$-$C_4$)alkyl or 1,3,5-triazin-2-yl($C_1$-$C_4$)alkyl, wherein said preceding $R_4$ heterocycles are optionally mono- or di-substituted independently with halo, trifluoromethyl, ($C_1$-$C_4$)alkyl, ($C_1$-$C_4$) alkoxy, amino or hydroxy and said mono-or di-substituents are bonded to carbon;

$R_5$ is H, hydroxy, fluoro, ($C_1$-$C_5$)alkyl, ($C_1$-$C_5$)alkoxy, ($C_1$-$C_6$)alkanoyl, amino($C_1$-$C_4$)alkoxy, mono-N- or di-N, N-($C_1$-$C_4$)alkylamino($C_1$-$C_4$)alkoxy, carboxy($C_1$-$C_4$)alkoxy, ($C_1$-$C_5$)alkoxy-carbonyl($C_1$-$C_4$)alkoxy, benzyloxycarbonyl($C_1$-$C_4$)alkoxy, or carbonyloxy wherein said carbonyloxy is carbon-carbon linked with phenyl, thiazolyl, imidazolyl, 1H-indolyl, furyl, pyrrolyl, oxazolyl, pyrazolyl, isoxazolyl, isothiazolyl, pyridazinyl, pyrimidinyl, pyrazinyl or 1,3,5-triazinyl and wherein said preceding $R_5$ rings are optionally monosubstituted with halo, ($C_1$-$C_4$)alkyl, ($C_1$-$C_4$) alkoxy, hydroxy, amino or trifluoromethyl and said mono-substituents are bonded to carbon;

$R_7$ is H, fluoro or ($C_1$-$C_5$)alkyl; or

$R_5$ and $R_7$ can be taken together to be oxo;

$R_6$ is carboxy, ($C_1$-$C_8$)alkoxycarbonyl, C(O)N$R_8R_9$ or C(O)$R_{12}$,

wherein

$R_8$ is H, ($C_1$-$C_3$)alkyl, hydroxy or ($C_1$-$C_3$)alkoxy; and

$R_9$ is H, ($C_1$-$C_8$)alkyl, hydroxy, ($C_1$-$C_8$)alkoxy, methylene-perfluorinated($C_1$-$C_8$)alkyl, phenyl, pyridyl, thienyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, piperidinyl, morpholinyl, pyridazinyl, pyrimidinyl, pyrazinyl, piperazinyl or 1,3,5-triazinyl wherein said preceding $R_9$ rings are carbon-nitrogen linked; or

$R_9$ is mono-, di- or tri-substituted ($C_1$-$C_5$)alkyl, wherein said substituents are independently H, hydroxy, amino, mono-N- or di-N,N-($C_1$-$C_5$)alkylamino; or

$R_9$ is mono- or di-substituted ($C_1$-$C_5$)alkyl, wherein said substituents are independently phenyl, pyridyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, pyridinyl, piperidinyl, morpholinyl, pyridazinyl, pyrimidinyl, pyrazinyl, piperazinyl or 1,3,5-triazinyl

wherein the nonaromatic nitrogen-containing $R_9$ rings are optionally mono-substituted on nitrogen with ($C_1$-$C_6$)alkyl, benzyl, benzoyl or ($C_1$-$C_6$)alkoxycarbonyl and wherein the $R_9$ rings are optionally mono-substituted on carbon with halo, ($C_1$-$C_4$)alkyl, ($C_1$-$C_4$)alkoxy, hydroxy, amino, or mono-N- and di-N,N ($C_1$-$C_5$)alkylamino provided that no quaternized nitrogen is included and there are no nitrogen-oxygen, nitrogen-nitrogen or nitrogen-halo bonds;

$R_{12}$ is piperazin-1-yl, 4-($C_1$-$C_4$)alkylpiperazin-1-yl, 4-formylpiperazin-1-yl, morpholino, thiomorpholino, 1-oxothiomorpholino, 1,1-dioxo-thiomorpholino, thiazolidin-3-yl, 1-oxo-thiazolidin-3-yl, 1,1-dioxo-thiazolidin-3-yl, 2-($C_1$-$C_6$) alkoxycarbonylpyrrolidin-1-yl, oxazolidin-3-yl or 2(R)-hydroxymethylpyrrolidin-1-yl; or

$R_{12}$ is 3- and/or 4-mono-or di-substituted oxazetidin-2-yl, 2-, 4-, and/or 5 - mono- or di-substituted oxazolidin-3-yl, 2-, 4-, and/or 5- mono- or di- substituted thiazolidin-3-yl, 2-, 4-, and/or 5- mono- or di- substituted 1-oxothiazolidin-3-yl, 2-, 4-, and/or 5- mono- or di- substituted 1,1-dioxothiazolidin-3-yl, 3- and/or 4-, mono- or di-substituted pyrrolidin-1-yl, 3-, 4- and/or 5-, mono-, di- or tri-substituted piperidin-1-yl, 3-, 4-, and/or 5- mono-, di-, or tri-substituted piperazin-1-yl, 3-substituted azetidin-1-yl, 4- and/or 5-, mono- or di-substituted 1,2-oxazinan-2-yl, 3-and/or 4-mono- or di-substituted pyrazolidin-1-yl, 4- and/or 5-, mono- or di-substituted isoxazolidin-2-yl, 4- and/or 5-, mono- and/or di-substituted isothiazolidin-2-yl wherein said $R_{12}$ substituents are independently H, halo, ($C_1$-$C_5$)-alkyl, hydroxy, amino, mono-N- or di-N,N-($C_1$-$C_5$)alkylamino, formyl, oxo, hydroxyimino, ($C_1$-$C_5$)alkoxy, carboxy, carbamoyl, mono-N-or di-N,N-($C_1$-$C_4$)alkylcarbamoyl, ($C_1$-$C_4$)alkoxyimino, ($C_1$-$C_4$)alkoxymethoxy, ($C_1$-$C_6$)alkoxycarbonyl, carboxy($C_1$-$C_5$)alkyl or hydroxy($C_1$-$C_5$)alkyl;

with the proviso that if $R_4$ is H, methyl, ethyl or n-propyl $R_5$ is OH;

with the proviso that if $R_5$ and $R_7$ are H, then $R_4$ is not H, methyl, ethyl, n-propyl, hydroxy($C_1$-$C_3$)alkyl or ($C_1$-$C_3$) alkoxy($C_1$-$C_3$)alkyl and $R_6$ is C(O)N$R_8R_9$, C(O)$R_{12}$ or ($C_1$-$C_4$)alkoxycarbonyl.

Preferred glycogen phosphorylase inhibitors include compounds having the Formula ID

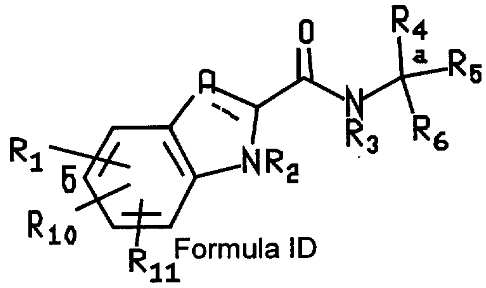

Formula ID

and the pharmaceutically acceptable salts and prodrugs thereof

wherein

the dotted line (---) is an optional bond;

A is -C(H)=, -C(($C_1$-$C_4$)alkyl)=, -C(halo)= or -N=, when the dotted line (---) is a bond, or A is methylene or -CH(($C_1$-$C_4$)alkyl)-, when the dotted line (---) is not a bond;

$R_1$, $R_{10}$ or $R_{11}$ are each independently H, halo, cyano, 4-, 6-, or 7-nitro, ($C_1$-$C_4$)alkyl, ($C_1$-$C_4$)alkoxy, fluoromethyl, difluoromethyl or trifluoromethyl;

$R_2$ is H;

$R_3$ is H or ($C_1$-$C_5$)alkyl;

$R_4$ is H, methyl, ethyl, n-propyl, hydroxy($C_1$-$C_3$)alkyl, ($C_1$-$C_3$)alkoxy($C_1$-$C_3$)alkyl, phenyl($C_1$-$C_4$)alkyl, phenylhydroxy($C_1$-$C_4$)alkyl, (phenyl)(($C_1$-$C_4$)alkoxy)($C_1$-$C_4$)alkyl, thien-2- or -3-yl($C_1$-$C_4$)alkyl or fur-2- or -3-yl($C_1$-$C_4$)alkyl wherein said $R_4$ rings are mono-, di- or tri-substituted independently on carbon with H, halo, ($C_1$-$C_4$)alkyl, ($C_1$-$C_4$) alkoxy, trifluoromethyl, hydroxy, amino, cyano or 4,5-dihydro-1H-imidazol-2-yl; or

$R_4$ is pyrid-2-, -3- or -4-yl($C_1$-$C_4$)alkyl, thiazol-2-, -4- or -5-yl($C_1$-$C_4$)alkyl, imidazol-2-, -4- or -5-yl($C_1$-$C_4$)alkyl, pyrrol-2- or -3-yl($C_1$-$C_4$)alkyl, oxazol-2-, -4- or -5-yl($C_1$-$C_4$)alkyl, pyrazol-3-, -4- or -5-yl($C_1$-$C_4$)alkyl, isoxazol-3-, -4- or -5-yl($C_1$-$C_4$)alkyl, isothiazol-3-, -4- or -5-yl($C_1$-$C_4$)alkyl, pyridazin-3- or -4-yl($C_1$-$C_4$)alkyl, pyrimidin-2-, -4-, -5- or -6-yl($C_1$-$C_4$)alkyl, pyrazin-2- or -3-yl($C_1$-$C_4$)alkyl, 1,3,5-triazin-2-yl($C_1$-$C_4$)alkyl or indol-2-($C_1$-$C_4$)alkyl, wherein said preceding $R_4$ heterocycles are optionally mono- or di-substituted independently with halo, trifluoromethyl, ($C_1$-$C_4$) alkyl, ($C_1$-$C_4$)alkoxy, amino, hydroxy or cyano and said substituents are bonded to carbon; or

$R_4$ is $R_{15}$-carbonyloxymethyl, wherein said $R_{15}$ is phenyl, thiazolyl, imidazolyl, 1H-indolyl, furyl, pyrrolyl, oxazolyl, pyrazolyl, isoxazolyl, isothiazolyl, pyridyl, pyridazinyl, pyrimidinyl, pyrazinyl or 1,3,5-triazinyl and wherein said preceding $R_{15}$ rings are optionally mono- or di-substituted independently with halo, amino, hydroxy, ($C_1$-$C_4$)alkyl, ($C_1$-$C_4$)alkoxy or trifluoromethyl and said mono- or di-substituents are bonded to carbon;

$R_5$ is H;

$R_6$ is carboxy, ($C_1$-$C_8$)alkoxycarbonyl, benzyloxycarbonyl, C(O)N$R_8R_9$ or C(O)$R_{12}$

wherein

$R_8$ is H, ($C_1$-$C_6$)alkyl, cyclo($C_3$-$C_6$)alkyl, cyclo($C_3$-$C_6$)alkyl($C_1$-$C_5$)alkyl, hydroxy or ($C_1$-$C_8$)alkoxy; and

$R_9$ is H, cyclo($C_3$-$C_8$)alkyl, cyclo($C_3$-$C_8$)alkyl($C_1$-$C_5$)alkyl, cyclo($C_4$-$C_7$)alkenyl, cyclo($C_3$-$C_7$)alkyl($C_1$-$C_5$)alkoxy, cyclo($C_3$-$C_7$)alkyloxy, hydroxy, methylene-perfluorinated($C_1$-$C_8$)alkyl, phenyl, or a heterocycle wherein said heterocycle is pyridyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, pyridinyl, piperidinyl, morpholinyl, pyridazinyl, pyrimidinyl, pyrazinyl, piperazinyl, 1,3,5-triazinyl, benzothiazolyl, benzoxazolyl, benzimidazolyl, thiochromanyl or tetrahydrobenzothiazolyl wherein said heterocycle rings are carbon-nitrogen linked; or

$R_9$ is ($C_1$-$C_6$)alkyl or ($C_1$-$C_8$)alkoxy wherein said ($C_1$-$C_6$)alkyl or ($C_1$-$C_8$)alkoxy is optionally monosubstituted with cyclo($C_4$-$C_7$)alken-1-yl, phenyl, thienyl, pyridyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, piperidinyl, morpholinyl, thiomorpholinyl, 1-oxothiomorpholinyl, 1,1-dioxothiomorpholinyl, pyridazinyl, pyrimidinyl, pyrazinyl, piperazinyl, 1,3,5-triazinyl or indolyl and wherein said ($C_1$-$C_6$)alkyl or ($C_1$-$C_8$)alkoxy are optionally additionally independently mono- or di-substituted with halo, hydroxy, ($C_1$-$C_5$)alkoxy, amino, mono-N- or di-N,N-($C_1$-$C_5$)alkylamino, cyano, carboxy, or ($C_1$-$C_4$)alkoxycarbonyl; and

wherein the $R_9$ rings are optionally mono- or di-substituted independently on carbon with halo, $(C_1-C_4)$alkyl, $(C_1-C_4)$alkoxy, hydroxy, hydroxy$(C_1-C_4)$alkyl, amino$(C_1-C_4)$alkyl, mono-N- or di-N,N-$(C_1-C_4)$alkylamino$(C_1-C_4)$alkyl, $(C_1-C_4)$alkoxy$(C_1-C_4)$alkyl, amino, mono-N- or di-N,N-$(C_1-C_4)$alkylamino, cyano, carboxy, $(C_1-C_5)$alkoxycarbonyl, carbamoyl, formyl or trifluoromethyl and said $R_9$ rings may optionally be additionally mono- or di-substituted independently with $(C_1-C_5)$alkyl or halo;

with the proviso that no quaternized nitrogen on any $R_9$ heterocycle is included;

$R_{12}$ is morpholino, thiomorpholino, 1-oxothiomorpholino, 1,1-dioxothiomorpholino, thiazolidin-3-yl, 1-oxothiazolidin-3-yl, 1,1-dioxothiazolidin-3-yl, pyrrolidin-1-yl, piperidin-1-yl, piperazin-1-yl, piperazin-4-yl, azetidin-1-yl, 1,2-oxazinan-2-yl, pyrazolidin-1-yl, isoxazolidin-2-yl, isothiazolidin-2-yl, 1,2-oxazetidin-2-yl, oxazolidin-3-yl, 3,4-dihydroisoquinolin-2-yl, 1,3-dihydroisoindol-2-yl, 3,4-dihydro-2H-quinol-1-yl, 2,3-dihydro-benzo[1,4]oxazin-4-yl, 2,3-dihydro-benzo[1,4]-thiazine-4-yl, 3,4-dihydro-2H-quinoxalin-1-yl, 3,4-dihydro-benzo[c][1,2]oxazin-1-yl, 1,4-dihydro-benzo[d][1,2]oxazin-3-yl, 3,4-dihydro-benzo[e][1,2]-oxazin-2-yl, 3H-benzo[d]isoxazol-2-yl, 3H-benzo[c]isoxazol-1-yl or azepan-1-yl,

wherein said $R_{12}$ rings are optionally mono-, di- or tri-substituted independently with halo, $(C_1-C_5)$alkyl, $(C_1-C_5)$alkoxy, hydroxy, amino, mono-N- or di-N,N-$(C_1-C_5)$alkylamino, formyl, carboxy, carbamoyl, mono-N- or di-N,N-$(C_1-C_5)$alkylcarbamoyl, $(C_1-C_6)$alkoxy$(C_1-C_3)$alkoxy, $(C_1-C_5)$alkoxycarbonyl, benzyloxycarbonyl, $(C_1-C_5)$alkoxycarbonyl$(C_1-C_5)$alkyl, $(C_1-C_4)$alkoxycarbonylamino, carboxy$(C_1-C_5)$alkyl, carbamoyl$(C_1-C_5)$alkyl, mono-N- or di-N,N-$(C_1-C_5)$alkylcarbamoyl$(C_1-C_5)$alkyl, hydroxy$(C_1-C_5)$alkyl, $(C_1-C_4)$alkoxy$(C_1-C_4)$alkyl, amino$(C_1-C_4)$alkyl, mono-N- or di-N,N-$(C_1-C_4)$alkylamino$(C_1-C_4)$alkyl, oxo, hydroxyimino or $(C_1-C_6)$alkoxyimino and wherein no more than two substituents are selected from oxo, hydroxyimino or $(C_1-C_6)$alkoxyimino and oxo, hydroxyimino or $(C_1-C_6)$alkoxyimino are on non-aromatic carbon; and

wherein said $R_{12}$ rings are optionally additionally mono- or di-substituted independently with $(C_1-C_5)$alkyl or halo;

with the proviso that when $R_6$ is $(C_1-C_5)$alkoxycarbonyl or benzyloxycarbonyl then $R_1$ is 5-halo, 5-$(C_1-C_4)$alkyl or 5-cyano and $R_4$ is (phenyl)(hydroxy)$(C_1-C_4)$alkyl, (phenyl)($(C_1-C_4)$alkoxy)$(C_1-C_4)$alkyl, hydroxymethyl or Ar$(C_1-C_2)$alkyl, wherein Ar is thien-2- or -3-yl, fur-2- or -3-yl or phenyl wherein said Ar is optionally mono- or di-substituted independently with halo; with the provisos that when $R_4$ is benzyl and $R_5$ is methyl, $R_{12}$ is not 4-hydroxy-piperidin-1-yl or when $R_4$ is benzyl and $R_5$ is methyl $R_6$ is not $C(O)N(CH_3)_2$;

with the proviso that when $R_1$ and $R_{10}$ and $R_{11}$ are H, $R_4$ is not imidazol-4-ylmethyl, 2-phenylethyl or 2-hydroxy-2-phenylethyl;

with the proviso that when both $R_8$ and $R_9$ are n-pentyl, $R_1$ is 5-chloro, 5-bromo, 5-cyano, 5$(C_1-C_5)$alkyl, 5$(C_1-C_5)$alkoxy or trifluoromethyl;

with the proviso that when $R_{12}$ is 3,4-dihydroisoquinol-2-yl, said 3,4-dihydroisoquinol-2-yl is not substituted with carboxy($(C_1-C_4)$alkyl;

with the proviso that when $R_8$ is H and R $_9$ is $(C_1-C_6)$alkyl, $R_9$ is not substituted with carboxy or $(C_1-C_4)$alkoxycarbonyl on the carbon which is attached to the nitrogen atom N of $NHR_9$; and

with the proviso that when $R_6$ is carboxy and $R_1$, $R_{10}$, $R_{11}$ and $R_5$ are all H, then $R_4$ is not benzyl, H, (phenyl)(hydroxy)methyl, methyl, ethyl or n-propyl.

In general an effective dosage for the pharmacological combination compositions of this invention, for example the ischemic damage reducing activities of combinations containing the glycogen phosphorylase inhibitor compounds of this invention, is in the range from about 0.005 to about 50 mg/kg/day, preferably from about 0.01 to about 25 mg/kg/day and most preferably from about 0.1 to about 15 mg/kg/day.

Any sorbitol dehydrogenase inhibitor may be used as the second compound of this invention. Such compounds inhibit the formation of sorbitol dehydrogenase. Such actions are readily determined by those skilled in the art according to standard assays (e.g., as described hereinafter). A variety of these compounds will be known to those skilled in the art (e.g., U.S. Patent No. 5,728,704).

The compounds of the present invention pharmacologically affect the cardioprotective effects of ischemic preconditioning by activating adenosine A-3 receptors and hence are useful as therapeutic or prophylactic agents for diseases caused or aggravated by ischemia or hypoxia, or ischemia/reperfusion for example, cardiovascular diseases [e.g., arteriosclerosis, arrhythmia (e.g. ischemic arrhythmia, arrhythmia due to myocardial infarction, myocardial stunning, myocardial dysfunction, arrhythmia after PTCA or after thrombolysis, etc.), angina pectoris, cardiac hypertrophy, myocardial infarction, heart failure (e.g. congestive heart failure, acute heart failure, cardiac hypertrophy, etc.), restenosis after PTCA, PTCI, shock (e.g. hemorrhagic shock, endotoxin shock, etc.)], renal diseases (e.g. diabetes mellitus, diabetic nephropathy, ischemic acute renal failure, etc.), organ disorders associated with ischemia or ischemic reperfusion [(e.g. heart muscle ischemic reperfusion associated disorders, acute renal failure, or disorders induced by surgical treatment such as coronary artery bypass grafting (CABG) surgeries, vascular surgeries, organ transplantation, non-cardiac surgeries or percutaneous transluminal coronary angioplasty (PTCA)], cerebrovascular diseases (e.g., ischemic stroke, hemorrhagic stroke, etc.), cerebro ischemic disorders (e.g., disorders associated with cerebral infarction, disorders caused after cerebral apoplexy as sequelae, or cerebral edema. The compounds of this invention can also be used as an agent for myocardial protection during coronary artery bypass grafting (CABG) surgeries, vascular surgeries, percutaneous transluminal coronary angioplasty (PTCA), PTCI, organ transplantation, or non-cardiac surgeries.

Preferably, the compounds of this invention can be used as agents for myocardial protection before, during, or after coronary artery bypass grafting (CABG) surgeries, vascular surgeries, percutaneous transluminal coronary angioplasty (PTCA), organ transplantation, or non-cardiac surgeries.

Preferably, the compounds of this invention can be used as agents for myocardial protection in patients presenting with ongoing cardiac ischemic events (acute coronary syndromes, e.g. myocardial infarction or unstable angina) or cerebral ischemic events (e.g., stroke).

Preferably, the compounds of this invention can be used as agents for chronic myocardial protection in patients with diagnosed coronary heart disease (e.g., previous myocardial infarction or unstable angina) or patients who are at high risk for myocardial infarction (e.g., age greater than 65 and two or more risk factors for coronary heart disease).

Accordingly, the compounds of this invention reduce mortality.

The utility of the compounds of the present invention as medical agents in the treatment of diseases, such as are detailed herein in mammals (e.g., humans), for example, myocardial protection during surgery or myocardial protection in patients presenting with ongoing cardiac or cerebral ischemic or hypoxic events or chronic cardioprotection in patients with diagnosed coronary heart disease, or at risk for coronary heart disease, cardiac dysfunction or myocardial stunning is demonstrated by the activity of the compounds of this invention in conventional preclinical cardioprotection assays [see the *in vivo* assay in Klein, H. et al., Circulation 92:912-917 (1995); the isolated heart assay in Tracey, W. R. et al., Cardiovascular Research 33:410-415 (1997); the antiarrhythmic assay in Yasutake M. et al., Am. J. Physiol., 36:H2430-H2440 (1994); the NMR assay in Kolke et al., J. Thorac. Cardiovasc. Surg. 112: 765-775 (1996)] and the additional *in vitro* and *in vivo* assays described below. Such assays also provide a means whereby the activities of the compounds of this invention can be compared with the activities of other known compounds. The results of these comparisons are useful for determining dosage levels in mammals, including humans, for the treatment of such diseases.

### Human Adenosine A1 and A3 Receptor Assays

<u>Materials</u>

Full-length human adenosine $A_1$ and $A_3$ receptor cDNA's subcloned into the eukaryotic expression vector pRcCMV (Invitrogen) were purchased from The Garvan Institute, Sydney, Australia. Chinese hamster ovary (CHO-K1) cells were obtained from the American Type Tissue Culture Collection (Rockville, MD, USA). DMEM and DMEM/F12 culture media and foetal calf serum were obtained from Gibco-BRL (Grand Island, NY, USA). The A1/A3 adenosine receptor agonist N6-(4-amino-3-[1251]iodobenzyl)adenosine ($^{125}$I-ABA) was prepared by New England Nuclear (Boston, MA, USA). Adenosine deaminase (ADA) was obtained from Boehringer Mannheim (Indianapolis, IN, USA). The phosphodiesterase inhibitor RO-20-1724 was obtained from Research Biochemicals International (Natick, MA, USA).

### Expression of Human Adenosine A1 and A3 Receptors

For stable expression studies, adenosine receptor $A_1$ and $A_3$ expression plasmids (20µg) are transfected into CHO-K1 cells, or HEK 293s cells, respectively, grown in DMEM/F12 (CHO) or DMEM (HEK 293s), with 10% foetal calf serum media, using a calcium phosphate mammalian cell transfection kit (5 Prime-3 Prime). Stable transfectants are obtained by selection in complete media containing 500µg/ml (CHO) or 700µg/ml (HEK 293s) active neomycin (G418) and screened for expression by [$^{125}$I]-ABA binding.

### Receptor Membrane Preparation

Cells stably expressing either human $A_1$ or human $A_3$ receptors are collected by centrifugation at 300 x g for 5 minutes, the supernatant is discarded and the cell pellet is resuspended in cell buffer consisting of (mmoles/L): HEPES (10), $MgCl_2$ (5), PMSF (0.1), bacitracin (100µg/ml), leupeptin (10µg/ml), DNAse I (100µg/ml), ADA (2 U/ml), pH 7.4. Crude cell membranes are prepared by repeated aspiration through a 21 gauge needle, collected by centrifugation at 60,000 x g for 10 minutes and stored in cell buffer at -80°C.

### Estimation of Compound Binding Affinity Constants ($K_i$)

Receptor membranes are resuspended in incubation buffer consisting of (mmoles/L): HEPES (10), EDTA (1),

$MgCl_2$ (5), pH 7.4. Binding reactions (10-20 μg membrane protein) are carried out for one hour at room temperature in 250 μl incubation buffer containing 0.1 nM of $^{125}$I-ABA (2200 Ci/mmol) and increasing concentrations of compound (0.1 nM - 30 μM). The reaction is stopped by rapid filtration with ice-cold PBS, through glass fibre filters (presoaked in 0.6% polyethylenimine) using a Tomtec 96-well harvester (Orange, CT, USA). Filters are counted in a Wallac Microbeta liquid scintillation counter (Gaithersberg, MD, USA). Nonspecific binding is determined in the presence of 5 μM I-ABA. Compound inhibitory constants ($K_i$) are calculated by fitting binding data via nonlinear least squares regression analysis to the equation: % Inhibition = $100/[1 + (10^C/10^X)^D]$, where X = log [compound concentration], C ($IC_{50}$) = log [compound concentration at 50% inhibtion], and D = the Hill slope. At the concentration of radioligand used in the present study (10 fold < $K_D$), $IC_{50} = K_i$.

**Assessment of Human Adenosine A3 Receptor Agonist Activity**

Adenosine A3 agonist activity is assessed by compound inhibition of isoproterenol-stimulated cAMP levels. HEK293s cells stably transfected with human A3 receptors (as described above) are washed with Phosphate Buffered Saline (PBS) (Ca/Mg-free) and detached with 1.0 mM EDTA/PBS. Cells are collected by centrifugation at 300 x g for 5 minutes and the supernatant discarded. The cell pellet is dispersed and resuspended in cell buffer (DMEM/F12 containing 10 mM HEPES, 20 μM RO-20-1724 and 1 U/ml ADA). Following preincubation of cells (100,000/well) for 10 min at 37°C, 1μM isoproterenol, with or without increasing concentrations (0.1 nM - 300 nM) test compound, and the incubation is continued for 10 min. Reactions are terminated by the addition of 1.0 N HCl followed by centrifugation at 2000 x g for 10 minutes. Sample supematants (10μl) are removed and cAMP levels determined by radioimmunoassay (New England Nuclear, Boston, MA, USA). The basal and control isoproterenol-stimulated cAMP accumulation (pmol/ml/100,000 cells) are routinely 3 and 80, respectively. Smooth curves are fitted to the data via nonlinear least squares regression analysis to the equation: % isoproterenol-stimulated cAMP = $100/[1 + (10^X/10^C)^D]$, where X = log [compound concentration], C ($IC_{50}$) = log [compound concentration at 50% inhibition], and D = the Hill slope.

As background information, it is noted that brief periods of myocardial ischemia followed by coronary artery reperfusion protects the heart from subsequent severe myocardial ischemia (Murry et al., Circulation 74:1124-1136, 1986).

The therapeutic effects of the compounds of this invention in preventing heart tissue damage resulting from an ischemic insult can be demonstrated *in vitro* along lines presented in Tracey et al. (Cardiovasc. Res., **33**:410-415, 1997), as described specifically herein. Cardioprotection, as indicated by a reduction in infarcted myocardium, can be induced pharmacologically using adenosine receptor agonists in isolated, retrogradely perfused rabbit hearts as an *in vitro* model of myocardial ischemic preconditioning (Tracey et al. (Cardiovasc. Res., **33**:410-415, 1997)). The *in vitro* test described below demonstrates that a test compound (*i.e.*, a compound as claimed herein) can also pharmacologically induce cardioprotection, *i.e.,* reduced myocardial infarct size, when administered to a rabbit isolated heart. The effects of the test compound are compared to ischemic preconditioning. The exact methodology is described below.

The protocol used for these experiments closely follows that described by Tracey et al. (Cardiovasc. Res., **33**:410-415, 1997). Male New Zealand White rabbits (3-4 kg) are anesthetized with sodium pentobarbital (30 mg/kg, i.v.). After deep anesthesia is achieved (determined by the absence of an ocular blink reflex) the animal is intubated and ventilated with 100% $O_2$ using a positive pressure ventilator. A left thoracotomy is performed, the heart exposed, and a snare (2-0 silk) is placed loosely around a prominent branch of the left coronary artery, approximately 2/3 of the distance from the apex of the heart. The heart is removed from the chest and rapidly (<30 sec) mounted on a Langendorff apparatus. The heart is retrogradely perfused in a non-recirculating manner with a modified Krebs solution (NaCl 118.5 mM, KCl 4.7 mM, Mg $SO_4$ 1.2 mM, $KH_2PO_4$ 1.2 mM, $NaHCO_3$ 24.8 mM, $CaCl_2$ 2.5 mM, and glucose 10 mM), at a constant pressure of 80 mmHg and a temperature of 37°C. Perfusate pH is maintained at 7.4-7.5 by bubbling with 95% $O_2$/5% $CO_2$. Heart temperature is tightly controlled by using heated reservoirs for the physiological solution and water jacketing around both the perfusion tubing and the isolated heart. Heart rate and left ventricular pressures are determined via a latex balloon that is inserted in the left ventricle and connected by stainless steel tubing to a pressure transducer. The intraventricular balloon is inflated to provide a systolic pressure of 80-100 mmHg, and a diastolic pressure ≤ 10 mmHg. Total coronary flow is also continuously monitored using an in-line flow probe and normalized for heart weight.

The heart is allowed to equilibrate for 30 min, over which time the heart must show stable left ventricular pressures within the parameters outlined above. If the heart rate falls below 180 bpm at any time prior to the 30 min period of regional ischemia, the heart is paced at 200 bpm for the remainder of the experiment. Ischemic preconditioning is induced by total cessation of cardiac perfusion (global ischemia) for 5 min, followed by reperfusion for 10 min. The regional ischemia is provided by tightening the snare around the coronary artery branch. Following the 30 min regional ischemia, the snare is released and the heart reperfused for an additional 120 min.

Pharmacological cardioprotection is induced by infusing the test compound at predetermined concentrations, for a 5 min period which ends 10 min before the 30 min regional ischemia. Hearts that receive test compounds do not

undergo the period of ischemic preconditioning.

At the end of the 120 min reperfusion period, the coronary artery snare is tightened, and a 0.5% suspension of fluorescent zinc cadmium sulfate particles (1-10 μm) Duke Scientific Corp.(Palo Alto, CA) is perfused through the heart; this stains all of the myocardium, except that area-at-risk for infarct development (area-at-risk). The heart is removed from the Langendorff apparatus, blotted dry, wrapped in aluminum foil and stored overnight at -20°C. The next day, the heart is sliced into 2 mm transverse sections from the apex to the top of the ventricles. The slices are stained with 1 % triphenyl tetrazolium chloride (TTC) in phosphate-buffered saline for 20 min at 37°C. Since TTC reacts with living tissue (containing NAD-dependent dehydrogenases), this stain differentiates between living (red stained) tissue, and dead tissue (unstained infarcted tissue). The infarcted area (no stain) and the area-at-risk (no fluorescent particles) are calculated for each slice of left ventricle using a precalibrated image analyzer. To normalize the ischemic injury for differences in the area-at-risk between hearts, the data is expressed as the ratio of infarct area vs. area-at-risk (%IA/AAR). All data are expressed as mean ± SE and compared statistically using a Mann-Whitney non-parametric test with a Bonferroni correction for multiple comparisons. Significance is considered as $p < 0.05$.

The results from the above *in vitro* test demonstrate that compounds of this invention induce significant cardioprotection relative to the control group.

The therapeutic effects of the compounds of this invention in preventing heart tissue damage resulting from an ischemic insult can also be demonstrated *in vivo* along lines presented in Liu et al. (Circulation, Vol. 84:350-356, 1991) as described specifically herein. The *in vivo* assay tests the cardioprotection of the test compound relative to the control group which receives saline vehicle. Cardioprotection, as indicated by a reduction in infarcted myocardium, can be induced pharmacologically using intravenously administered adenosine receptor agonists in intact, anesthetized rabbits studied as an *in vivo* model of myocardial ischemic preconditioning (Liu et al., Circulation 84:350-356, 1991). The *in vivo* assay tests whether compounds can pharmacologically induce cardioprotection, i.e., reduced myocardial infarct size, when parenterally administered to intact, anesthetized rabbits. The effects of the compounds of this invention can be compared to ischemic preconditioning. The methodology is described below. <u>Surgery:</u> New Zealand White male rabbits (3-4 kg) are anesthetized with sodium pentobarbital as a bolus dose (30 mg/kg, i.v.) followed by an infusion (100 mg/kg/hr, i.v.) to maintain a surgical plane of anesthesia. A tracheotomy is performed via a ventral midline cervical incision and the rabbits are ventilated with 100% oxygen using a positive pressure ventilator. The ventilation is adjusted to maintain pH and $PCO_2$ within physiological ranges. Body temperature is held constant at 38 °C using a heating pad. Catheters are placed in the left jugular vein for drug administration and in the left carotid artery for blood pressure measurements. The hearts are then exposed through a left thoracotomy and a snare (00 silk) placed around a prominent branch of the left coronary artery approximately two-thirds of the distance from the apex of the heart. Ischemia is induced by pulling the snare tight. Releasing the snare allows the ischemic area to reperfuse. Myocardial ischemia is evidenced by regional cyanosis; reperfusion is evidenced by reactive hyperemia.

<u>Protocol:</u> Once arterial pressure and heart rate have been stable for at least 120 minutes the test is started. Ischemic preconditioning is induced by occluding the coronary artery for 5 min followed by a 10 min reperfusion. Pharmacological preconditioning is induced by infusing test compound over, for example, 5 minutes and allowing 10 minutes before further intervention. Following ischemic preconditioning, pharmacological preconditioning or no conditioning (unconditioned, vehicle control) the artery is occluded for 30 minutes and then reperfused for two hours to induce myocardial infarction.

At the end of the 2 hour reperfusion period, the hearts are quickly removed, placed on a Langendorff apparatus, and perfused for 1 minute with normal saline heated to body temperature (38°C). The silk suture used as the snare is then tied tightly to reocclude the artery and a 0.5% suspension of fluorescent zinc cadmium sulphate particles (1-10 μm) Duke Scientific Corp. (Palo Alto, CA) is infused with the perfusate to stain all of the myocardium except for the area at risk (nonfluorescent ventricle). The hearts are then removed from the apparatus, blotted dry, wrapped in aluminum foil and stored overnight at -20°C. On the following day, the ventricles are sliced into 2 mm transverse slices sections from apex to base and stained with 1% triphenyl tetrazolium chloride (TTC) in phosphate buffered saline for 20 minutes at 38°C. Since TTC reacts with living tissue (NAD-dependent dehydrogenase present), this stain differentiates between living (red stained) tissue, and dead tissue (unstained infarcted tissue). The infarcted area (no stain) and the area at risk (no fluorescent particles) are calculated for each slice of left ventricle using a pre-calibrated image analyzer. To normalize the ischemic injury for differences in the area at risk between hearts, the data is expressed as the ratio of infarct area vs. area at risk (%IA/AAR). All data are expressed as Mean±SEM and compared statistically using single factor ANOVA or Mann Whitney non parametric test. Significance is considered as $p<0.05$.

The compounds of this invention can be tested for their utility in reducing or preventing ischemic or hypoxic injury in non-cardiac tissues, for example, the brain, or the liver, utilizing procedures reported in the scientific literature. The compounds of this invention in such tests can be administered by the preferred route and vehicle of administration and at the preferred time of administration either prior to the ischemic episode, during the ischemic or hypoxic episode, following the ischemic or hypoxic episode (reperfusion period) or during any of the below-mentioned experimental stages.

The benefit of the invention to reduce ischemic or hypoxic brain damage can be demonstrated, for example, in mammals using the method of Park, et al, (Ann. Neurol. 1988;**24**:543-551). According to the procedure of Park, et al., adult male Sprague Dawley rats are anesthetized initially with 2% halothane, and thereafter by mechanical ventilation with a nitrous oxide-oxygen mixture (70%:30%) containing 0.5-1% halothane. A tracheostomy is then performed. The stroke volume of the ventilator is adjusted to maintain arterial carbon dioxide tension at approximately 35 mm Hg and adequate arterial oxygenation ($PaO_2$>90 mm Hg). Body temperature can be monitored by a rectal thermometer, and the animals can be maintained normothermic, if necessary, by external heating. The animals next undergo subtemporal craniectomy to expose the main trunk of the left middle cerebral artery (MCA) under an operating microscope, and the exposed artery is occluded with microbipolar coagulation to generate large ischemic lesions in the cerebral cortex and basal ganglia. After three hours of MCA occlusion, the rats are deeply anesthetized with 2% halothane and a thoracotomy is performed to infuse heparinized saline into the left ventricle. The effluent is collected via an incision of the right atrium. The saline washout is followed by approximately 200 ml of a 40% formaldehyde, glacial acetic acid and absolute methanol solution (FAM; 1:1:8, v/v/v), then the animals are decapitated and the head is stored in fixative for 24 hours. The brain is then removed, dissected, embedded in paraffin wax, and sectioned (approximately 100 sections of 0.2mm per brain). The sections are then stained with hematoxylin-eosin or with a combination of cresyl violet and Luxol® fast blue, and examined by light microscopy to identify and quantitate the ischemic damage using a precalibrated image analyzer. The ischemic volumes and areas are expressed in absolute units ($mm^3$ and $mm^2$) and as a percentage of the total region examined. The effect of the compounds, compositions and methods of this invention to reduce ischemic brain damage induced by MCA occlusion is noted based on a reduction in the area or volume of relative or absolute ischemic damage in the brain sections from the rats in the treatment group compared to brain sections from rats in a placebo-treated control group.

Other methods which could alternatively be utilized to demonstrate the benefit of the invention to reduce ischemic or hypoxic brain damage include those described by Nakayama, et al. in Neurology 1988,**38**:1667-1673; Memezawa, et al. in Stroke 1992,**23**:552-559; Folbergrova, et al. in Proc. Natl. Acad. Sci 1995,**92**:5057-5059; and Gotti, et al. in Brain Res. 1990,**522**:290-307.

The benefit of the compounds, compositions and methods of this invention to reduce ischemic or hypoxic liver damage can be demonstrated, for example, in mammals using the method of Yokoyama, et al. (Am. J. Physiol. 1990;**258**:G564-G570). According to the procedure of Yokoyama, et al., fasted adult male Sprague Dawley rats are anesthetized with sodium pentobarbital (40 mg/kg i.p.), then the animals are tracheotomized and mechanically ventilated with room air. The liver is extirpated and placed in an environmental chamber maintained at constant temperature (37°C), then perfused through the portal vein at a constant pressure of 15 cm $H_2O$ with a modified, hemoglobin-free Krebs-Henseleit buffer (in mM: 118 NaCl, 4.7 KCl, 27 $NaHCO_3$, 2.5 $CaCl_2$, 1.2 $MgSO_4$, 1.2 $KH_2PO_4$, 0.05 EDTA, and 11 mM glucose, plus 300 U heparin). The pH of the perfusate is maintained at 7.4 by gassing the buffer with 95% $O_2$ - 5% $CO_2$. Each liver is perfused at a flow rate of 20 ml/min in a single-pass manner for a 30 min washout and equilibration period (preischemic period), followed by a 2 hour period of global ischemia, and then a 2 hour period of reperfusion under conditions identical to the preischemic period. Aliquots (20 ml) of the perfusate are collected during the preischemic period, immediately after the occlusive ischemic period, and every 30 min of the 2 hour reperfusion period. The perfusate samples are assayed for the appearance of hepatocellular enzymes, for example, aspartate amino-transferase (AST), alanine amino-transferase (ALT), and lactate dehydrogenase (LDH), which are taken to quantitatively reflect the degree of ischemic liver tissue damage during the procedure. AST, ALT, and LDH activities in the perfusate can be determined by several methods, for example, by the refractometry method using an automatic Kodak Ektachem 500 analyzer reported by Nakano, et al. (Hepatology, **22**, 539-545 (1995)). The effect of the compounds, compositions and methods of this invention in reducing ischemic liver damage induced by occlusion is noted based on a reduction in the release of hepatocellular enzymes immediately following the occlusive period and/or during the postischemic-reperfusion period in the perfused livers from the rats in the treatment group compared to perfused livers from rats in a placebo-treated control group.

Other methods and parameters which could alternatively be utilized to demonstrate the benefit of the compounds, compositions and methods of this invention in reducing ischemic or hypoxic liver damage include those described by Nakano, et al. (Hepatology, **22**, 539-545 (1995)).

**Measurement of Human NHE-1 Inhibitory Activity**

Methodologies for measurement of human NHE-1 activity and inhibitor potency are based on those published by Watson et al., Am. J. Physiol., **24**, G229-G238, (1991), where NHE-mediated recovery of intracellular pH is measured following intracellular acidification. Thus, fibroblasts stably expressing human NHE-1 (Counillon, L. et al., Mol. Pharmacol., **44**, 1041-1045 (1993)) are plated onto collagen coated 96 well plates (50,000/well) and grown to confluence in growth media (DMEM high glucose, 10% fetal bovine serum, 50 u/ml penicillin and streptomycin). Confluent plates are incubated for 30 min at 37 °C with the pH sensitive fluorescent probe BCECF (5 μM; Molecular Probes, Eugene,

OR). BCECF loaded cells are incubated for 30 min at 37°C in acid loading media (70 mM choline chloride, 50 mM $NHCl_4$, 5 mM KCl, 1 mM $MgCl_2$, 1.8 mM $CaCl_2$, 5 mM glucose, 10 mM HEPES, pH 7.5), and then placed in a Fluorescent Imaging Plate Reader (Molecular Devices, CA). BCECF fluorescence is monitored using excitation and emission wavelengths of 485 nM and 525 nM, respectively. Intracellular acidification is initiated via rapid replacement of acid loading media with recovery media (120 mM NaCl, 5 mM KCl, 1 mM $MgCl_2$, 1.8 mM $CaCl_2$, 5 mM glucose, 10 mM HEPES, pH 7.5) ± test compound, and NHE-mediated recovery of intracellular pH is monitored as the subsequent time-dependent increase in BCECF fluorescence. The potency of human NHE-1 inhibitors is calculated as the concentration that reduces recovery of intracellular pH by 50% ($IC_{50}$).

## ALDOSE REDUCTASE INHIBITOR ASSAYS

Male Sprague-Dawley rats are rendered diabetic by injection of streptozocin at 55 mg/kg, i.v., in pH 4.5 citrate buffer. They are fed ad libitum in controlled conditions of housing, temperature and lighting. After five weeks, the rats are anesthetized with an overdose of pentobarbital, and tissues are rapidly removed and analyzed for sorbitol and fructose.

Sorbitol levels are analyzed according to the method of Donald M. Eades et al., "Rapid Analysis of Sorbitol, Galactitol, Mannitol and Myoinositol Mixtures From Biological Sources", Journal of Chromatography, **490**, 1-8, (1989).

Fructose in rat tissues is enzymatically measured using a modification of the method of Ameyama (Methods in Enzymology, **89**:20-29, 1982), in which ferricyanide is replaced by resazurin, a dye that is reduced to the highly fluorescent resorufin. The amount of resorufin fluorescence is stoichiometric with the amount of fructose oxidized by fructose dehydrogenase. The assay contains 0.1 ml neutralized 6% perchloric acid nerve extract in a final volume of 1.5 ml. Following incubation for 60 minutes at room temperature in a closed drawer, sample fluorescence is determined at an excitation of 560 nm, an emission of 580 nm with slits of 5 mm each in a Perkin-Elmer model 650-40 fluorescence spectrophotometer. Fructose concentrations are calculated by comparison with a series of known fructose standards.

### Measurement of SDH Activity

Male Sprague-Dawley rats (350-400 g) are used for these experiments. Diabetes is induced in some of the rats by a tail vein injection of streptozocin, 85 mg/kg. Twenty-four hours later, 4 groups of diabetic rats are given a single dose of the test compound (0.001 to 100 mg/kg) by oral gavage. Animals are sacrificed 4-6 hours after dosing and blood and sciatic nerves are harvested. Tissues and cells are extracted with 6% perchloric acid.

Sorbitol in erythrocytes and nerves is measured by a modification of the method of R. S. Clements et al. (Science, **166**, 1007-8, 1969). Aliquots of tissue extracts are added to an assay system that has final concentrations of reagents of 0.033 M glycine, pH 9.4, 800 mM β-nicotine adenine dinucleotide, and 4 units/ml of sorbitol dehydrogenase. After incubation for 30 minutes at room temperature, sample fluorescence is determined on a fluorescence spectrophotometer with excitation at 366 nm and emission at 452 nm. After subtracting appropriate blanks, the amount of sorbitol in each sample is determined from a linear regression of sorbitol standards processed in the same manner as the tissue extracts.

Fructose is determined by a modification of the method described by M. Ameyama, Methods in Enzymology, **89,** 20-25 (1982). Resazurin is substituted for ferricyanide. Aliquots of tissue extracts are added to the assay system, which has final concentrations of reagents of 1.2 M citric acid, pH 4.5, 13 mM resazurin, 3.3 units/ml of fructose dehydrogenase and 0.068% Triton X-100™. After incubation for 60 minutes at room temperature, sample fluorescence is determined on a fluorescence spectrophotometer with excitation at 560 nm and emission at 580 nm. After subtracting appropriate blanks, the amount of fructose in each sample is determined from a linear regression of fructose standards processed in the same manner as the tissue extracts.

SDH activity is measured by a modification of the method described by U. Gerlach, Methodology of Enzymatic Analyses, edited by H. U. Bergmeyer, **3**, 112-117 (1983). Aliquots of sera or urine are added to the assay system, which has final concentrations of reagents of 0.1 M potassium phosphate buffer, pH 7.4, 5 mM NAD, 20 mM sorbitol, and 0.7 units/ml of sorbitol dehydrogenase. After incubation for 10 minutes at room temperature, the average change in sample absorbance is determined at 340 nm. SDH activity is presented as milli$OD_{340}$ units/minute ($OD_{340}$ = optical density at 340 nm).

### Glycogen Phosphorylase Inhibitor Assays

The three different purified glycogen phosphorylase (GP) isoenzymes, wherein glycogen phosphorylase is in the activated "a" state (referred to as glycogen phosphorylase a, or the abbreviation GPa), and referred to here as human liver glycogen phosphorylase a (HLGPa), human muscle glycogen phosphorylase a (HMGPa), and human brain glycogen phosphorylase a (HBGPa), can be obtained by the following procedures.

Expression and fermentation

The HLGP and HMGP cDNAs are expressed from plasmid pKK233-2 (Pharmacia Biotech. Inc., Piscataway, New Jersey) in *E. coli* strain XL-1 Blue (Stratagene Cloning Systems, LaJolla, CA). The strain is inoculated into LB medium (consisting of 10 g tryptone, 5 g yeast extract, 5 g NaCl, and 1 ml 1N NaOH per liter) plus 100 mg/L ampicillin, 100 mg/L pyridoxine and 600 mg/L $MnCl_2$ and grown at 37°C to a cell density of $OD_{550}$= 1.0. At this point, the cells are induced with 1 mM isopropyl-1-thio-β-D-galactoside (IPTG). Three hours after induction the cells are harvested by centrifugation and cell pellets are frozen at -70°C until needed for purification.

The HBGP cDNA can be expressed by several methodologies, for example, by the method described by Crerar, et al. (J. Biol. Chem. **270**:13748-13756). The method described by Crerar, et al. (J. Biol. Chem. **270**: 13748-13756) for the expression of HBGP is as follows: the HBGP cDNA can be expressed from plasmid pTACTAC in *E. Coli* strain 25A6. The strain is inoculated into LB medium (consisting of 10 g tryptone, 5 g yeast extract, 5 g NaCl, and 1 ml 1N NaOH per liter) plus 50 mg/L ampicillin and grown overnight, then resuspended in fresh LB medium plus 50 mg/L ampicillin, and reinoculated into a 40X volume of LB/amp media containing 250 μM isopropyl-1-thio-β-D-galactoside (IPTG), 0.5 mM pyridoxine and 3 mM $MgCl_2$ and grown at 22°C for 48-50 hours. The cells can then be harvested by centrifugation and cell pellets are frozen at -70°C until needed for purification.

The HLGP cDNA is expressed from plasmid pBlueBac III (Invitrogen Corp., San Diego, CA) which is cotransfected with BaculoGold Linear Viral DNA (Pharmingen, San Diego, CA) into Sf9 cells. Recombinant virus is subsequently plaque-purified. For production of protein, Sf9 cells grown in serum-free medium are infected at a multiplicity of infection (moi) of 0.5 and at a cell density of $2x10^6$ cells/ml. After growth for 72 hours at 27 °C, cells are centrifuged, and the cell pellets frozen at -70°C until needed for purification.

Purification of Glycogen Phosphorylase expressed in *E.* coli

The *E. coli* cells in pellets described above are resuspended in 25 mM β-glycerophosphate (pH 7.0) with 0.2 mM DTT, 1 mM $MgCl_2$, plus the following protease inhibitors:

| | |
|---|---|
| 0.7 μg/mL | Pepstatin A |
| 0.5 μg/mL | Leupeptin |
| 0.2 mM | phenylmethylsulfonyl fluoride (PMSF), and |
| 0.5 mM | EDTA, |

lysed by pretreatment with 200 μg/mL lysozyme and 3 μg/mL DNAase followed by sonication in 250 mL batches for 5 x 1.5 minutes on ice using a Branson Model 450 ultrasonic cell disrupter (Branson Sonic Power Co., Danbury CT). The *E. coli* cell lysates are then cleared by centrifugation at 35,000 X g for one hour followed by filtration through 0.45 micron filters. GP in the soluble fraction of the lysates (estimated to be less than 1% of the total protein) is purified by monitoring the enzyme activity (as described in GPa Activity Assay section, below) from a series of chromatographic steps detailed below.

Immobilized Metal Affinity Chromatography (IMAC)

This step is based on the method of Luong et al (Luong et al. Journal of Chromatography, **584,** 77-84 (1992)). Five hundred mL of the filtered soluble fraction of cell lysates (prepared from approximately 160 - 250 g of original cell pellet) are loaded onto a 130 mL column of IMAC Chelating-Sepharose (Pharmacia LKB Biotechnology, Piscataway, New Jersey) which has been charged with 50 mM $CuCl_2$ and 25 mM β-glycerophosphate, 250 mM NaCl and 1 mM imidazole at pH 7 buffer. The column is washed with buffer until the $A_{280}$ returns to baseline. The sample is then eluted from the column with the same buffer containing 100 mM imidazole to remove the bound GP and other bound proteins. Fractions containing the GP activity are pooled (approximately 600 mL), and ethylenediaminetetraacetic acid (EDTA), DL-dithiothreitol (DTT), phenylmethylsulfonyl fluoride (PMSF), leupeptin and pepstatin A are added to obtain 0.3 mM, 0.2 mM, 0.2 mM, 0.5 μg/mL and 0.7 μg/mL concentrations respectively. The pooled GP is desalted over a Sephadex G-25 column (Sigma Chemical Co., St. Louis, Missouri) equilibrated with 25 mM Tris-HCl (pH 7.3), 3 mM DTT buffer (Buffer A) to remove imidazole and is stored on ice until the second chromatographic step.

5'- AMP-Sepharose Chromatography

The desalted pooled GP sample (approximately 600mL) is next mixed with 70 mL of 5'-AMP Sepharose (Pharmacia LKB Biotechnology, Piscataway, New Jersey) which has been equilibrated with Buffer A (see above). The

mixture is gently agitated for one hour at 22 °C then packed into a column and washed with Buffer A until the $A_{280}$ returns to baseline. GP and other proteins are eluted from the column with 25 mM Tris-HCl, 0.2 mM DTT and 10 mM adenosine 5'-monophosphate (AMP) at pH 7.3 (Buffer B). GP-containing fractions are pooled following identification by determining enzyme activity (described below) and visualizing the $M_r$ approximately 97 kdal GP protein band by sodium dodecyl sulfate polyacrylamide gel electrophoresis (SDS-PAGE) followed by silver staining (2D-silver Stain II "Daiichi Kit", Daiichi Pure Chemicals Co., LTD., Tokyo, Japan) and then pooled. The pooled GP is dialyzed into 25 mM β-glycerophosphate, 0.2 mM DTT, 0.3 mM EDTA, 200 mM NaCl, pH 7.0 buffer (Buffer C) and stored on ice until use.

Prior to use of the GP enzyme, the enzyme is converted from the inactive form as expressed in *E. coli* strain XL-1 Blue (designated GPb) (Stragene Cloning Systems, La Jolla, California), to the active form (designated GPa) by the procedure described in Section (A) Activation of GP below.

Purification of Glycogen Phosphorylase expressed in Sf9 cells

The Sf9 cells in pellets described above are resuspended in 25 mM β-glycerophosphate (pH 7.0) with 0.2 mM DTT, 1 mM $MgCl_2$, plus the following protease inhibitors:

| | |
|---|---|
| 0.7 μg/mL | Pepstatin A |
| 0.5 μg/mL | Leupeptin |
| 0.2 mM | phenylmethylsulfonyl fluoride (PMSF), and |
| 0.5 mM | EDTA, |

lysed by pretreatment with 3 μg/mL DNAase followed by sonication in batches for 3 x 1 minutes on ice using a Branson Model 450 ultrasonic cell disrupter (Branson Sonic Power Co., Danbury CT). The Sf9 cell lysates are then cleared by centrifugation at 35,000 X g for one hour followed by filtration through 0.45 micron filters. GP in the soluble fraction of the lysates (estimated to be 1.5% of the total protein) is purified by monitoring the enzyme activity (as described in GPa Activity Assay section, below) from a series of chromatographic steps detailed below.

Immobilized Metal Affinity Chromatography (IMAC)

Immobilized Metal Affinity Chromatography is performed as described in the section above. The pooled, desalted GP is then stored on ice until further processed.

Activation of GP

Before further chromatography, the fraction of inactive enzyme as expressed in Sf9 cells (designated GPb) is converted to the active form (designated GPa) by the following procedure described in Section (A) Activation of GP below.

Anion Exchange Chromatography

Following activation of the IMAC purified GPb to GPa by reaction with the immobilized phosphorylase kinase, the pooled GPa fractions are dialyzed against 25 mM Tris-HCl, pH 7.5, containing 0.5 mM DTT, 0.2 mM EDTA, 1.0 mM phenylmethylsulfonyl fluoride (PMSF), 1.0 μg/mL leupeptin and 1.0 μg/mL pepstatin A. The sample is then loaded onto a MonoQ Anion Exchange Chromatography column (Pharmacia Biotech. Inc., Piscataway, New Jersey). The column is washed with equilibration buffer until the $A_{280}$ returns to baseline. The sample is then eluted from the column with a linear gradient of 0-0.25 M NaCl to remove the bound GP and other bound proteins. GP-containing fractions elute between 0.1-0.2 M NaCl range, as detected by monitoring the eluant for peak protein absorbance at $A_{280}$. The GP protein is then identified by visualizing the $M_r$ approximately 97 kdal GP protein band by sodium dodecyl sulfate polyacrylamide gel electrophoresis (SDS-PAGE) followed by silver staining (2D-silver Stain II "Daiichi Kit", Daiichi Pure Chemicals Co., LTD., Tokyo, Japan) and then pooled. The pooled GP is dialyzed into 25 mM N,N-bis[2-Hydroxyethyl]-2-aminoethanesulfonic acid, 1.0 mM DTT, 0.5 mM EDTA, 5 mM NaCl, pH 6.8 buffer and stored on ice until use.

Determination of GP Enzyme Activity

A) Activation of GP: Conversion of GPb to GPa

Prior to the determination of GP enzyme activity, the enzyme is converted from the inactive form as expressed

in *E. coli* strain XL-1 Blue (designated GPb) (Stragene Cloning Systems, La Jolla, California), to the active form (designated GPa) by phosphorylation of GP using phosphorylase kinase as follows. The fraction of inactive enzyme as expressed in Sf9 cells (designated GPb) is also converted to the active form (designated GPa) by the following procedure.

GP reaction with Immobilized Phosphorylase Kinase

Phosphorylase kinase (Sigma Chemical Company, St. Louis, MO) is immobilized on Affi-Gel 10 (BioRad Corp., Melvile, NY) as per the manufacturer's instructions. In brief, the phosphorylase kinase enzyme (10 mg) is incubated with washed Affi-Gel beads (1 mL) in 2.5 mL of 100 mM HEPES and 80 mM $CaCl_2$ at pH 7.4 for 4 hours at 4°C. The Affi-Gel beads are then washed once with the same buffer prior to blocking with 50 mM HEPES and 1 M glycine methyl ester at pH 8.0 for one hour at room temperature. Blocking buffer is removed and replaced with 50 mM HEPES (pH 7.4), 1 mM β-mercaptoethanol and 0.2% $NaN_3$ for storage. Prior to use to convert GPb to GPa, the Affi-Gel immobilized phosphorylase kinase beads are equilibrated by washing in the buffer used to perform the kinase reaction, consisting of 25 mM β-glycerophosphate, 0.3 mM DTT, and 0.3mM EDTA at pH 7.8 (kinase assay buffer).

The partially purified, inactive GPb obtained from 5'-AMP-Sepharose chromatography above (from *E. coli*) or the mixture of GPa and GPb obtained from IMAC above (from Sf9 cells) is diluted 1:10 with the kinase assay buffer then mixed with the aforementioned phosphorylase kinase enzyme immobilized on the Affi-Gel beads. NaATP is added to 5 mM and $MgCl_2$ to 6 mM. The resulting mixture is mixed gently at 25°C for 30 to 60 minutes. The sample is removed from the beads and the percent activation of GPb by conversion to GPa is estimated by determining GP enzyme activity in the presence and absence of 3.3 mM AMP. The percentage of total GP enzyme activity due to GPa enzyme activity (AMP-independent) is then calculated as follows:

$$\text{\% of total HLGPa} = \frac{\text{HLGP activity - AMP}}{\text{HLGP activity + AMP}}$$

Alternately, the conversion of GPb to GPa can be monitored by isoelectric focusing, based on the shift in electrophoretic mobility that is noted following conversion of GPb to GPa. GP samples are analyzed by isoelectric focusing (IEF) utilizing the Pharmacia PfastGel System (Pharmacia Biotech. Inc., Piscataway, New Jersey) using precast gels (pl range 4-6.5) and the manufacturer's recommended method. The resolved GPa and GPb bands are then visualized on the gels by silver staining (2D-silver Stain II "Daiichi Kit", Daiichi Pure Chemicals Co., LTD., Tokyo, Japan). Identification of GPa and GPb is made by comparison to *E. coli* derived GPa and GPb standards that are run in parallel on the same gels as the experimental samples.

B) GPa Activity Assay

The disease/condition treating/preventing activities described herein of the glycogen phosphorylase inhibitor compounds of this invention can be indirectly determined by assessing the effect of the compounds of this invention on the activity of the activated form of glycogen phosphorylase (GPa) by one of two methods; glycogen phosphorylase a activity is measured in the forward direction by monitoring the production of glucose-1-phosphate from glycogen or by following the reverse reaction, measuring glycogen synthesis from glucose-1-phosphate by the release of inorganic phosphate. All reactions can be run in triplicate in 96-well microtiter plates and the change in absorbance due to formation of the reaction product is measured at the wavelength specified below in a MCC/340 MKII Elisa Reader (Lab Systems, Finland), connected to a Titertech Microplate Stacker (ICN Biomedical Co, Huntsville, Alabama).

To measure the GPa enzyme activity in the forward direction, the production of glucose-1-phosphate from glycogen is monitored by the multienzyme coupled general method of Pesce et al. [Pesce, M.A., Bodourian, S.H., Harris, R.C. and Nicholson, J.F. (1977) Clinical Chemistry 23, 1711-1717] modified as follows: 1 to 100 μg GPa, 10 units phosphoglucomutase and 15 units glucose-6-phosphate dehydrogenase (Boehringer Mannheim Biochemicals, Indianapolis, IN) are diluted to 1 mL in Buffer A (described hereinafter). Buffer A is at pH 7.2 and contains 50 mM HEPES, 100 mM KCl, 2.5 mM ethyleneglycoltetraacetic acid (EGTA), 2.5 mM $MgCl_2$, 3.5 mM $KH_2PO_4$ and 0.5 mM dithiothreitol. 20 μl of this stock is added to 80 μl of Buffer A containing 0.47 mg/mL glycogen, 9.4 mM glucose, 0.63 mM of the oxidized form of nicotinamide adenine dinucleotide phosphate ($NADP^+$). The compounds to be tested are added as 5 μL of solution in 14% dimethylsulfoxide (DMSO) prior to the addition of the enzymes. The basal rate of GPa enzyme activity in the absence of inhibitors is determined by adding 5 μL of 14% DMSO and a fully-inhibited rate of GPa enzyme activity is obtained by adding 20 μL of 50 mM of the positive control test substance, caffeine. The reaction is followed at room temperature by measuring the conversion of oxidized $NADP^+$ to reduced NADPH at 340 nm.

To measure the GPa enzyme activity in the reverse direction, the conversion of glucose-1-phosphate into glycogen plus inorganic phosphate is measured by the general method described by Engers et al. [Engers, H.D.,

Shechosky, S. and Madsen, N.B. Can. J. Biochem. **48**, 746-754 (1970)] modified as follows: 1 to 100 μg GPa is diluted to 1 mL in Buffer B (described hereinafter). Buffer B is at pH 7.2 and contains 50 mM HEPES, 100 mM KCl, 2.5 mM EGTA, 2.5 mM $MgCl_2$ and 0.5 mM dithiothreitol. 20 μL of this stock is added to 80 μL of Buffer B with 1.25 mg/mL glycogen, 9.4 mM glucose, and 0.63 mM glucose-1-phosphate. The compounds to be tested are added as 5 μL of solution in 14% DMSO prior to the addition of the enzyme. The basal rate of GPa enzyme activity in the absence of added inhibitors is determined by adding 5 μL of 14% DMSO and a fully-inhibited rate of GPa enzyme activity is obtained by adding 20 μL of 50 mM caffeine. This mixture is incubated at room temperature for 1 hour and the inorganic phosphate released from the glucose-1-phosphate is measured by the general method of Lanzetta et al. [Lanzetta, P.A., Alvarez, L.J., Reinach, P.S. and Candia, O.A,Anal. Biochem. **100**, 95-97 (1979)] modified as follows: 150 μL of 10 mg/mL ammonium molybdate, 0.38 mg/mL malachite green in 1 N HCl is added to 100 μL of the enzyme mix. After a 20 minute incubation at room temperature, the absorbance is measured at 620 nm.

The above assays carried out with a range of concentrations of test compound allows the determination of an $IC_{50}$ value (concentration of test compound required for 50% inhibition) for the *in vitro* inhibition of GPa enzyme activity by that test compound.

Administration of the compounds of this invention can be via any method which delivers a compound of this invention preferentially to the desired tissue (e.g., liver and/or cardiac tissues). These methods include oral routes, parenteral, intraduodenal routes, etc. Generally, the compounds of the present invention are administered in single (e.g., once daily) or multiple doses or via constant infusion.

The compounds of this invention are useful, for example, in reducing or minimizing damage effected directly to any tissue that may be susceptible to either ischemia/reperfusion injury or injury resulting from hypoxia (e.g., heart, brain, lung, kidney, liver, gut, skeletal muscle, retina) as the result of an ischemic or hypoxic event (e.g., myocardial infarction). The active compound is therefore usefully employed prophylactically to prevent, i.e. (prospectively or prophylactically) to blunt or stem, tissue damage (e.g., myocardial tissue) in patients who are at risk for ischemia or hypoxia (e.g., myocardial ischemia).

Generally, the compounds of this invention are administered orally, or parenterally (e.g., intravenously, intramuscularly, subcutaneously or intramedullary). Topical administration may also be indicated, for example, where the patient is suffering from gastrointestinal disorders or whenever the medication is best applied to the surface of a tissue or organ as determined by the attending physician.

As used herein, the term "effective amount" refers to an amount of compound or compounds of the present invention which is capable of inhibiting or preventing various pathological conditions and sequelae, herein described. The terms "inhibit" or "inhibiting" refers to prohibiting, treating, alleviating, ameliorating, halting, restraining, slowing or reversing the progression, or reducing the severity of a pathological condition related to or resultant from tissue damage (e.g., myocardial tissue) in patients who are at risk for ischemia or hypoxia (e.g., myocardial ischemia). As such, these methods include both medical therapeutic (acute) and/or prophylactic (prevention) administration as appropriate. The amount and timing of compounds administered will, of course, be dependent on the subject being treated, on the severity of the affliction, on the manner of administration and on the judgement of the prescribing physician. Thus, because of patient to patient variability, the dosages given below are a guideline and the physician may titrate doses of the drug to achieve the treatment that the physician considers appropriate for the patient. In considering the degree of treatment desired, the physician must balance a variety of factors such as age of the patient, presence of preexisting disease, as well as presence of other diseases (e.g., cardiovascular disease). An amount of the compound or the present invention that is effective for ischemic or hypoxic treatment or protection is preferably a dosage from about 0.001 to about 100 mg/kg/day. An especially preferred dosage is about 0.01 to about 50 mg/kg/day of a compound of this invention.

Thus, for example, in one mode of administration the compounds of this invention may be administered just prior to surgery (e.g., within twenty-four hours before surgery, for example, cardiac surgery), during and/or subsequent to surgery (e.g., within twenty-four hours after surgery) where there is risk of ischemia (e.g., myocardial ischemia). In another mode of administration, the compounds of this invention are administered with an initial loading dose (e.g., bolus injection or infusion) prior to surgery followed by a constant infusion prior to, during and post surgery. The compounds of this invention may also be administered in a chronic daily mode.

The compounds of the present invention are generally administered in the form of a pharmaceutical composition comprising at least one of the compounds of this invention together with a pharmaceutically acceptable vehicle, carrier or diluent. Thus, the compounds of this invention can be administered individually or together in any conventional oral, parenteral (e.g., intravenous, intramuscular injection), rectal or transdermal dosage form.

For oral administration a pharmaceutical composition can take the form of solutions, suspensions, tablets, pills, capsules, powders, and the like. Tablets containing various excipients such as sodium citrate, calcium carbonate and calcium phosphate are employed along with various disintegrants such as starch and preferably potato or tapioca starch and certain complex silicates, together with binding agents such as polyvinylpyrrolidone, sucrose, gelatin and acacia. Additionally, lubricating agents such as magnesium stearate, sodium lauryl sulfate and talc are often very useful

for tabletting purposes. Solid compositions of a similar type are also employed as fillers in soft and hard-filled gelatin capsules; preferred materials in this connection also include lactose or milk sugar as well as high molecular weight polyethylene glycols. When aqueous suspensions and/or elixirs are desired for oral administration, the compounds of this invention can be combined with various sweetening agents, flavoring agents, coloring agents, emulsifying agents and/or suspending agents, as well as such diluents as water, ethanol, propylene glycol, glycerin and various like combinations thereof.

For purposes of parenteral administration, solutions, for example, in sesame or peanut oil or in aqueous propylene glycol can be employed, as well as sterile aqueous solutions of the corresponding water-soluble salts. Such aqueous solutions may be suitably buffered, if necessary, and the liquid diluent first rendered isotonic with sufficient saline or glucose. These aqueous solutions are especially suitable for intravenous, intramuscular, subcutaneous and intraperitoneal injection purposes. In this connection, the sterile aqueous media employed are all readily obtainable by standard techniques well-known to those skilled in the art.

For purposes of transdermal (e.g., topical) administration, dilute sterile, aqueous or partially aqueous solutions (usually in about 0.1% to 5% concentration), otherwise similar to the above parenteral solutions, are prepared.

Methods of preparing various pharmaceutical compositions with a certain amount of active ingredient are known, or will be apparent in light of this disclosure, to those skilled in this art. For examples of methods of preparing pharmaceutical compositions, see <u>Remington's Pharmaceutical Sciences,</u> Mack Publishing Company, Easter, Pa., 16th Edition (1980).

Pharmaceutical compositions according to the invention may contain, for example, 0.0001%-95% of the compound(s) of this invention. In any event, the composition or formulation to be administered will contain a quantity of a compound(s) according to the invention in an amount effective to treat the disease/condition of the subject being treated.

Advantageously, the present invention also provides kits for use by a consumer having, or at risk of having, a disease or condition resulting from, for example, ischemia or hypoxia, which can be ameliorated by an $A_3$ agonist. Such kits include a suitable dosage form such as an injectable parenteral solution particularly adapted for intravenous or intramuscular injection and instructions describing the method of using such dosage form to reduce the risk of tissue damage to the consumer. The instructions would direct the consumer or medical personnel to administer the parenteral solution according to administration modes known to those skilled in the art. Such kits could advantageously be packaged and sold in single or multiple parenteral kit units.

The two different compounds of the combination aspect of this invention can be co-administered simultaneously or sequentially in any order, or as a single pharmaceutical composition comprising a compound of the present invention and an aldose reductase inhibitor as described above or a glycogen phosphorylase inhibitor as described above or a sorbitol dehydrogenase inhibitor or a cardiovascular agent.

Since the present invention has an aspect that relates to the treatment of the disease/conditions described herein with a combination of active ingredients which may be administered separately, the invention also relates to combining separate pharmaceutical compositions in kit form. The kit comprises two separate pharmaceutical compositions: a compound of Formula I, a prodrug thereof or a salt of such compound or prodrug and a second compound as described above. The kit comprises a container (e.g., a divided bottle or a divided foil packet). Typically, the kit comprises directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician.

An example of such a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process recesses are formed in the plastic foil. The recesses have the size and shape of the tablets or capsules to be packed. Next, the tablets or capsules are placed in the recesses and the sheet of relatively stiff material is sealed against the plastic foil at the face of the foil which is opposite from the direction in which the recesses were formed. As a result, the tablets or capsules are sealed in the recesses between the plastic foil and the sheet. Preferably the strength of the sheet is such that the tablets or capsules can be removed from the blister pack by manually applying pressure on the recesses whereby an opening is formed in the sheet at the place of the recess. The tablet or capsule can then be removed via said opening.

It may be desirable to provide a memory aid on the kit, e.g., in the form of numbers next to the tablets or capsules whereby the numbers correspond with the days of the regimen which the tablets or capsules so specified should be ingested. Another example of such a memory aid is a calendar printed on the card, e.g., as follows "First Week, Monday, Tuesday, ...etc.... Second Week, Monday, Tuesday,..." etc. Other variations of memory aids will be readily apparent. A "daily dose" can be a single tablet or capsule or several pills or capsules to be taken on a given day. Also, a daily dose of a first compound can consist of one tablet or capsule while a daily dose of the second compound can consist of several tablets or capsules and vice versa. The memory aid should reflect this.

In another specific embodiment of the invention, a dispenser designed to dispense the daily doses one at a time in the order of their intended use is provided. Preferably, the dispenser is equipped with a memory aid, so as to further facilitate compliance with the regimen. An example of such a memory aid is a mechanical counter which indicates the number of daily doses that has been dispensed. Another example of such a memory aid is a battery powered microchip memory coupled with a liquid crystal readout, or audible reminder signal which, for example, reads out the date that the last daily dose has been taken and/or reminds one when the next dose is to be taken.

The compounds of this invention generally will be administered in a convenient formulation. The following formulation examples are illustrative only and are not intended to limit the scope of the present invention.

In the formulations which follow, "active ingredient" means a compound(s) of this invention. It will be understood by those skilled in the art that the active ingredient may be formulated as a combination of agents.

| ***Formulation 1: Gelatin Capsules*** | |
|---|---|
| Hard gelatin capsules are prepared using the following: | |
| Ingredient | Quantity (mg/capsule) |
| Active ingredient | 0.25-100 |
| Starch, NF | 0-650 |
| Starch flowable powder | 0-50 |
| Silicone fluid 350 centistokes | 0-15 |

| ***Formulation 2: Tablets*** | |
|---|---|
| A tablet formulation is prepared using the ingredients below: | |
| Ingredient | Quantity (mg/tablet) |
| Active ingredient | 0.25-100 |
| Cellulose, microcrystalline | 200-650 |
| Silicon dioxide, fumed | 10-650 |
| Stearic acid | 5-15 |

The components are blended and compressed to form tablets.

| ***Formulation 3: Tablets*** | |
|---|---|
| Alternatively, tablets each containing 0.25-100 mg of active ingredients are made up as follows: | |
| Ingredient | Quantity (mg/tablet) |
| Active ingredient | 0.25-100 |
| Starch | 45 |
| Cellulose, microcrystalline | 35 |
| Polyvinylpyrrolidone (as 10% solution in water) | 4 |
| Sodium carboxymethyl cellulose | 4.5 |
| Magnesium stearate | 0.5 |
| Talc | 1 |

The active ingredient, starch, and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50° - 60°C and passed through a No. 18 mesh U.S. sieve. The sodium carboxymethyl starch, magnesium stearate, and talc, previously passed through a No. 60 U.S. sieve, are then added to the granules which, after mixing, are compressed on a tablet machine to yield tablets.

| Formulation 4: Suspensions | |
|---|---|
| Suspensions each containing 0.25-100 mg of active ingredient per 5 ml dose are made as follows: | |
| Ingredient | Quantity (mg/5 ml) |
| Active ingredient | 0.25-100 mg |
| Sodium carboxymethyl cellulose | 50 mg |
| Syrup | 1.25 mg |
| Benzoic acid solution | 0.10 mL |
| Flavor | q.v. |
| Color | q.v. |
| Purified Water to | 5 mL |

The active ingredient is passed through a No. 45 mesh U.S. sieve and mixed with the sodium carboxymethyl cellulose and syrup to form smooth paste. The benzoic acid solution, flavor, and color are diluted with some of the water and added, with stirring. Sufficient water is then added to produce the required volume.

| Formulation 5: Aerosol | |
|---|---|
| An aerosol solution is prepared containing the following ingredients: | |
| Ingredient | Quantity (% by weight) |
| Active ingredient | 0.25 |
| Ethanol | 25.75 |
| Propellant 22 (Chlorodifluoromethane) | 74.00 |

The active ingredient is mixed with ethanol and the mixture added to a portion of the propellant 22, cooled to 30°C, and transferred to a filling device. The required amount is then fed to a stainless steel container and diluted with the remaining propellant. The valve units are then fitted to the container.

| Formulation 6: Suppositories | |
|---|---|
| Suppositories are prepared as follows: | |
| Ingredient | Quantity (mg/suppository) |
| Active ingredient | 250 |
| Saturated fatty acid glycerides | 2,000 |

The active ingredient is passed through a No. 60 mesh U.S. sieve and suspended in the saturated fatty acid glycerides previously melted using the minimal necessary heat. The mixture is then poured into a suppository mold of nominal 2 g capacity and allowed to cool.

| Formulation 7: Intravenous Solution | |
|---|---|
| An intravenous formulation is prepared as follows: | |
| Ingredient | Quantity |
| Active ingredient | 25 mg-10,000 mg |
| Isotonic saline | 1,000 mL |

The solution of the above ingredients is intravenously administered to a patient.

The pharmaceutical compositions (or formulations described above may be packaged in a variety of ways depending upon the method used for administering the drug. Generally, an article (or kit) for distribution includes a container which contains the pharmaceutical formulation in an appropriate form. Suitable containers are well-known to those skilled in the art and include materials such as bottles (plastic and glass), sachets, ampoules, plastic bags,

metal cylinders, and the like. The container may also include a tamper-proof assemblage to prevent indiscreet access to the contents of the package. In addition, the container has deposited thereon a label that describes the contents of the container. The label may also include appropriate warnings.

The following examples provide an exemplification of compounds within the scope of the invention and the preparations thereof. It will be understood by those skilled in that the art that other synthetic routes may be possible and that equivalent modifications (e.g., bioisostere substitutions) may be made which would fall within the scope and spirit of the invention.

EXAMPLES

## General Experimental Procedures

NMR spectra were recorded on a Varian XL-300 (Varian Co., Palo Alto, California), a Bruker AM-300 spectrometer (Bruker Co., Billerica, Massachusetts) or a Varian Unity 400 at about 23°C at 300 or 400 MHz for proton. Chemical shifts are expressed in parts per million downfield from tetramethylsilane. The peak shapes are denoted as follows: s, singlet; d, doublet; t, triplet, q, quartet; m, multiplet; and bs, broad singlet. Resonances designated as exchangeable did not appear in a separate NMR experiment where the sample was shaken with several drops of $D_2O$ in the same solvent. Atmospheric pressure chemical ionization mass spectra (APCIMS) were obtained on a Fisons Platform II Spectrometer. Chemical ionization mass spectra (CIMS) were obtained on a Hewlett-Packard 5989 instrument (Hewlett-Packard Co., Palo Alto, California) (ammonia ionization, PBMS). Where the intensity of chlorine or bromine-containing ions are described the expected intensity ratio was observed (approximately 3:1 for $^{35}Cl/^{37}Cl$-containing ions and 1:1 for $^{79}Br/^{81}Br$-containing ions) and M is based on $^{35}Cl$ and $^{79}Br$. In some cases only representative $^1H$ NMR and APCIMS peaks are given.

Column chromatography was performed with either Baker Silica Gel (40 μm) (J.T. Baker, Phillipsburg, N.J.) or Silica Gel 60 (EM Sciences, Gibbstown, N.J.) in glass columns or in Flash 40™ or Flash 12™ (Biotage, Charlottesville, VA) columns under nitrogen pressure. Radial Chromatography was performed using a Chromatron, (Harrison Research, Palo Alto, CA.). Unless otherwise specified, reagents were used as obtained from commercial sources. Dimethylformamide, 2-propanol, tetrahydrofuran, and dichloromethane used as reaction solvents were the anhydrous grade supplied by Aldrich Chemical Company (Milwaukee, Wisconsin). Microanalyses were performed by Schwarzkopf Microanalytical Laboratory, Woodside, NY. The terms "concentrated" and "coevaporated" refer to removal of solvent at water aspirator pressure on a rotary evaporator with a bath temperature of less than 50°C. The abbreviation "min" and "h" stand for "minutes" and "hours" respectively and rt stands for room temperature.

## Preparation of Intermediates and Starting Materials

Intermediate compounds and starting materials used in the preparation of Compounds 1-36 exemplified in Examples 1-36 were prepared as described below. All other materials are either available commercially or their preparations are well-known to those skilled in the chemical arts.

*Preparation A*

(2S,3S,4R,5R) 3-Azido-5-{6-[2-(2,5-dimethoxy-phenyl)-ethylamino]-purin-9-yl}-4-hydroxy-tetrahydro-furan-2-carboxylic acid methylamide (Compound A1)

2,5-Dimethoxyphenethylamine (141 μL, 0.848 mmol), (2S,3S,4R,5R) 4-acetoxy-3-azido-5-(6-chloro-purin-9-yl)-tetrahydro-furan-2-carboxylic acid methyl ester (270 mg, 0.707 mmol), ethanol (3.0 mL), and triethylamine (295 μL, 2.1 mmol) were combined and heated to 70°C overnight. The mixture was allowed to cool to room temperature, methylamine (2.12 mL, 2M in MeOH) was added, and the solution was stirred for 5 hours. The product was purified over silica gel via Flash 40, eluting with 7% $MeOH/CH_2Cl_2$.

$^1H$ NMR (400 MHz, $CD_3OD$) δ 8.33 to 8.24 (m, 1H); 8.22 (s, 1H); 6.82 (d, 1H, J = 8.9 Hz); 6.78 to 6.67 (m, 3H); 5.98 (d, 1H, J = 7.1 Hz); 5.06 to 5.00 (m, 1H); 4.43 to 4.37 (m, 2H); 3.79 to 3.73 (m, 3H); 3.72 to 3.67 (m, 1H); 3.66 to 3.63 (m, 3H); 2.99 to 2.92 (m, 2H); 2.86 to 2.81 (m, 3H).

Compounds A2-A25 were prepared according to the general procedure described above for the preparation of Compound A1 using the appropriate amine.

| Compound No. | Compound Name |
|---|---|
| A2 | (2S,3S,4R,5R) 3-Azido-4-hydroxy-5-[6-(3-methoxy-benzylamino)-purin-9-yl]-tetrahydro-furan-2-carboxylic acid methylamide |
| A3 | (2S,3S,4R,5R) 3- Azido -5-[6-(4-benzyloxy-benzylamino)-purin-9-yl]-4-hydroxy-tetrahydro-furan-2-carboxylic acid methylamide |
| A4 | (2S,3S,4R,5R) 3- Azido -4-hydroxy-5-[6-(2-hydroxy-5-methoxybenzylamino)-purin-9-yl]-tetrahydro-furan-2-carboxylic acid methylamide |
| A5 | (2S,3S,4R,5R) 3- Azido -5-[6-(3-butoxy-benzylamino)-purin-9-yl]-4-hydroxy-tetrahydro-furan-2-carboxylic acid methylamide |
| A6 | (2S,3S,4R,5R) 3- Azido -5-[6-(2,5-dimethyl-benzylamino)-purin-9-y\|]-4-hydroxy-tetrahydro-furan-2-carboxylic acid methylamide |
| A7 | (2S,3S,4R,5R) 3- Azido -5-[6-(2,5-dichloro-benzylamino)-purin-9-yl]-4-hydroxy-tetrahydro-furan-2-carboxylic acid methylamide |
| A8 | (2S,3S,4R,5R) 3- Azido -4-hydroxy-5-{6-[3-(2-morpholin-4-yl-ethoxy)-benzylamino]-purin-9-yl}-tetrahydro-furan-2-carboxylic acid methylamide |
| A9 | (2S,3S,4R,5R) 3- Azido -4-hydroxy-5-{6-[3-(3-methyl-isoxazol-5-ylmethoxy)-benzylamino]-purin-9-yl}-tetrahydro-furan-2-carboxylic acid methylamide |
| A10 | (2S,3S,4R,5R) 3- Azido -4-hydroxy-5-[6-(2-methoxy-5-methylbenzylamino)-purin-9-yl]-tetrahydro-furan-2-carboxylic acid methylamide |
| A11 | (2S,3S,4R,5R) 3- Azido -5-{6-[2-(bicyclo[2.2.1]hept-2-yloxy)-5-methoxy-benzylamino]-purin-9-yl}-4-hydroxy-tetrahydro-furan-2-carboxylic acid methylamide |
| A12 | (2S,3S,4R,5R) 3- Azido -5-[6-(2,5-diethyl-benzylamino)-purin-9-yl]-4-hydroxy-tetrahydro-furan-2-carboxylic acid methylamide |
| A13 | (2S,3S,4R,5R) 3- Azido -5-{6-[2-(1-ethyl-propoxy)-5-methoxybenzylamino]-purin-9-yl}-4-hydroxy-tetrahydro-furan-2-carboxylic acid methylamide |
| A14 | (2S,3S,4R,5R) 3- Azido -5-[6-(3-cyclopentyloxy-benzylamino)-purin-9-yl]-4-hydroxy-tetrahydro-furan-2-carboxylic acid methylamide |
| A15 | (2S,3S,4R,5R) 3- Azido -5-[6-(2-cyclopentyloxy-benzylamino)-purin-9-yl]-4-hydroxy-tetrahydro-furan-2-carboxylic acid methylamide |
| A16 | (2S,3S,4R,5R) 3- Azido -5-[6-(5-chloro-2-isopropoxybenzylamino)-purin-9-yl]-4-hydroxy-tetrahydro-furan-2-carboxylic acid methylamide |
| A17 | (2S,3S,4R,5R) 3- Azido -5-[6-(2-benzyloxy-benzylamino)-purin-9-yl]-4-hydroxy-tetrahydro-furan-2-carboxylic acid methylamide |
| A18 | (2S,3S,4R,5R) 3- Azido -5-{6-[2-(4-fluoro-phenyl)-ethylamino]-purin-9-yl}-4-hydroxy-tetrahydro-furan-2-carboxylic acid methylamide |
| A19 | (2S,3S,4R,5R) 3- Azido -5-{6-[2-(4-benzyloxy-3,5-dimethoxyphenyl)-ethylamino]-purin-9-yl}-4-hydroxy-tetrahydro-furan-2-carboxylic acid methylamide |
| A20 | (2S,3S,4R,5R) 3- Azido -4-hydroxy-5-(6-methylamino-purin-9-yl)-tetrahydro-furan-2-carboxylic acid methylamide |
| A21 | (2S,3S,4R,5R) 3- Azido -5-{6-[2-(4-fluoro-3-methoxy-phenyl)-ethylamino]-purin-9-yl}-4-hydroxy-tetrahydro-furan-2-carboxylic acid methylamide |
| A22 | (2S,3S,4R,5R) 3- Azido -5-[6-(2,5-dimethoxy-benzylamino)-purin-9-yl]-4-hydroxy-tetrahydro-furan-2-carboxylic acid methylamide |
| A23 | (2S,3S,4R,5R) 3- Azido -4-hydroxy-5-(6-phenethylamino-purin-9-yl)-tetrahydro-furan-2-carboxylic acid methylamide |

(continued)

| Compound No. | Compound Name |
|---|---|
| A24 | (2S,3S,4R,5R) 3- Azido -4-hydroxy-5-[6-(2-phenylcyclopropylamino)-purin-9-yl]-tetrahydro-furan-2-carboxylic acid methylamide |
| A25 | (2S,3S,4R,5R) 3- Azido -5-{6-[2-(4-benzyloxy-3-methoxyphenyl)-ethylamino]-purin-9-yl}-4-hydroxy-tetrahydro-furan-2-carboxylic acid methylamide |

*Preparation B*

(2S,3S,4R,5R) 3- Azido -5-{6-[(3-benzyloxy-6-methyl-pyridin-2-ylmethyl)-amino]-purin-9-yl}-4-hydroxy-tetrahydro-furan-2-carboxylic acid methylamide

(Compound B1)

3-Benzyloxy-6-methyl-pyridin-2-yl methyl amine (41 mg, 0.17 mmol), (2S,3S,4R,5R) 3-azido-5-(6-chloro-purin-9-yl)- 4-hydroxy-tetrahydro-furan-2-carboxylic acid methyl amide (50 mg, 0.15 mmol), ethanol (5.0 mL), and triethylamine (100 µL, 0.73 mmol) were combined and heated to 70°C overnight. The mixture was concentrated and the residue was dissolved in dichloromethane and reconcentrated 3x to afford a quantitative yield of the title compound as a colorless foam. MS 531 $(M+H)^+$.

Compounds B2-B12 were prepared according to the general procedure described above for the preparation of Compound B1 using the appropriate amine.

| Sample No. | Compound Name |
|---|---|
| B2 | (2S,3S,4R,5R) 3- Azido-5-[6-(2,2-diphenyl-ethylamino)-purin-9-yl]-4-hydroxy-tetrahydro-furan-2-carboxylic acid methylamide |
| B3 | (2S,3S,4R,5R) 3- Azido -5-[2-chloro-6-(2,5-dimethoxy-benzylamino)-purin-9-yl]-4-hydroxy-tetrahydro-furan-2-carboxylic acid methylamide |
| B4 | (2S,3S,4R,5R) 3- Azido -5-{6-[2-(3-benzyloxy-4-methoxy-phenyl)-ethylamino]-purin-9-yl}-4-hydroxy-tetrahydro-furan-2-carboxylic acid methylamide |
| B5 | (2S,3S,4R,5R) 3- Azido -4-hydroxy-5-[6-(2-pyridin-3-yl-ethylamino)-purin-9-yl]-tetrahydro-furan-2-carboxylic acid methylamide |
| B6 | (2S,3S,4R,5R) 3- Azido -5-(2-chloro-6-methylamino-purin-9-yl)-4-hydroxy-tetrahydro-furan-2-carboxylic acid methylamide |
| B7 | (2S,3S,4R,5R) 3- Azido -5-[2-chloro-6-(2,5-dichloro-benzylamino)-purin-9-yl]-4-hydroxy-tetrahydro-furan-2-carboxylic acid methylamide |
| B8 | (2S,3S,4R,5R) 3- Azido -4-hydroxy-5-{6-[2-(2-morpholin-4-yl-thiazol-5-yl)-ethylamino]-purin-9-yl}-tetrahydro-furan-2-carboxylic acid methylamide |
| B9 | (2S,3S,4R,5R) 3- Azido -4-hydroxy-5-[6-(2-naphthalen-1-ylethylamino)-purin-9-yl]-tetrahydro-furan-2-carboxylic acid methylamide |
| B10 | (2S,3S,4R,5R) 3- Azido -5-{6-[(5-fluoro-1H-indol-3-ylmethyl)-amino]-purin-9-yl}-4-hydroxy-tetrahydro-furan-2-carboxylic acid methylamide |
| B11 | (2S,3S,4R,5R) 3- Azido -4-hydroxy-5-[6-(2-pyridin-2-yl-ethylamino)-purin-9-yl]-tetrahydro-furan-2-carboxylic acid methylamide |
| B12 | (2S,3S,4R,5R) 3- Azido -4-hydroxy-5-[6-(2-phenyl-cyclopropylamino)-purin-9-yl]-tetrahydro-furan-2-carboxylic acid methylamide |

*Preparation C*

(2S,3S,4R,5R)3-Azido-5-(6-chloro-purin-9-yl)-4-hydroxy-tetrahydro-furan-2-carboxylic acid methylamide (Compound C1)

Triethyl amine (4.4 mL, 0.032 mL) was added to a solution of (2S,3S,4R,5R)3-azido-5-(6-chloro-purin-9-yl)-4-acetoxy-tetrahydro-furan-2-carboxylic acid methylamide (4g, 0.011 mmol) in methanol (80 mL). After stirring for 15 hours, the mixture was concentrated and the residue purified by flash chromatography (5% methanol/dichloromethane) to afford 2.7 g (77%) of the title compound as a colorless solid. MS 339 $(M+H)^+$: $^1H$ NMR (400 MHz, $CDCl_3$) δ 8.72 (s, 1H); 8.2 (s, 1H); 7.62 (bs, 1H); 5.98 (d, 1H, J = 6.7 Hz); 5.07 (t, 1H, J = 6.2 Hz); 4.65 (dd, 1H, J = 5.4, 2.7 Hz); 4.58 (m, 1H); 4.2 (bs, 1H); 2.88 (d, 3H, J = 4.9 Hz); 1.67 (bs, 1H).

*Preparation D*

Alternate preparation of (2S,3S,4R,5R)3-azido-5-(6-chloropurin-9-yl)-4-hvdroxytetrahydrofuran-2-carboxylic acid methylamide (Compound C1)

To a solution of acetic acid 4-azido-2-(6-chloropurin-9-yl)-5-methylcarbamoyl-tetrahydrofuran-3-yl ester (1.1 g, 2.9 mmol) in anhydrous methanol (100 mL), cooled to 0 °C, was added triethylamine (1.2 mL, 8.6 mmol). The solution was stirred for 2 h at room temperature, under anhydrous conditions. After removal of solvent by rotary evaporation, the resulting solid was purified via flash chromatography (7% $MeOH/CH_2Cl_2$) to yield the title compound as a white foam.

$C_{11}H_{11}ClN_8O_3$; MW 338.72; MS 339.1 $(M+H)^+$; $^1H$ NMR (400 MHz, DMSO-$d_6$) δ 9.01 (s, 1H); 8.81 (s, 1 H); 8.22 (quart, 1 H, J = 4.2 Hz); 6.41 (dd, 1 H, J = 5.2 Hz, J = 2.1 Hz); 6.12 (d, 1H, J = 5.2 Hz); 5.03 (quart, 1H, J = 5.2 Hz); 4.57-4.47 (mult, 1H); 4.41 (d, 1H, J = 3.9 Hz); 2.61 (d, 3H, J = 4.2 Hz).

*Preparation E*

(2S,3S,4R,5R)3-Azido-5-(6-chloro-purin-9-yl)-4-acetoxy-tetrahydro-furan-2-carboxylic acid methylamide (Compound E1)

6-Chloropurine (20.4 g, 0.132 mol) was suspended in hexamethyl disilazane (165 mL) and heated at 110°C. After 2h, the now homogeneous mixture was concentrated and the solid residue reconcentrated from toluene 2x and placed under high vacuum for 1 hour. The resulting solid was combined with (2S,3S,4R)-3-azido-4,5-diacetoxytetrahydrofuran-2-carboxylic acid methylamide (12.7 g, 0.044 mol) and dissolved in anhydrous dichloroethane (350 mL). Powdered 4Å molecular sieves (15 g) were added and the mixture stirred for 15 minutes. Trimethylsilyl triflate (TMSOTf) (16.0 mL, 0.088 mol) was added and the reaction was then heated to 60°C for two hours, cooled and quenched by the careful addition of saturated sodium bicarbonate solution (200 mL). Ethyl acetate (350 mL) was added and the mixture was filtered through sintered glass. The filtrate was extracted with ethyl acetate (3x) and the combined organic layers were washed with brine, dried ($Na_2SO_4$), filtered and concentrated. The residue was purified by flash chromatography, (15 then 20% acetone/dichloromethane) to afford the title compound (10.6 g) as an off-white foam.

MS 381 $(M+H)^+$: $^1H$ NMR (400 MHz, $CDCl_3$) δ 8.8 (s, 1H); 8.2 (s, 1H); 7.6 (bs, 1H); 6.13 (d, 1H, J = 7.0 Hz); 5.87 (dd, 1H, J = 7.0, 5.6 Hz); 4.85 (dd, 1H, J = 5.6, 3.0 Hz); 4.58 (d, 1H, J = 3.0 Hz); 2.9 (d, 1H, J = 4.9 Hz); 2.11 (s, 3H).

(2S,3S,4R,5R)3-Azido-5-(2,6-dichloro-purin-9-yl)-4-hydroxy-tetrahydro-furan-2-carboxylic acid methylamide (Compound E2) was prepared using the same general procedure described above for the preparation of Compound E1 from the appropriate starting material.

*Preparation F*

(2S,3S,4R)-3-Azido-4,5-diacetoxytetrahydrofuran-2-carboxylic acid methyl amide

3-Azido-3-deoxy-4,5-O-isopropylidene-α-D-ribofuranuronic acid methylamide (12 g, 0.05 mmol) was dissolved in acetic acid (150 mL) and acetic anhydride (50 mL). The mixture was cooled in an ice bath and a solution of sulfuric acid (1 mL dissolved in 5 mL acetic acid) was added. The mixture was allowed to warm to room temperature and stirred for 18 hours. The reaction mixture was added dropwise to a saturated solution of sodium bicarbonate (2 L), and then extracted with chloroform (3x). The combined organic layers were washed with water (2x) and sat. $NaHCO_3$ (2x) and brine (1x), dried ($Na_2SO_4$), filtered and concentrated to afford the title compound as a mixture of anomeric acetates as a tan oil.

*Preparation G*

Preparation of 3-azido-3-dexoy-4,5-O-isopropylidene-α-D-ribofuranuronic acid methylamide.

Oxalyl chloride (15 mL) was added to a solution of 3-azido-3-deoxy-4,5-O-isopropyiidene-α-D-ribofuranuronic acid (30 g) in anhydrous THF (250 mL) at 0°C. DMF (1 mL) was added and the reaction was allowed to warm to room temperature at which time gas evolution commenced. After five hours, the mixture was concentrated and the residue dissolved in dichloromethane (100 mL), and then cooled to 0°C. A solution of methyl amine in THF (260 mL of a 2M solution) was added slowly. After 30 minutes, the mixture was diluted with water (500 mL) and extracted with chloroform (3x). The combined organic layers were dried ($Na_2SO_4$) filtered and concentrated to afford the title compound as a light brown solid.
$^1H$ NMR (400 MHz, $CDCl_3$) δ 6.39 (bs, 1H); 5.80 (d, 1H, J = 3.7 Hz); 4.67 (dd, 1H, J = 4.0, 3.7 Hz); 4.42 (d, 1H, J = 9.3 Hz); 3.6 (dd, 1H, J = 9.3, 4.0 Hz); 2.9 (d, 3H, J = 5.0 Hz); 1.54 (s, 3H); 1.34 (s, 3H).

*Preparation H*

(2S,3S,4R,5R) 4-Acetoxy-3-azido-5-(6-chloropurin-9-yl)tetrahydrofuran-2-carboxylic acid methyl ester (Compound H1)

A solution of 6-chloropurine (4.96 g, 32 mmol) and hexamethyldisilazane (40 mL) were combined and heated to 100°C for 3 h under anhydrous conditions. The mixture was allowed to cool to room temperature and was then concentrated to a solid on a rotary evaporator using anhydrous toluene (3 x 50mL) to help remove the solvent. The solid was pumped on high vacuum for 15 minutes and then anhydrous acetonitrile (50 mL) was added. (2S,3S,4R)-3-Azido-4,5-diacetoxytetrahydrofuran-2-carboxylic acid methyl ester (3.09 g, 10.7 mmol) was dissolved in anhydrous acetonitrile (20 mL) and added to the reaction. Trimethylsilyl trifluoromethanesulfonate (7.5 mL, 41.4 mmol) was added. The reaction was stirred at 70°C, under anhydrous conditions, for 15 h. The reaction mixture was quenched with saturated aqueous sodium bicarbonate (200 mL). Water (160 mL) was added, and the reaction mixture was extracted with EtOAc (4 x 100 mL), dried over sodium sulfate, and concentrated to a solid on a rotary evaporator. This solid was purified via flash chromatography (7:3 hexane:EtOAc) to afford 2.88 g of the title compound as a white foam.
Mp 94.0 - 96.0°C: $^1H$ NMR (400 MHz, $CDCl_3$) δ 8.73 (s, 1H); 8.60 (s, 1H); 6.36 (d, 1H, J = 5.4 Hz); 5.83 (t, 1H, J = 5.4 Hz); 4.83 - 4.77 (mult, 1H); 4.65 (d, 1H, J = 4.2 Hz); 3.84 (s, 3H); 2.14 (s, 3H).

*Preparation I*

(2S,3S,4R) 3-Azido-4,5-diacetoxy-tetrahydrofuran-2-carboxylic acid methyl ester (Compound I1)

3-Azido-3-deoxy-4,5-O-isopropylidene-α-D-ribofuranuronic acid, methyl ester (4.85 g, 20 mmol), concentrated $H_2SO_4$ (5.5 mL), glacial acetic acid (65 mL), and acetic anhydride (18 mL, 20 mmol) were combined and stirred at room temperature, under anhydrous conditions, for 15 h. The reaction mixture was then taken up in water (500 mL), neutralized with solid sodium hydroxide to pH 7 and extracted with EtOAc (3 x 250 mL). The combined organic layers were washed with saturated aqueous NaCl (500 mL), dried over sodium sulfate and concentrated on a rotary evaporator to a solid, which was purified via flash chromatography (2:1 hexane:EtOAc) to afford 3.09 g of the title compound as a clear, colorless oil.
$^1H$ NMR (400 MHz, $CDCl_3$) δ 6.18 (s, 1H); 5.33 (d, 1H, J = 5.0 Hz); 4.53 (d, 1H, J = 7.5 Hz); 4.44 - 4.38 (mult, 1H); 3.83 (s, 3H); 2.17 (s, 3H); 2.08 (s, 3H).

*Preparation J*

3-Azido-3-deoxy-4,5-O-isopropylidene-α-D-ribofuranuronic acid, methyl ester (Compound J1)

To a solution of 3-azido-3-deoxy-4,5-O-isopropylidene-α-D-ribofuranuronic acid (4.23 g, 18 mmol) in DMF (50 mL) was added potassium carbonate (3 g, 22 mmol) and iodomethane (2.3 mL, 37 mmol). The reaction mixture was stirred at room temperature under anhydrous conditions for 15 h. The reaction mixture was then taken up in EtOAc (500 mL) and washed with water (500 mL), saturated aqueous $NaHCO_3$ (2 x 500 mL), and saturated aqueous NaCl (500 mL). The combined organic layers were dried over sodium sulfate and concentrated on a rotary evaporator to an oil. The product was purified by flash chromatography (7:3 hexane:EtOAc) to afford 4.45 g of the title compound as a clear, colorless oil.
$^1H$ NMR (400 MHz, $CDCl_3$) δ 5.89 (d, 1H, J = 3.3 Hz); 4.74 - 4.68 (mult, 1H); 4.53 (d, 1H, J = 9.6 Hz); 3.82

(s, 3H); 3.69 - 3.63 (mult, 1H); 1.55 (s, 3H); 1.34 (s, 3H).

*Preparation K*

3-Azido-3-deoxy-1,2-O-isopropylidene-α-D-ribofuranuronic acid (Compound K1)

A solution of 3-azido-1,2,5,6- *bis*-O-isopropylidene-3-deoxy-D- allofuranose (451 g, 1.58 mol) in diethyl ether (4.5 L) was treated with periodic acid (540 g, 2.37 mol) at ambient temperature which was maintained with a water bath. After 24 hours, the precipitated salts were filtered and washed with ether. The filtrate was concentrated and the crude aldehyde was added to a mixture of chloroform (2.5 L), acetonitrile (2.5 L) and water (3.4 L). To this vigorously stirred mixture was added sodium periodate (1211 g, 5.67 mol) and ruthenium trichloride hydrate (14.5 g, 0.69 mol) at room temperature maintained with a water bath. After 20 hours, the mixture was diluted with chloroform (4 L) and water (4 L) and the mixture was filtered through Celite™. The layers were separated and the aqueous phase reextracted with chloroform. The combined organic layers were concentrated in vacuo and the residue partitioned between saturated aqueous sodium bicarbonate (2 L) and ethyl acetate (3 L). The layers were separated and the aqueous layer reextracted with ethyl acetate (2 L). The aqueous layer was acidified with 2N HCl solution and extracted with ethyl acetate (3 x 3 L). The combined organic layers were dried ($Na_2SO_4$), filtered and concentrated to give 208 g of the title compound as an off-white solid, pure by tlc and NMR.

$^1H$ NMR (300 MHz, $CDCl_3$) δ 7.5 (bs, 1H); 5.95 (d, 1H, J = 3.7); 4.8 (dd, 1H, J = 4.0, 3.7 Hz); 4.6 (d, 1H, J = 9.5 Hz); 3.72 (dd, 1H, J = 9.5, 4.0 Hz); 1.58 (s, 3H); 1.38 (s, 3H).

*Preparation L*

3-Azido-1,2,5,6- bis-O-isopropylidene-3-deoxy-D- allofuranose (Compound L1)

Triflic anhydride (1500 g, 5.3 mol) was added dropwise to a solution of pyridine (493 g, 6.2 mol) in dichloromethane (7.5 L) at -15°C. After 30 minutes, a solution of 1,2,5,6- bis-O-isopropylidene-D- glucofuranose (735 g, 2.84 mol) was added as a solution in dichloromethane (2.5 L). After 1 hour, the reaction was quenched by the dropwise addition of water (4 L) allowing the reaction temperature to warm to 0°C. The layers were separated and the organic layer dried with sodium sulfate, filtered and concentrated to give a red oil. The triflate was immediately dissolved in DMF (8 L) and treated with $NaN_3$ (554 g, 8.5 mol) and warmed to 35°C. After 18 hours, the mixture was poured into water (12 L) and extracted with ethyl acetate (3 x 4 L). The combined organic layers were washed with water (2 x 3 L) and brine (1 x 3 L), dried ($Na_2SO_4$), filtered and concentrated. The residue was preadsorbed onto silica gel and purified by flash chromatography (6:1 hexane/EtOAc then 4:1 hexane/EtOAc) to afford 228 g of the title compound as a colorless oil.

$^1H$ NMR (400 MHz, $CDCl_3$) δ 5.77 (d, 1H, J = 3.7 Hz); 4.7 (dd, 1H, J = 4.4, 3.7 Hz); 4.15 (m, 2H); 4.0 (m, 2H); 3.5 (dd, 1H, J = 9.1, 4.4 Hz); 1.56 (s, 3H); 1.47 (s, 3H); 1.37 (s, 3H); 1.34 (s, 3H).

*Preparation M*

2-Benzyloxy-benzyl amine hydrochloride (Compound M1)

2-Benzyloxy-benzonitrile (1.36 g, 6.50 mmol) was dissolved in anhydrous THF (42 mL) and was added to a flask containing lithium aluminum hydride (14.9 mL, 1M in THF). The reaction was stirred at room temperature for 90 minutes. The reaction was cooled in an icebath, and sodium hydroxide (2.16 mL, 1M in $H_2O$) was added slowly over a time period of 5 minutes. The reaction was filtered and the filtrate was concentrated to an oil. The oil was taken up in ethanol and subjected to aqueous hydrochloric acid (1eq., 1N) and the reaction was stirred for 15 minutes. The reaction was concentrated, taken up in ether, and filtered collecting a white solid.

$^1H$ NMR (400 MHz, DMSO) δ 8.40 to 8.17 (m, 3H); 7.50 to 7.42 (m, 2H); 7.41 to 7.23 (m, 5H); 7.08 (d, 1H, J = 5.9 Hz); 6.95 (t of d, 1H, J = 7.5 Hz, J = 0.8 Hz); 5.14 (s, 2H); 4.00 to 3.91 (m, 2H).

The following compounds M2-M9 were prepared from the appropriate nitrile according to the general procedure above for the preparation of Compound M1.

| Compound No. | Compound Name |
|---|---|
| M2 | 4-Benzyloxy-benzyl amine |
| M3 | 3-n-Butoxy-benzyl amine |

(continued)

| Compound No. | Compound Name |
|---|---|
| M4 | 4-Methoxy-2-(3-pentyloxy)-benzyl amine |
| M5 | 3-Cyclopentyloxy-benzyl amine |
| M6 | 2-Cyclopentyloxy-benzyl amine |
| M7 | 5-Chloro-2-isopropyloxy-benzyl amine |
| M8 | 4-Benzyloxy-3,5-dimethoxy-phenethyl amine |
| M9 | 4-Fluoro-3-methoxy-phenethyl amine |

*Preparation N*

2,5, Diethyl-benzyl amine hydrochloride (Compound N1)

2-Azidomethyl-1,4-diethyl-benzene (1.84 g, 9.72 mmol) was dissolved in anhydrous THF (50 mL) and the solution was cooled to 0°C. To this solution was added, under $N_2$, triphenylphosphine (2.55 g, 9.72 mmol) and the reaction was stirred at 0°C for 30 minutes. At the end of that time period distilled water (0.25 mL) and ammonium hydroxide (0.39 mL, conc.) were added. The reaction was allowed to slowly come to room temperature, where it was stirred overnight. The solution was concentrated down and the product was taken up in absolute ethanol (60 mL) and aqueous hydrochloric acid (9.7 mL, 1N) was added and stirred at room temperature for 30 minutes. The solvent was stripped and toluene (30 mL) was added, then the solution was stripped to an oil. Ether (10 mL) was added, and once the solid formed, toluene (40 mL) was added and the solution was stirred overnight. The white solid was filtered off and washed with toluene, followed by ether, then hexanes.
$^1$H NMR (400MHz, DMSO) δ 8.46 to 8.28 (m, 3H); 7.30 to 7.24 (m, 1H); 7.14 to 7.06 (m, 2H); 3.93 (s, 2H); 2.64 to 2.48 (m, 4H); 1.19 to 1.04 (m, 6H).
Compounds N2-N4 were prepared from the appropriate azide according to the general procedure described above for the preparation of Compound N1.

| Compound No. | Compound Name |
|---|---|
| N2 | 2-Methoxy-5-methyl-benzyl amine |
| N3 | 3-Benzyloxy-6-methyl-pyridin-2-yl methyl amine |
| N4 | 2-Hydroxy-5-methoxy-benzyl amine |

*Preparation O*

5-Chloro-2-isopropoxy-benzonitrile (Compound O1)

5-Chloro-2-hydroxy-benzonitrile (1.0 g, 6.51 mmol), anhydrous THF (100 mL), 2-propanol (0.50 mL, 6.53 mmol), and triphenylphosphine (2.57 g, 9.80 mmol) were combined together. To this solution diethylazodicarboxylate (1.54 mL, 9.78 mmol) was added by syringe and the reaction was stirred at room temperature overnight. The solvent was stripped off and the product was purified *via* a Flash 40, 90g column, eluted with a 2:1 ratio of hexanes:$CH_2Cl_2$ (1L).
$^1$H NMR (400 MHz, DMSO) δ 7.83 (d, 1H, J = 2.7 Hz); 7.63 (dd, 1H, J = 9.1, 2.7 Hz); 7.25 (d, 1H, J = 9.1 Hz); 4.74 (sept, 1H, J = 6.0 Hz); 1.25 (d, 1H, J = 6.2 Hz).
Compounds O2-O6 were prepared from the appropriate starting materials using the general procedure described above for the preparation of Compound O1.

| Compound No. | Compound Name |
|---|---|
| O2 | 2-Cyclopentyloxy-benonitrile |
| O3 | 3-Cyclopentyloxy-benzonitrile |
| O4 | 2-(1-Ethyl-propoxy)-5-methoxy-benzonitrile |
| O5 | 3-*n*-butoxy-benzonitrile |

(continued)

| Compound No. | Compound Name |
|---|---|
| O6 | 3-*n*-butoxy-benzonitrile |

*Preparation P*

2-Azidomethyl-1-methoxy-4-methyl-benzene (Compound P1)

(2-Methoxy-5-methyl-phenyl)-methanol (1.14 g, 7.49 mmol) was dissolved in anhydrous toluene (100 mL), the mixture was then cooled in an icebath to 0°C. Diphenylphosphoryl azide (2.10 mL, 9.74 mmol) was added dropwise by syringe, then 1,8-diazabicyclo[5.4.0]undec-7-ene (1.60 mL, 10.70 mmol) was added to the reaction the same way. The reaction was stirred at 0°C and allowed to slowly rise to room temperature, where it was stirred overnight. The resulting solution was poured into water (200 mL) and toluene (100 mL), the layers were separated, extracted with toluene (2 x 100 mL). The combined organic layers were washed with water (1 x 100 mL) and brine (1 x 100 0mL, sat.), then dried over sodium sulfate, filtered and concentrated down to an oil.

$^1$H NMR (400 MHz, DMSO) δ 7.14 to 7.05 (mult, 2H); 6.91 to 6.87 (mult, 1H); 4.27 (s, 2H); 3.72 (s, 3H); 2.19 (s, 3H).

Compounds P2-P4 were prepared from the appropriate starting materials according to the general procedure described above for the preparation of Compound P1.

| Compound No. | Compound Name |
|---|---|
| P2 | 2-Azidomethyl-1,4-diethyl-benzene |
| P3 | (2-Azidomethyl-4-methoxy-phenoxy)-tert-butyl-dimethyl-silane |
| P4 | 2-Azidomethyl-3-benzyloxy-6-methyl- pyridine |

***Example 1***

(2S,3S,4R,5R) 3-Amino-5-{6-[2-(2,5-dimethoxy-phenyl)-ethylamino]-purin-9-yl}-4-hvdroxy-tetrahydro-furan-2-carboxylic acid methylamide (Compound 1)

(2S,3S,4R,5R) 3-Azido-5-{6-[2-(2,5-dimethoxy-phenyl)-ethylamino]-purin-9-yl}-4-hydroxy-tetrahydro-furan-2-carboxylic acid methylamide (100 mg, 0.207 mmol) was dissolved in THF (2.0 mL), under $N_2$, and cooled to 0°C. Triphenylphosphine (81.5 mg, 0.310 mmol) was added and the reaction was stirred cold for 30 minutes. At the end of this time period water (1 drop) and ammonium hydroxide (90 µL, conc.) were added. The reaction was brought to room temperature, and stirred overnight. The product was concentrated to an oil and purified over silica gel via Flash 40, eluting with 10% $MeOH/CH_2Cl_2$, affording 50 mg of the title compound (1) as a white powder.

$C_{21}H_{27}N_7O_5$: MW = 457.49: MS 458 $(M+H)^+$: $^1$H NMR (400 MHz, $CD_3OD$) δ 8.30 (s, 1H); 8.28 to 8.23 (m, 1H); 6.82 (d, 1H, J = 8.9 Hz); 6.75 (d, 1H, J = 3.1 Hz); 6.73 to 6.67 (m, 2H); 6.04 (d, 1H, J = 4.2 Hz); 5.47 (d, 1H, J = 1.2 Hz); 4.59 to 4.53 (m, 1H); 4.30 (d of m, 1 H, J = 5.6 Hz); 3.76 (s, 3H); 3.75 to 3.70 (m, 1H); 3.67 (d, 1H, J = 1.0 Hz); 2.99 to 2.92 (m, 2H); 2.82 (s, 3H).

Compounds 2-36 in Examples 2-36 were prepared using the same general procedure described above using the appropriate starting materials and intermediates.

***Example 2***

(2S,3S,4R,5R) 3-Amino-4-hydroxy-5-[6-(3-methoxy-benzylamino)-purin-9-yl]-tetrahydro-furan-2-carboxylic acid methylamide (Compound 2)

$C_{19}H_{23}N_7O_4$: MW = 413.44: MS 414 $(M+H)^+$: $^1$H NMR (300 MHz, $CD_3OD$) δ 8.31 (s, 1H); 8.27 (s, 1H); 7.21 (t, 1H, J = 8.1 Hz); 6.99 to 6.91 (m, 2H); 6.84 to 6.76 (m, 1H); 6.11 to 6.07 (m, 1H); 4.82 to 4.74 (m, 1H); 4.72 (m, 1H); 4.41 (d, 1H, J = 6.1 Hz); 4.03 to 3.94 (m, 1H); 3.75 (s, 3H); 2.80 (s, 3H).

*Example 3*

(2S,3S,4R,5R) 3-Amino-5-[6-(4-benzyloxy-benzylamino)-purin-9-yl]-4-hydroxy-tetrahydro-furan-2-carboxylic acid methylamide. (Compound 3)

$C_{25}H_{27}N_7O_4$: MW = 489.54: MS 490 $(M+H)^+$: $^1H$ NMR (400 MHz, $CD_3OD$) δ 8.31 (s, 1H); 8.26 (s, 1H); 7.41 to 7.35 (m, 2H); 7.34 to 7.23 (m, 5H); 6.91 (d, 2H, J = 8.7 Hz); 6.03 (d, 1H, J = 4.2 Hz); 5.03 (s, 2H); 4.95 to 4.85 (m, 2H); 4.78 to 4.73 (m, 1H); 4.27 (d, 1H, J = 5.6 Hz); 3.76 to 3.68 (m, 1H); 2.79 (s, 3H).

*Example 4*

(2S,3S,4R,5R) 3-Amino-4-hydroxy-5-[6-(2-hydroxy-5-methoxy-benzylamino)-purin-9-yl]-tetrahydro-furan-2-carboxylic acid methylamide. (Compound 4)

$C_{19}H_{23}N_7O_5$: MW = 429.44: MS 430 $(M+H)^+$: $^1H$ NMR (400 MHz, $CD_3OD$) δ 8.36 to 8.34 (m, 1 H); 8.34 to 8.29 (m, 1H); 8.24 to 8.21 (m, 1 H); 7.94 to 7.87 (m, 1H); 6.89 to 6.85 (m, 1H); 6.81 to 6.78 (m, 1H); 6.72 to 6.67 (m, 3H); 6.05 (d, 1H, J = 4.2 Hz); 6.03 (d, 1H, J = 4.8 Hz); 4.68 to 4.61 (m, 3H); 4.60 to 4.25 (m, 2H); 3.10 to 3.02 (m, 3H); 2.82 to 2.78 (m, 2H).

*Example 5*

(2S,3S,4R,5R) 3-Amino-5-[6-(3-butoxy-benzylamino)-purin-9-yl]-4-hydroxy-tetrahydro-furan-2-carboxylic acid methylamide. (Compound 5)

$C_{22}H_{29}N_7O_4$: MW = 455.52: MS 456 $(M+H)^+$: $^1H$ NMR (300 MHz, DMSO) δ 8.56 (s, 1H); 8.52 (d, 1H, J = 4.5 Hz); 8.52 to 8.39 (m, 1H); 8.26 (s, 1H); 7.22 to 7.11 (m, 1H); 6.94 to 6.83 (m, 2H); 6.80 to 6.70 (m, 1H); 6.03 (d, 1H, J = 3.9 Hz); 6.00 to 5.83 (m, 1H); 4.76 to 4.57 (m, 2H); 4.46 to 4.32 (m, 1H); 4.14 (d, 1H, J = 5.6 Hz); 3.94 to 3.83 (m, 2H); 3.63 to 3.53 (m, 1H); 2.68 (d, 3H, J = 4.5 Hz); 1.90 to 1.72 (m, 2H); 1.71 to 1.57 (m, 2H); 1.47 to 1.30 (m, 2H); 0.94 to 0.82 (m, 3H).

*Example 6*

(2S,3S,4R,5R) 3-Amino-5-[6-(2,5-dimethyl-benzylamino)-purin-9-yl]-4-hydroxy-tetrahydro-furan-2-carboxylic acid methylamide. (Compound 6)

$C_{20}H_{25}N_7O_3$: MW = 411.47: MS 412$(M+H)^+$: $^1H$ NMR (300 MHz, DMSO) δ 8.56 (s, 1H); 8.53 (d, 1H, J = 4.6 Hz); 8.40 to 8.29 (m, 1H); 8.25 (s, 1H); 7.09 to 6.98 (m, 2H); 6.91 (d, 1H, J = 7.6 Hz); 6.03 (d, 1H, J = 4.0 Hz); 6.00 to 5.87 (m, 1H); 4.76 to 4.55 (m, 2H); 4.45 to 4.31 (m, 1H); 4.14 (d, 1H, J = 5.6 Hz); 3.62 to 3.53 (m, 1H); 2.68 (d, 3H, J = 4.5 Hz); 2.28 (s, 3H); 2.16 (s, 3H); 1.90 to 1.70 (m, 2H).

*Example 7*

(2S,3S,4R,5R) 3-Amino-5-[6-(2,5-dichloro-benzylamino)-purin-9-yl]-4-hydroxy-tetrahydro-furan-2-carboxylic acid methylamide. (Compound 7)

$C_{18}H_{19}Cl_2N_7O_3$: MW = 452.30: MS 452 $(M+H)^+$: $^1H$ NMR (400 MHz, $CD_3OD$) δ 8.37 (s, 1H); 8.29 (s, 1H); 7.43 to 7.36 (m, 2H); 7.29 to 7.22 (m, 1H); 6.08 (d, 1 H, J = 3.9 Hz); 4.90 to 4.85 (m, 2H); 4.64 to 4.58 (m, 1 H); 4.36 to 4.28 (m, 1H); 3.80 to 3.75 (m, 1H); 2.81 (s, 3H).

*Example 8*

(2S,3S,4R,5R) 3-Amino-4-hydroxy-5-{6-[3-(2-morpholin-4-yl-ethoxy)-benzylamino]-purin-9-yl}tetrahydro-furan-2-carboxylic acid methylamide. (Compound 8)

$C_{24}H_{32}N_8O_5$: MW = 512.57: MS 513 $(M+H)^+$: $^1H$ NMR (400 MHz, DMSO) δ 8.54 to 8.43 (m, 2H); 8.27 to 8.20 (m, 1H); 7.87 to 7.77 (m, 1H); 7.21 to 7.16 (m, 1 H); 6.92 to 6.84 (m, 2H); 6.80 to 6.76 (m, 1H); 6.00 (d, 1 H, J = 4.2 Hz); 5.95 to 5.85 (m, 1H); 4.40 to 4.33 (m, 1H); 4.11 (d, 1H, J = 5.6 Hz); 4.04 to 3.98 (m, 1H); 3.60 to 3.48 (m, 3H); 2.99 to 2.87 (m, 2H); 2.66 (d, 3H, J = 4.6 Hz); 2.64 to 2.59 (m, 1 H); 2.56 to 2.44 (m, 4H); 2.44 to 2.37 (m, 4H); 2.03

to 1.86 (m, 2H).

***Example 9***

(2S,3S,4R,5R) 3-Amino-4-hydroxy-5-{6-[3-(3-methyl-isoxazol-5-ylmethoxy)-benzylamino]-purin-9-yl}-tetrahydro-furan-2-carboxylic acid methylamide. (Compound 9)

$C_{23}H_{26}N_8O_5$: MW = 427.47: MS 428 $(M+H)^+$:$^1H$ NMR (300 MHz, $CDCl_3$) δ 8.38 to 8.31 (m, 1 H); 8.31 to 8.22 (m, 1 H); 7.86 to 7.78 (m, 1 H); 7.77 to 7.70 (m, 1H); 7.23 to 7.15 (m, 1H); 7.02 to 6.91 (m, 2H); 6.91 to 6.83 (m, 2H); 6.09 to 6.00 (m, 1H); 6.00 to 5.93 (m, 1H); 5.10 to 5.01 m, 2H); 4.90 to 4.72 (m, 2H); 4.64 to 4.52 (m, 1H); 4.36 to 4.26 (m, 1H); 4.20 to 4.05 (m, 1H); 2.89 to 2.72 (m, 3H); 2.42 to 2.35 (m, 3H); 2.35 to 2.23 (m, 2H).

***Example 10***

(2S,3S,4R,5R) 3-Amino-4-hydroxy-5-[6-(2-methoxy-5-methyl-benzylamino)-purin-9-yl-tetrahydro-furan-2-carboxylic acid methylamide. (Compound 10)

$C_{20}H_{25}N_7O_4$: MW = 427.479: MS 428 $(M+H)^+$: $^1H$ NMR (400 MHz, DMSO) δ 8.54 (s, 1H); 8.49 (d, 1H, J = 4.6 Hz); 8.21 (s, 1H); 8.20 to 8.10 (m, 1H); 7.02 to 6.95 (m, 1H); 6.95 to 6.87 (m, 1H); 6.85 (d, 1H, J = 8.1 Hz); 6.15 to 5.75 (m, 1H); 6.02 (d, 1H, J = 4.2 Hz); 4.70 to 4.58 (m, 2H); 4.46 to 4.38 (m, 1H); 4.14 (d, 1H, J = 5.4 Hz); 3.77 (s, 3H); 3.65 to 3.58 (m, 1H); 2.66 (d, 3H, J = 4.6 Hz); 2.60 to 2.30 (m, 2H).

***Example 11***

(2S,3S,4R,5R) 3-Amino-5-[6-(2,5-diethyl-benzylamino)-purin-9-yl]-4-hydroxy-tetrahydro-furan-2-carboxylic acid methylamide. (Compound 11)

$C_{22}H_{29}N_7O_3$: MW = 439.52: MS 440 $(M+H)^+$: $^1H$ NMR (400 MHz, DMSO) δ 8.52 (s, 1H); 8.49 (d, 1H, J = 4.6 Hz); 8.36 to 8.26 (m, 1H); 8.23 (m, 1H); 7.14 to 7.09 (m, 1H); 7.06 (d, 1H, J = 7.7 Hz); 6.98 (d of m, 1H, J = 7.7 Hz); 6.00 (d, 1H, J = 3.9 Hz); 5.94 to 5.83 (m, 1H); 4.76 to 4.62 (m, 2H); 4.39 to 4.34 (m, 1H); 4.11 (d, 1H, J = 5.6 Hz); 3.60 to 3.54 (m, 1H); 2.70 to 2.61 (m, 5H); 2.52 to 2.40 (m, 2H); 1.95 to 1.82 (m, 2H); 1.14 (t, 3H, J = 7.6 Hz); 1.06 (t, 3H, J = 7.6 Hz).

***Example 12***

(2S,3S,4R,5R) 3-Amino-5-{6-[2-(1-ethyl-propoxy)-5-methoxy-benzylamino]-purin-9-yl}-4-hydroxy-tetrahydro-furan-2-carboxylic acid methylamide. (Compound 12)

$C_{24}H_{33}N_7O_5$: MW = 499.58: MS 500 $(M+H)^+$: $^1H$ NMR (400 MHz, $CD_3OD$) δ 8.32 (s, 1H); 8.28 (s, 1H); 6.90 to 6.82 (m, 2H); 6.79 to 6.74 (m, 1H); 6.06 (d, 1H, J = 4.2 Hz); 4.82 to 4.73 m, 2H); 4.62 (m, 1H); 4.30 (d, 1H, J = 5.6 Hz); 4.20 to 4.13 (m, 1H); 3.79 to 3.73 (m, 1H); 3.68 (s, 3H); 2.81 (s, 3H); 1.73 to 1.60 (m, 4H); 0.98 to 0.87 (m, 6H).

***Example 13***

(2S,3S,4R,5R)3-Amino-5-[6-(3-cyclopentyloxy-benzylamino)-purin-9-yl]-4-hydroxy-tetrahydro-furan-2-carboxylic acid methylamide. (Compound 13)

$C_{23}H_{29}N_7O_4$: MW = 467.53: MS 468 $(M+H)^+$: $^1H$ NMR (400 MHz, $CD_3OD$) δ 8.34 (s, 1H); 8.28 (s, 1H); 7.22 to 7.15 (m, 1H); 6.93 to 6.86 (m, 2H); 6.79 to 6.74 (m, 1H); 6.06 (d, 1H, J = 4.2 Hz); 4.80 to 4.73 (m, 3H); 4.62 to 4.58 (m, 1H); 4.30 (d, 1H, J = 5.6 Hz); 3.79 to 3.73 (m, 1H); 2.81 (s, 3H); 1.94 to 1.81 (m, 2H); 1.81 to 1.70 (m, 4H); 1.67 to 1.56 (m, 2H).

***Example 14***

(2S,3S,4R,5R) 3-Amino-5-[6-(2-cyclopentyloxy-benzylamino)-purin-9-yl]-4-hydroxy-tetrahydro-furan-2-carboxylic acid methylamide. (Compound 14)

$C_{23}H_{29}N_7O_4$: MW = 467.53: MS 468 $(M+H)^+$: $^1H$ NMR (400 MHz, DMSO) δ 8.54(s, 1H); 8.49 (d, 1H, J =

4.6Hz); 8.19 (s, 1H); 8.16 to 8.03 (m, 1H); 7.20 to 7.12 (m, 1H); 7.12 to 7.02 (m, 1H); 6.92 (d, 1H, J = 8.1 Hz); 6.82 to 6.73 (m, 1H); 6.00 (d, 1 H, J = 4.2 Hz); 5.95 to 5.82 (m, 1 H); 4.88 to 4.81 (m, 1H); 4.68 to 4.52 (m, 2H); 4.40 to 4.33 (m, 1H); 4.10 (d, 1H, J = 5.6 Hz); 3.60 to 3.50 (m, 1H); 2.65 (d, 3H, J = 4.8 Hz); 1.96 to 1.82 (m, 8H); 1.82 to 1.46 (m, 2H).

***Example 15***

(2S,3S,4R,5R) 3-Amino-5-[6-(5-chloro-2-isopropoxy-benzylamino)-purin-9-yl]-4-hvdroxy-tetrahydro-furan-2-carboxylic acid methylamide. (Compound 15)

$C_{21}H_{26}ClN_7O_4$: MW = 475.94: MS 476 $(M+H)^+$: $^1H$ NMR (400 MHz, DMSO) δ 8.57 (s, 1H); 8.47 (d, 1H, J = 4.6 Hz); 8.32 to 8.23 (m, 1H); 8.22 (s, 1H); 7.18 (d of d, 1H, J = 8.9 Hz, J = 2.7 Hz); 7.07 to 7.02 (m, 1H); 7.00 (d, 1H, J = 8.9 Hz); 6.01 (d, 1H, J = 4.0 Hz); 5.95 to 5.83 (m, 1H); 4.68 to 4.55 (m, 1H); 4.10 (d, 1H, J = 5.6 Hz); 3.60 to 3.52 (m, 1H); 2.66 (d, 3H, J = 4.6 Hz); 1.86 to 1.75 (m, 2H); 1.26 (d, 6H, J = 5.6 Hz).

***Example 16***

(2S,3S,4R,5R) 3-Amino-5-[6-(2-benzyloxy-benzylamino)-purin-9-yl]-4-hydroxy-tetrahydro-furan-2-carboxylic acid methylamide. (Compound 16)

$C_{25}H_{27}N_7O_4$: MW = 489.54: MS 490 $(M+H)^+$: $^1H$ NMR (400 MHz, DMSO) δ 8.56 (s, 1 H); 8.53 to 8.48 (m, 1H); 8.28 to 8.20 (m, 1 H); 8.20 (s, 1H); 7.53 to 7.46 (m, 1H); 7.41 to 7.36 (m, 2H); 7.36 to 7.26 (m, 1H); 7.21 to 7.10 (m, 1H); 6.86 to 6.80 (m, 1H); 6.02 (d, 1H, J = 3.9 Hz); 5.95 to 5.86 (m, 1H); 5.17 (s, 2H); 4.79 to 4.68 (m, 2H); 4.42 to 4.36 (m, 1H); 4.12 (d, 1H, J = 5.6 Hz); 3.60 to 3.54 (m, 1H); 2.67 (d, 3H, J = 4.8 Hz); 1.83 to 1.74 (m, 2H).

***Example 17***

(2S,3S,4R,5R) 3-Amino-5-{6-[2-(4-fluoro-phenyl)-ethylamino]-purin-9-yl}-4-hydroxy-tetrahydro-furan-2-carboxylic acid methylamide. (Compound 17)

$C_{19}H_{22}FN_7O_3$: MW = 415.43: MS 416 $(M+H)^+$: $^1H$ NMR (400 MHz, $CD_3OD$) δ 8.34 (s, 1H); 8.29 (bs, 1H); 7.25 (m, 2H); 7.0 (m, 2H); 6.01 (d, 1H, J = 4.1 Hz); 4.6 (t, 1H, J = 4.2 Hz); 4.3 (d, 1H, J = 5.5 Hz); 3.8 (bs, 2H); 3.77 (m, 1H); 2.98 (t, 2H, J = 7.0 Hz); 2.8 (s, 3H).

***Example 18***

(2S,3S,4R,5R) 3-Amino-5-{6-[2-(4-benzyloxy-3,5-dimethoxy-phenyl)-ethylamino]-purin-9-yl}-4-hydroxy-tetrahydro-furan-2-carboxylic acid methylamide.

(Compound 18)

$C_{28}H_{33}N_7O_6$: MW = 563.62: MS 564 $(M+H)^+$: $^1H$ NMR (400 MHz, $CD_3OD$) δ 8.31 (s, 1H); 8.25 (bs, 1H); 7.4 (m, 2H); 7.22 (m, 2H); 6.55 (s, 2H); 6.01 (d, 1H, J = 4.1 Hz); 4.82 (s, 2H); 4.55 (t, 1 H, J = 4.4 Hz); 4.24 (d, 1H, J = 5.8 Hz); 3.8 (bs, 2H); 3.72 (s, 6H); 3.70 m, 1H); 2.9 (t, 2H, J = 7.0 Hz); 2.8 (s, 3H).

***Example 19***

(2S,3S,4R,5R) 3-Amino-4-hydroxy-5-(6-methylamino-purin-9-yl)-tetrahydro-furan-2-carboxylic acid methylamide. (Compound 19)

$C_{12}H_{17}N_7O_3$: MW = 307.31: MS 308 $(M+H)^+$: $^1H$ NMR (400 MHz, $CD_3OD$) δ 8.32 (s, 1H); 8.27 (s, 1H); 6.9 (m, 2H); 6.02 (d, 1H, J = 4.0 Hz); 4.59 (m, 1H); 4.30 (d, 1H, J = 5.5 Hz); 3.72 (m, 1H); 3.07 (bs, 3H); 2.8 (s, 3H).

***Example 20***

(2S,3S,4R,5R) 3-Amino-5-{6-[2-(4-fluoro-3-methoxy-phenyl)-ethylamino]-purin-9-yl}-4-hydroxy-tetrahydro-furan-2-carboxylic acid methylamide. (Compound 20)

$C_{20}H_{24}FN_7O_4$: MW = 445.46: MS 446 $(M+H)^+$:$^1H$ NMR (400 MHz, $CD_3OD$) δ 8.33 (s, 1H); 8.3 (bs, 1H); 7.0

(m, 3H); 6.01 (d, 1H, J = 4.1 Hz); 4.6 (t, 1H, J = 4.5 Hz); 4.28 (d, 1H, J = 5.7 Hz); 3.8 (s, 3H); 3.79 (bs, 2H); 3.70 (m, 1H); 2.9 (t, 2H, J = 6.9 Hz); 2.8 (s, 3H).

***Example 21***

<u>(2S,3S,4R,5R) 3-Amino-5-{6-[(3-benzyloxy-6-methyl-pyridin-2-ylmethyl)-amino]-purin-9-yl}-4-hydroxy-tetrahydro-furan-2-carboxylic acid methylamide</u>. (Compound 21)

$C_{25}H_{28}N_8O_4$: MW = 504.55: MS 505 $(M+H)^+$: $^1H$ NMR (400 MHz, DMSO) δ 8.52 (s, 1H); 8.43 (bs, 1H); 8.2 (bs, 1H); 7.7 (bs, 1H); 7.45-7.2 (m, 6H); 7.08 (d, 1H, J = 7.03 Hz); 6.0 (d, 1H, J = 4.1 Hz); 5.85 (bs, 1H); 5.16 (s, 2H); 4.71 (bs, 2H); 4.32 (bs, 1H); 4.07 (d, 1H, J = 5.6 Hz); 3.5 (m, 1H); 2.6 m, 3H); 2.32 (s, 3H).

***Example 22***

<u>(2S,3S,4R,5R) 3-Amino-5-[6-(2,2-diphenyl-ethylamino)-purin-9-yl]-4-hydroxy-tetrahydro-furan-2-carboxylic acid methylamide.</u> (Compound 22)

$C_{25}H_{27}N_7O_3$: MW = 473.54: MS 474 $(M+H)^+$: $^1H$ NMR (400 MHz, $CD_3OD$) δ 8.35 (s, 1H); 8.28 (bs, 1H); 7.4-7.1 (m, 10H); 6.02 (bd, 1H, J = 4.1 Hz); 4.6 (m, 1H); 4.48 (t, 1H, J = 7.9 Hz); 4.22 (bs, 2H); 3.72 (t, 1H, J = 5.7 Hz); 2.8 (s, 3H).

***Example* 23**

<u>(2S,3S,4R,5R) 3-Amino-5-[2-chloro-6-(2,5-dimethoxy-benzylamino)-purin-9-yl]-4-hvdroxy-tetrahydro-furan-2-carboxylic acid methylamide.</u> (Compound 23)

$C_{20}H_{24}ClN_7O_5$: MW = 477.91: MS 478 $(M+H)^+$: $^1H$ NMR (400 MHz, DMSO) δ 8.79 (bs, 1H); 8.61 (s, 1H); 8.2 (bs, 2H); 6.9 (m, 1H); 6.8 (m, 1H); 6.7 (s, 1H); 5.98 (bs, 3H); 4.6 (m, 2H); 4.3 (bs, 1H); 4.07 (bs, 1H); 3.8 (s, 3H); 3.6 (s, 3H); 3.56 (bs, 1H); 2.63 (s, 3H).

***Example 24***

<u>(2S,3S,4R,5R) 3-Amino-5-{6-[2-(3-benzyloxy-4-methoxy-phenyl)-ethylamino]-purin-9-yl}-4-hydroxy-tetrahydro-furan-2-carboxylic acid methylamide.</u> (Compound 24)

$C_{27}H_{31}N_7O_5$: MW = 533.59: MS 534 (M+H): $^1H$ NMR (400 MHz, $CD_3OD$) δ 8.35 (s, 1H); 8.25 (bs, 1H); 7.4-7.2 (m, 5H); 6.9-6.8 (m, 3H); 6.0 (bd, 1H, J = 3.7 Hz); 5.0 (s, 2H); 4.59 (t, 1H, J = 4.2 Hz); 4.28 (d, 1H, J = 5.8 Hz); 3.8 (s, 3H); 3.7 (t, 1H, J = 5.6 Hz); 2.86 (t, 2H, J = 6.3 Hz); 2.8 (s, 3H).

***Example* 25**

<u>(2S,3S,4R,5R) 3-Amino-4-hydroxy-5-[6-(2-pyridin-3-yl-ethylamino)-purin-9-yl]-tetrahydro-furan-2-carboxylic acid methylamide.</u> (Compound 25)

$C_{18}H_{22}N_8O_3$: MW = 398.43: MS 399 $(M+H)^+$: $^1H$ NMR (400 MHz, $CD_3OD$) δ 8.43 (bs, 1H); 8.38 (m, 1H); 8.37 (s, 1H); 8.3 (bs, 1H); 7.8 (m, 1H); 7.4 (m, 1H); 6.03 (d, 1H, J = 4.0 Hz); 4.6 (t, 1H, J = 4.2 Hz); 4.33 (d, 1H, J = 5.8 Hz); 3.9 (bs, 2H); 3.8 (t, 1H, J = 5.8 Hz); 3.05 (t, 2H, J = 7.0 Hz); 2.8 (s, 3H).

***Example 26***

<u>(2S,3S,4R,5R) 3-Amino-5-[6-(2,5-dimethoxy-benzylamino)-purin-9-yl]-4-hydroxy-tetrahydro-furan-2-carboxylic acid methylamide.</u> (Compound 26)

$C_{20}H_{25}N_7O_5$: MW = 443.46: MS 444 $(M+H)^+$: $^1H$ NMR (400 MHz, $CD_3OD$) δ 8.35 (s, 1 H); 8.25 (bs, 1H); 6.9 (m, 2H); 6.8 (m, 2H); 6.0 (bd, 1H, J = 4.0 Hz); 4.78 (bs, 2H); 4.6 (t, 1H, J = 4.4 Hz); 4.3 (d, 1H, J = 5.5 Hz); 3.8 (s, 3H); 3.7 (t, 1H, J = 5.5 Hz); 3.65 (s, 3H); 2.8 (s, 3H).

***Example 27***

(2S,3S,4R,5R) 3-Amino-4-hydroxy-5-(6-phenethylamino-purin-9-yl)-tetrahydro-furan-2-carboxylic acid methylamide. (Compound 27)

$C_{19}H_{23}N_7O_3$: MW = 397.44: MS 398 $(M+H)^+$: $^1H$ NMR (400 MHz, $CD_3OD$) δ 8.3 (s, 1H); 8.25 (bs, 1H); 7.23 (m, 4H); 7.17 (m, 1H); 6.0 (bd, 1H, J = 4.2 Hz); 4.55 (m, 1H); 4.27 (d, 1H, J = 5.7 Hz); 3.8 (bs, 2H); 3.7 (m, 1H); 2.95 (t, 2H, J = 6.9 Hz); 2.8 (s, 3H).

***Example 28***

(2S,3S,4R,5R) 3-Amino-5-(2-chloro-6-methylamino-purin-9-yl)-4-hydroxy-tetrahydro-furan-2-carboxylic acid methylamide. (Compound 28)

$C_{12}H_{16}ClN_7O_3$: MW = 341.76: MS 342 $(M+H)^+$: $^1H$ NMR (400 MHz, DMSO) δ 8.58 (s, 1H); 8.3 (bs, 1H); 8.2 (bs, 2H); 5.98 (d, 1H, J = 4.0 Hz); 4.3 (bs, 1H); 4.1 (m, 1H); 3.6 (m, 1H); 3.35 (m, 1H); 2.9 (bs, 3H); 2.63 (s, 3H).

***Example 29***

(2S,3S,4R,5R) 3-Amino-4-hydroxy-5-[6-(2-phenyl-cyclopropylamino)-purin-9-yl]-tetrahydro-furan-2-carboxylic acid methylamide. (Compound 29)

$C_{20}H_{23}N_7O_3$: MW = 409.45: MS 410 $(M+H)^+$: $^1H$ NMR (400 MHz, $CD_3OD$) δ 8.34 (s, 1H); 8.3 (s, 1H); 7.4-7.3 (m, 5H); 6.03 (d, 1H, J = 4.1 Hz); 4.6 (m, 1H); 4.35 (d, 1H, J = 56 Hz); 3.8 (m, 1H); 3.2 (bs, 1H); 2.8 (s, 3H); 2.2 (m, 1H); 1.3 (m, 2H).

***Example 30***

(2S,3S,4R,5R) 3-Amino-5-[2-chloro-6-(2,5-dichloro-benzylamino)-purin-9-yl]-4-hydroxy-tetrahydro-furan-2-carboxylic acid methylamide. (Compound 30)

$C_{18}H_{18}Cl_3N_7O_3$: MW = 486.75: MS 486 $(M+H)^+$ $^1H$ NMR (400 MHz, $CD_3OD$) δ 8.36 (bs, 1H); 7.42 (bs, 1H); 7.4 (d, 1H, J = 8.6 Hz); 7.27 (m, 1H); 6.0 (bd, 1H, J = 3.7 Hz); 4.8 (bs, 2H); 4.58 (t, 1H, J = 4.1 Hz); 4.3 (d, 1H, J = 5.6 Hz); 3.75 (t, 1H, J=4.1 Hz); 2.82 (s, 3H).

***Example 31***

(2S,3S,4R,5R) 3-Amino-4-hydroxy-5-{6-[2-(2-morpholin-4-yl-thiazol-5-yl)-ethylamino]-purin-9-yl}-tetrahydro-furan-2-carboxylic acid methylamide. (Compound 31)

$C_{20}H_{27}N_9SO_4$: MW = 489.56: MS 490 $(M+H)^+$: $^1H$ NMR (400 MHz, $CD_3OD$) δ 8.3 (s, 1H); 8.23 (bs, 1H); 6.4 (s, 1H); 6.0 (bd, 1H, J = 4.1 Hz); 4.85 (bs, 1H); 4.57 (t, 1H, J = 4.4 Hz); 4.25 (d, 1H, J = 5.5 Hz); 3.8 (bs, 2H); 3.72 (m, 5H); 3.4 (m, 2H); 2.9 (t, 1H, J = 6.5 Hz); 2.8 (s, 3H); 2.78 (m, 2H).

***Example 32***

(2S,3S,4R,5R) 3-Amino-4-hydroxy-5-[6-(2-naphthalen-1-yl-ethylamino)-purin-9-yl]-tetrahydro-furan-2-carboxylic acid methylamide. (Compound 32)

$C_{23}H_{25}N_7O_3$: MW = 447.50: MS 448 $(M+H)^+$: $^1H$ NMR (400 MHz, $CD_3OD$) δ 8.32 (bs, 1H); 8.3 (bs, 1H); 7.82 (d, 1H, J = 6.0 Hz); 7.70 (d, 1H, J = 6.0 Hz); 7.5-7.3 (m, 5H); 6.02 (d, 1H, J = 5.5 Hz); 4.58 (t, 1H, J = 4.5 Hz); 4.29 (d, 1H, J = 4.5 Hz); 3.92 (bs, 2H); 3.7 (t, 1H, J = 6.5 Hz); 3.42 (t, 2H, J = 7.0 Hz); 2.8 (s, 3H).

***Example 33***

(2S,3S,4R,5R) 3-Amino-5-{6-[(5-fluoro-1H-indol-3-ylmethyl)-amino]-purin-9-yl}-4-hydroxy-tetrahydro-furan-2-carboxylic acid methylamide (Compound 33)

$C_{21}H_{23}FN_8O_3$: MW = 454.47: MS 455 (M+H): $^1$H NMR (400 MHz, $CD_3OD$) $\delta$ 8.6 (d, 1H, J = 21 Hz); 8.3 (bs, 1H); 7.22 (m, 2H); 6.8 (m, 3H); 6.0 (bd, 1H, J = 22 Hz); 4.6 (bs, 1H); 4.37 (bs, 1H); 4.2 (bs, 2H); 3.82 (bs, 2H); 3.15 (t, 1H, J = 7.2 Hz); 2.8 (d, 3H, J = 9 Hz).

***Example 34***

(2S,3S,4R,5R) 3-Amino-5-{6-[2-(4-benzyloxy-3-methoxy-phenyl)-ethylamino]-purin-9-yl}-4-hydroxy-tetrahydro-furan-2-carboxylic acid methylamide (Compound 34)

$C_{27}H_{31}N_7O_5$: MW = 533.59: MS 534 (M+H): $^1$H NMR (400 MHz, $CD_3OD$) $\delta$ 8.32 (s, 1H); 8.25 (bs, 1H); 7.4-7.2 (m, 5H); 6.9 (d, 1H, J = 1.7 Hz); 6.84 (d, 1H, J = 8.1 Hz); 6.75 (dd, 1H, J = 8.1, 1.7 Hz); 6.03 (d, 1H, J = 4.1 Hz); 5.02 (s, 2H); 4.6 (t, 1H, J = 5.1 Hz); 4.3 (d, 1H, J = 5.8 Hz); 3.81 (bs, 2H); 3.8 (s, 3H); 2.9 (t, 2H, J = 7.0 Hz); 2.8 (s, 3H).

***Example 35***

(2S,3S,4R,5R) 3-Amino-4-hydroxy-5-[6-(2-pyridin-2-yl-ethylamino)-purin-9-yl]-tetrahydro-furan-2-carboxylic acid methylamide (Compound 35)

$C_{18}H_{22}N_8O_3$: MW = 398.43: MS 399 $(M+H)^+$: $^1$H NMR (400 MHz, $CD_3OD$) $\delta$ 8.31 (s, 1H); 8.27 (bs, 1H); 7.3 (m, 4H); 6.02 (d, 1H, J = 3.8 Hz); 4.6 (t, 1H, J = 4.2 Hz); 4.3 (d, 1H, J = 5.8 Hz); 3.98 (bs, 2H); 3.75 (t, 1H, J = 5.8 Hz); 3.2 (t, 2H, J = 7.0 Hz); 2.8 (s, 3H).

***Example* 36**

(2S,3S,4R,5R) 3-Amino-4-hydroxy-5-[6-(2-phenyl-cyclopropylamino)-purin-9-yl]-tetrahydro-furan-2-carboxylic acid methylamide (Compound 36)

$C_{20}H_{23}N_7O_3$: MW = 409.45: MS 410 $(M+H)^+$: $^1$H NMR (400 MHz, $CD_3OD$) $\delta$ 8.34 (s, 1H); 8.3 (s, 1H); 7.4-7.3 (m, 5H); 6.03 (d, 1H, J = 4.1 Hz); 4.6 (m, 1H); 4.35 (d, 1 H, J = 5.6 Hz); 3.8 (m, 1H); 3.2 (bs, 1H); 2.8 (s, 3H); 2.2 (m, 1H); 1.3 (m, 2H).

The compounds exemplified above in Examples 1-36 demonstrated an A3 $IC_{50}$ from 15 nM to 500 nM. It should be understood that the invention is not limited to the particular embodiments described herein, but that various changes and modifications may be made without departing from the spirit and scope of this novel concept as defined by the following claims.

## Claims

**1.** A compound having Formula (I)

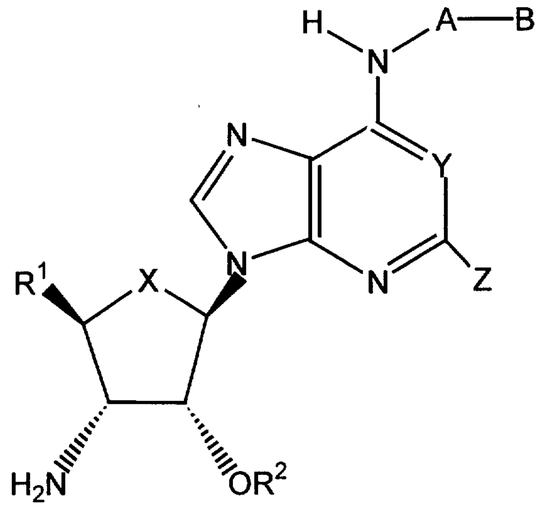

**(I)**

wherein

X is oxy, methylene or thio;
Y is CH or N;
Z is H, $(C_1-C_4)$alkyl, $(C_1-C_4)$alkyloxy, trifluoromethyl or halo;
$R^1$ is hydroxymethyl, $(C_1-C_3)$alkoxymethyl, $(C_3-C_5)$cycloalkoxymethyl, carboxy, $(C_1-C_3)$alkoxycarbonyl, $(C_3-C_5)$cycloalkoxycarbonyl, 1,1-aminoiminomethyl, 1,1-(mono-N-or di-N,N-$(C_1-C_4)$alkylamino)iminomethyl, 1,1-(mono-N- or di-N,N-$(C_3-C_5)$cycloalkylamino)iminomethyl, carbamoyl, mono-N- or di-N,N-$(C_1-C_4)$alkylaminocarbonyl, mono-N- or di-N,N-$(C_3-C_5)$cycloalkylaminocarbonyl, or N-$(C_1-C_4)$alkyl-N-$(C_3-C_5)$cycloalkylaminocarbonyl;
$R^2$ is H, $(C_1-C_3)$alkyl or $(C_3-C_5)$cycloalkyl;
A is $-(CH_2)_n-$ where n is an integer from 1 to 4, or $-(C_mH_{2m-2})-$ where m is an integer from 3 to 6; and
B is hydrogen, substituted or unsubstituted heteroaryl, substituted or unsubstituted aryl, $-CH(aryl)_2$, or

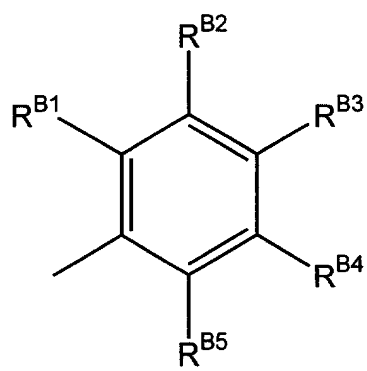

where $R^{B1}$, $R^{B2}$, $R^{B3}$, $R^{B4}$ and $R^{B5}$ are each independently selected from the group consisting of hydrogen, $(C_1-C_4)$alkyl, halo, hydroxy, thio, amino, $(C_1-C_6)$alkyloxy, $(C_1-C_6)$alkylthio, $(C_1-C_6)$alkylamino and -D-G, where D is oxy, thio, NH, $(C_1-C_6)$alkyloxy, $(C_1-C_6)$alkylthio or $(C_1-C_6)$alkylamino and
G is a partially saturated, fully saturated or fully unsaturated five to eight membered ring optionally having one to three heteroatoms selected independently from oxygen, sulfur and nitrogen, or a bicyclic ring consisting of two fused partially saturated, fully saturated or fully unsaturated three to six membered rings, taken independently, optionally having one to four heteroatoms selected independently from nitrogen, sulfur and oxygen,

wherein G is optionally mono-, di- or tri-substituted independently with halo, $(C_1-C_3)$alkyl, trifluoromethyl, trifluoromethoxy, nitro, cyano, $(C_3-C_5)$cycloalkyl, hydroxy or $(C_1-C_3)$alkoxy, or
G is cyano, $(C_1-C_4)$alkoxycarbonyl, $(C_3-C_5)$cycloalkoxycarbonyl, C(O)$NR^4R^5$, C(S)$NR^4R^5$, C(NH)$NR^4R^5$, C(N$(C_1-C_3)$alkyl)$NR^4R^5$ or C(N$(C_3-C_{10})$cycloalkyl)$NR^4R^5$, where
$R^4$ is H, $(C_1-C_{10})$alkyl, hydroxy, $(C_1-C_{10})$alkoxy, $(C_3-C_{10})$cycloalkoxy or a partially saturated, fully saturated or fully unsaturated five to eight membered ring, optionally linked through $(C_1-C_3)$alkyl, optionally having one to three heteroatoms selected independently from oxygen, sulfur and nitrogen, or, a bicyclic ring or a bicyclic ring with optional $(C_1-C_3)$ bridge optionally linked through $(C_1-C_3)$alkyl, said bicyclic ring or bridged bicyclic ring optionally having one to four heteroatoms selected independently from nitrogen, sulfur and oxygen wherein said $(C_1-C_{10})$alkyl, $(C_1-C_{10})$alkoxy, $(C_3-C_{10})$cycloalkoxy or $R^4$ ring(s) is optionally mono-, di- or tri-substituted independently with halo, $(C_1-C_3)$alkyl, trifluoromethyl, nitro, cyano, $(C_3-C_5)$cycloalkyl, hydroxy or $(C_1-C_3)$ alkoxy, and
$R^5$ is H, $(C_1-C_{10})$alkyl or $(C_1-C_{10})$cycloalkyl; or $R^4$ and $R^5$ taken together with the nitrogen to which they are attached form a fully saturated or partially unsaturated four to nine membered ring, said ring being optionally bridged, optionally having one to three additional heteroatoms selected independently from oxygen, sulfur and nitrogen, said ring being optionally mono- or di-substituted independently with oxo, hydroxy, $(C_1-C_6)$alkoxy, $(C_1-C_8)$alkyl, amino, mono-N- or di-N,N-$(C_1-C_4)$alkylaminocarbonyl, mono-N- or di-N,N-$(C_3-C_5)$cycloalkylaminocarbonyl, N-$(C_1-C_4)$alkyl-N-$(C_3-C_5)$cycloalkylaminocarbonyl, mono-N- or di-N,N-$(C_1-C_4)$alkylamino, mono-N- or di-N,N-$(C_3-C_5)$cycloalkylamino, N-$(C_1-C_4)$alkyl-N-$(C_3-C_5)$cycloalkylamino, formylamino, $(C_1-C_4)$alkylcarbonylamino, $(C_3-C_5)$cycloalkylcarbonylamino, $(C_1-C_4)$alkoxycarbonylamino, N-$(C_1-C_4)$alkoxycarbonyl-N-$(C_1-C_4)$alkylamino, $(C_1-C_4)$sulfamoyl, $(C_1-C_4)$alkylsulfonylamino, $(C_3-C_5)$cycloalkylsulfonylamino or a partially saturated, fully saturated or fully unsaturated five to eight membered ring, optionally linked through $(C_1-C_3)$alkyl, optionally having one to three heteroatoms selected independently from oxygen, sulfur and nitrogen, or a bicyclic ring consisting of two fused partially saturated, fully saturated or fully unsaturated three to six membered rings, taken independently, optionally linked through $(C_1-C_3)$alkyl, optionally having one to four heteroatoms selected independently from nitrogen, sulfur and oxygen, optionally mono- or di-substituted with halo, trifluoromethyl, trifluoromethoxy, $(C_1-C_3)$alkyl or $(C_1-C_3)$alkoxy;

provided that A is not $-(CH_2)_1-$, when $R^{B1}$ is -D-G, $R^{B4}$ is halo, trifluoromethyl, cyano, $(C_1-C_3)$ alkyl, $(C_1-C_3)$ alkyloxy, ethenyl or ethynyl, and $R^{B2}$, $R^{B3}$ and $R^{B5}$ are hydrogen;
a prodrug thereof, or a pharmaceutically acceptable salt, hydrate or solvate of said compound or said prodrug.

**2.** The compound of Claim 1 wherein

X is oxy;
Y is N;
Z is H or Cl;
$R^1$ is $(C_1-C_6)$alkylcarbamoyl;
$R^2$ is H;
A is $-(CH_2)_n-$, where n is 1 or 2, or cyclopropyl; and
B is substituted or unsubstituted heteroaryl, naphthyl, -CH(aryl)$_2$, or

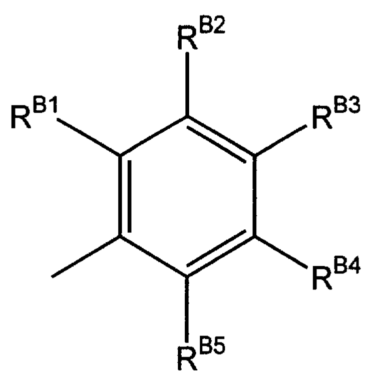

where $R^{B1}$, $R^{B2}$, $R^{B3}$, $R^{B4}$ and $R^{B5}$ are each independently selected from the group consisting of hydrogen, $(C_1-C_4)$ alkyl, halo, hydroxy, thio, amino, $(C_1-C_6)$alkyloxy, $(C_1-C_6)$alkylthio, $(C_1-C_6)$alkylamino and -D-G, where

D is oxy, thio, $(C_1-C_6)$alkyloxy or $(C_1-C_6)$alkylthio, and
G is phenyl, pyridyl, pyrimidinyl, pyrazinyl, thiazolyl, oxazolyl, isoxazolyl, pyridinazinyl, tetrazolyl, isothiazolyl, thiophenyl, furanyl, 1,2,4-oxadiazolyl, 1,2,4-thiadiazolyl, pyrazolyl, pyrrolyl, indolyl, naphthalenyl, quinolinyl, isoquinolinyl, benzo[b]furanyl, benzo[b]thiophenyl, benzothiazolyl, tetrahydrofuranyl, pyrrolidinyl, piperidinyl, tetrahydropyranyl, morpholinyl wherein said G is optionally mono-, di- or tri-substituted independently with halo, $(C_1-C_3)$alkyl or $(C_1-C_3)$alkoxy;

a prodrug thereof, or a pharmaceutically acceptable salt, hydrate or solvate of said compound or said prodrug.

**3.** The compound of Claim 2 wherein B is a substituted or unsubstituted pyridyl, indolyl or thiazolyl; a prodrug thereof, or a pharmaceutically acceptable salt, solvate, or hydrate of said compound or said prodrug.

**4.** The compound of Claim 3 wherein said substituted pyridyl, indolyl or thiazolyl is substituted with at least one substituent selected from the group consisting of $(C_1-C_4)$alkyl, halo, hydroxy, thio, amino, $(C_1-C_6)$alkyloxy, $(C_1-C_6)$alkylthio, $(C_1-C_6)$alkylamino and -D-G, where D is oxy, thio, $(C_1-C_6)$alkyloxy or $(C_1-C_6)$alkylthio, and G is phenyl, pyridyl, pyrimidinyl, pyrazinyl, thiazolyl, oxazolyl, isoxazolyl, pyridinazinyl, tetrazolyl, isothiazolyl, thiophenyl, furanyl, 1,2,4-oxadiazolyl, 1,2,4-thiadiazolyl, pyrazolyl, pyrrolyl, indolyl, naphthalenyl, quinolinyl, isoquinolinyl, benzo[b]furanyl, benzo[b]thiophenyl, benzothiazolyl, tetrahydrofuranyl, pyrrolidinyl, piperidinyl, tetrahydropyranyl, morpholinyl wherein said G is optionally mono-, di- or tri-substituted independently with halo, $(C_1-C_3)$alkyl or $(C_1-C_3)$alkoxy; a prodrug thereof, or a pharmaceutically acceptable salt, solvate, or hydrate of said compound or said prodrug.

**5.** The compound of Claim 2 wherein B is

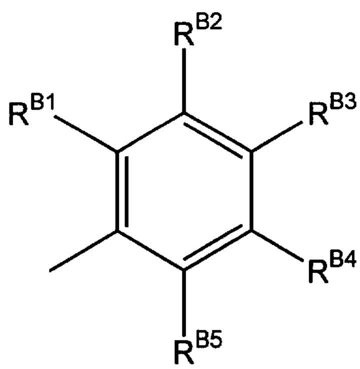

where $R^{B1}$, $R^{B2}$, $R^{B3}$, $R^{B4}$ and $R^{B5}$ are each independently selected from the group consisting of hydrogen, $(C_1-C_4)$alkyl, halo, hydroxy, $(C_1-C_6)$alkyloxy and -D-G, where

D is $(C_1-C_6)$alkoxy and
G is phenyl, pyridyl, thiazolyl, oxazolyl, isoxazolyl, isothiazolyl, furanyl, 1,2,4-oxadiazolyl, 1,2,4-thiadiazolyl, pyrazolyl, pyrrolyl, or morpholinyl wherein said G is optionally mono-, di- or tri-substituted independently with halo, $(C_1-C_3)$alkyl, trifluoromethoxy or $(C_1-C_3)$alkoxy;

a prodrug thereof, or a pharmaceutically acceptable salt, hydrate or solvate of said compound or said prodrug.

**6.** A compound selected from the group consisting of

(2S,3S,4R,5R) 3-amino-5-{6-[2-(2,5-dimethoxy-phenyl)-ethylamino]-purin-9-yl}-4-hydroxy-tetrahydro-furan-2-carboxylic acid methylamide,
(2S,3S,4R,5R) 3-amino-4-hydroxy-5-[6-(3-methoxy-benzylamino)-purin-9-yl]-tetrahydro-furan-2-carboxylic acid methylamide,
(2S,3S,4R,5R) 3-amino-5-[6-(4-benzyloxy-benzylamino)-purin-9-yl]-4-hydroxy-tetrahydro-furan-2-carboxylic acid methylamide,
(2S,3S,4R,5R) 3-amino-4-hydroxy-5-[6-(2-hydroxy-5-methoxybenzylamino)-purin-9-yl]-tetrahydro-furan-2-carboxylic acid methylamide,
(2S,3S,4R,5R) 3-amino-5-[6-(3-butoxy-benzylamino)-purin-9-yl]-4-hydroxy-tetrahydro-furan-2-carboxylic acid methylamide,
(2S,3S,4R,5R) 3-amino-5-[6-(2,5-dimethyl-benzylamino)-purin-9-yl]-4-hydroxy-tetrahydro-furan-2-carboxylic acid methylamide,
(2S,3S,4R,5R) 3-amino-5-[6-(2,5-dichloro-benzylamino)-purin-9-yl]-4-hydroxy-tetrahydro-furan-2-carboxylic acid methylamide,
(2S,3S,4R,5R) 3-amino-4-hydroxy-5-{6-[3-(2-morpholin-4-yl-ethoxy)-benzylamino]-purin-9-yl}-tetrahydro-furan-2-carboxylic acid methylamide,
(2S,3S,4R,5R) 3-amino-4-hydroxy-5-{6-[3-(3-methyl-isoxazol-5-ylmethoxy)-benzylamino]-purin-9-yl}-tetrahydro-furan-2-carboxylic acid methylamide,
(2S,3S,4R,5R) 3-amino-4-hydroxy-5-[6-(2-methoxy-5-methyl-benzylamino)-purin-9-yl-tetrahydro-furan-2-carboxylic acid methylamide,
(2S,3S,4R,5R) 3-amino-5-[6-(2,5-diethyl-benzylamino)-purin-9-yl]-4-hydroxy-tetrahydro-furan-2-carboxylic acid methylamide,
(2S,3S,4R,5R) 3-amino-5-{6-[2-(1-ethyl-propoxy)-5-methoxy-benzylamino]-purin-9-yl}-4-hydroxy-tetrahydro-furan-2-carboxylic acid methylamide,
(2S,3S,4R,5R) 3-amino-5-[6-(3-cyclopentyloxy-benzylamino)-purin-9-yl]-4-hydroxy-tetrahydro-furan-2-carboxylic acid methylamide.
(2S,3S,4R,5R) 3-amino-5-[6-(2-cyclopentyloxy-benzylamino)-purin-9-yl]-4-hydroxy-tetrahydro-furan-2-carboxylic acid methylamide,
(2S,3S,4R,5R) 3-amino-5-[6-(5-chloro-2-isopropoxy-benzylamino)-purin-9-yl]-4-hydroxy-tetrahydro-furan-2-carboxylic acid methylamide,
(2S,3S,4R,5R) 3-amino-5-[6-(2-benzyloxy-benzylamino)-purin-9-yl]-4-hydroxy-tetrahydro-furan-2-carboxylic acid methylamide,
(2S,3S,4R,5R) 3-amino-5-{6-[2-(4-fluoro-phenyl)-ethylamino]-purin-9-yl}-4-hydroxy-tetrahydro-furan-2-carboxylic acid methylamide.
(2S,3S,4R,5R) 3-amino-5-{6-[2-(4-benzyloxy-3,5-dimethoxy-phenyl)-ethylamino]-purin-9-yl}-4-hydroxy-tetrahydro-furan-2-carboxylic acid methylamide,
(2S,3S,4R,5R) 3-amino-4-hydroxy-5-(6-methylamino-purin-9-yl)-tetrahydrofuran-2-carboxylic acid methylamide,
(2S,3S,4R,5R) 3-amino-5-{6-[2-(4-fluoro-3-methoxy-phenyl)-ethylamino]-purin-9-yl}-4-hydroxy-tetrahydro-furan-2-carboxylic acid methylamide,
(2S,3S,4R,5R) 3-amino-5-{6-[(3-benzyloxy-6-methyl-pyridin-2-ylmethyl)-amino]-purin-9-yl}-4-hydroxy-tetrahydro-furan-2-carboxylic acid methylamide,
(2S,3S,4R,5R) 3-amino-5-[6-(2,2-diphenyl-ethylamino)-purin-9-yl]-4-hydroxy-tetrahydro-furan-2-carboxylic acid methylamide,
(2S,3S,4R,5R) 3-amino-5-[2-chloro-6-(2,5-dimethoxy-benzylamino)-purin-9-yl]-4-hydroxy-tetrahydro-furan-2-carboxylic acid methylamide,
(2S,3S,4R,5R) 3-amino-5-{6-[2-(3-benzyloxy-4-methoxy-phenyl)-ethylamino]-purin-9-yl}-4-hydroxy-tetrahydro-furan-2-carboxylic acid methylamide,
(2S,3S,4R,5R) 3-amino-4-hydroxy-5-[6-(2-pyridin-3-yl-ethylamino)-purin-9-yl]-tetrahydro-furan-2-carboxylic acid methylamide,
(2S,3S,4R,5R) 3-amino-5-[6-(2,5-dimethoxy-benzylamino)-purin-9-yl]-4-hydroxy-tetrahydro-furan-2-carboxylic acid methylamide,
(2S,3S,4R,5R) 3-amino-4-hydroxy-5-(6-phenethylamino-purin-9-yl)-tetrahydro-furan-2-carboxylic acid methylamide,
(2S,3S,4R,5R) 3-amino-5-(2-chloro-6-methylamino-purin-9-yl)-4-hydroxy-tetrahydro-furan-2-carboxylic acid methylamide,
(2S,3S,4R,5R) 3-amino-4-hydroxy-5-[6-(2-phenyl-cyclopropylamino)-purin-9-yl]-tetrahydro-furan-2-carboxylic acid methylamide,
(2S,3S,4R,5R) 3-amino-5-[2-chloro-6-(2,5-dichloro-benzylamino)-purin-9-yl]-4-hydroxy-tetrahydro-furan-2-carboxylic acid methylamide,
(2S,3S,4R,5R) 3-amino-4-hydroxy-5-{6-[2-(2-morpholin-4-yl-thiazol-5-yl)-ethylamino]-purin-9-yl}-tetrahydro-furan-2-carboxylic acid methylamide,
(2S,3S,4R,5R) 3-amino-4-hydroxy-5-[6-(2-naphthalen-1-yl-ethylamino)-purin-9-yl]-tetrahydro-furan-2-carboxylic acid methylamide,
(2S,3S,4R,5R) 3-amino-5-{6-[(5-fluoro-1H-indol-3-ylmethyl)-amino]-purin-9-yl}-4-hydroxy-tetrahydro-furan-2-carboxylic acid methylamide,
(2S,3S,4R,5R) 3-amino-5-{6-[2-(4-benzyloxy-3-methoxy-phenyl)-ethylamino]-purin-9-yl}-4-hydroxy-tetrahydro-furan-2-carboxylic acid methylamide,
(2S,3S,4R,5R) 3-amino-4-hydroxy-5-[6-(2-pyridin-2-yl-ethylamino)-purin-9-yl]-tetrahydro-furan-2-carboxylic acid methylamide, and
(2S,3S,4R,5R) 3-amino-4-hydroxy-5-[6-(2-phenyl-cyclopropylamino)-purin-9-yl]-tetrahydro-furan-2-carboxylic acid methylamide;

a prodrug thereof, or a pharmaceutically acceptable salt, solvate, or hydrate of said compound or said prodrug.

**7.** Use of a compound, a prodrug thereof, or a pharmaceutically acceptable salt, solvate, or hydrate of said compound or said prodrug according to any one of the preceding claims, in the manufacture of a medicament for reducing tissue damage resulting from ischemia or hypoxia.

**8.** The use of Claim 7 wherein the tissue is cardiac, brain, liver, kidney, lung, gut, skeletal muscle, spleen, pancreas, nerve, spinal cord, retina tissue, the vasculature, or intestinal tissue.

**9.** A pharmaceutical composition which comprises a compound, a prodrug thereof, or a pharmaceutically acceptable salt, solvate, or hydrate of said compound or said prodrug according to any one of Claims 1, 2, 3, 4, 5, or 6, and a pharmaceutically acceptable carrier, vehicle or diluent.

**10.** A pharmaceutical combination composition comprising: a therapeutically effective amount of a composition comprising

a) a first compound, said first compound being a compound, a prodrug thereof, or a pharmaceutically acceptable salt, solvate, or hydrate of said compound or said prodrug according to Claims 1, 2, 3, 4, 5, or 6;
b) a second compound, said second compound being a cardiovascular agent, a glycogen phosphorylase inhibitor, a sorbitol dehydrogenase inhibitor, or an aldose reductase inhibitor; and
c) a pharmaceutical carrier, vehicle or diluent.

**11.** A pharmaceutical kit comprising:

a) a first compound, said first compound being a compound, a prodrug thereof, or a pharmaceutically acceptable salt, solvate, or hydrate of said compound or said prodrug according to any one of Claims 1, 2, 3, 4, 5, or 6, and a pharmaceutically acceptable carrier, vehicle or diluent in a first unit dosage form;
b) a second compound, said second compound being a cardiovascular agent, a glycogen phosphorylase inhibitor, a sorbitol dehydrogenase inhibitor, or an aldose reductase inhibitor and a pharmaceutically acceptable carrier, vehicle or diluent in a second unit dosage form;

as a combined preparation for simultaneous, separate or sequential use in reducing tissue damage resulting from ischemia or hypoxia.

European Patent Office

# EUROPEAN SEARCH REPORT

Application Number
EP 02 25 1174

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,A | US 5 688 774 A (JEONG HEAOK KIM ET AL) 18 November 1997 (1997-11-18) * claims * | 1-11 | C07H19/16 C07D473/34 C07D471/04 A61K31/7076 A61P9/10 //(C07D471/04, 235:00,221:00) |
| A | WO 00 23447 A (MYERS MICHAEL R ;CHOI SLEDESKI YONG MI (US); PAULS HEINZ W (US); E) 27 April 2000 (2000-04-27) * claims * | 1-11 | |
| A | WO 98 08855 A (CV THERAPEUTICS INC ;LUM ROBERT T (US); NELSON MAREK G (US); PFIST) 5 March 1998 (1998-03-05) * claims * | 1-11 | |
| P,A | WO 01 23399 A (MASAMUNE HIROKO ;PFIZER PROD INC (US); DENINNO MICHAEL PAUL (US);) 5 April 2001 (2001-04-05) * claims * | 1-11 | |

TECHNICAL FIELDS SEARCHED (Int.Cl.7)

C07H
C07D
A61K
A61P

The present search report has been drawn up for all claims

| Place of search | Date of completion of the search | Examiner |
|---|---|---|
| THE HAGUE | 4 June 2002 | Chouly, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 02 25 1174

This annex lists the patent family members relating to the patent documents cited in the above-mentioned European search report.
The members are as contained in the European Patent Office EDP file on
The European Patent Office is in no way liable for these particulars which are merely given for the purpose of information.

04-06-2002

| Patent document cited in search report | | Publication date | Patent family member(s) | | | Publication date |
|---|---|---|---|---|---|---|
| US 5688774 | A | 18-11-1997 | US | 5773423 | A | 30-06-1998 |
| | | | AT | 206432 | T | 15-10-2001 |
| | | | AU | 7331094 | A | 13-02-1995 |
| | | | DE | 69428536 | D1 | 08-11-2001 |
| | | | EP | 0708781 | A1 | 01-05-1996 |
| | | | WO | 9502604 | A1 | 26-01-1995 |
| WO 0023447 | A | 27-04-2000 | US | 6376472 | B1 | 23-04-2002 |
| | | | AU | 6410799 | A | 08-05-2000 |
| | | | WO | 0023447 | A1 | 27-04-2000 |
| WO 9808855 | A | 05-03-1998 | US | 5789416 | A | 04-08-1998 |
| | | | AT | 202361 | T | 15-07-2001 |
| | | | AU | 726597 | B2 | 16-11-2000 |
| | | | AU | 4080997 | A | 19-03-1998 |
| | | | BR | 9711444 | A | 18-01-2000 |
| | | | CN | 1234803 | A | 10-11-1999 |
| | | | CZ | 9900614 | A3 | 13-10-1999 |
| | | | DE | 69705312 | D1 | 26-07-2001 |
| | | | DE | 69705312 | T2 | 11-10-2001 |
| | | | DK | 920438 | T3 | 10-09-2001 |
| | | | EP | 1081155 | A2 | 07-03-2001 |
| | | | EP | 0920438 | A2 | 09-06-1999 |
| | | | EP | 0992510 | A1 | 12-04-2000 |
| | | | ES | 2157593 | T3 | 16-08-2001 |
| | | | HU | 0001640 | A2 | 28-05-2001 |
| | | | JP | 2000501426 | T | 08-02-2000 |
| | | | JP | 3157842 | B2 | 16-04-2001 |
| | | | NO | 990787 | A | 19-02-1999 |
| | | | NO | 20020758 | A | 19-02-1999 |
| | | | NO | 20020759 | A | 19-02-1999 |
| | | | NZ | 334095 | A | 27-10-2000 |
| | | | PL | 331853 | A1 | 16-08-1999 |
| | | | PT | 920438 | T | 30-10-2001 |
| | | | TR | 9900377 | T2 | 21-05-1999 |
| | | | WO | 9808855 | A2 | 05-03-1998 |
| WO 0123399 | A | 05-04-2001 | AU | 7035200 | A | 30-04-2001 |
| | | | WO | 0123399 | A1 | 05-04-2001 |

EPO FORM P0459

For more details about this annex : see Official Journal of the European Patent Office, No. 12/82